# FASCICULE DE BREVET EUROPEEN

(11) **EP 2 280 986 B1**
(45) Date de publication et mention de la délivrance du brevet: **10.07.2013**
(21) Numéro de dépôt: 09765996.5
(22) Date de dépôt: 20.05.2009
(51) Int. Cl.: C07H 15/207, A61K 31/7034, A61P 29/00, A61P 31/00, A61P 37/00

(54) **ANALOGUES SYNTHETIQUES DES PHOSPHATIDYL-MYO-INOSITOL MANNOSIDES POURVUS D'UNE ACTIVITE INHIBITRICE DE LA REPONSE INFLAMMATOIRE.**
SYNTHETISCHE ANALOGE VON PHOSPHATIDYL-MYO-INOSITOL-MANNOSIDEN MIT HEMMENDER WIRKUNG AUF ENTZÜNDUNGSREAKTIONEN
SYNTHETIC ANALOGUES OF PHOSPHATIDYL-MYO-INOSITOL MANNOSIDES WITH AN INHIBITORY ACTIVITY ON THE INFLAMMATORY RESPONSE

(30) Priorité: 23.05.2008 FR 0853357
(43) Date de publication de la demande: 09.02.2011
(73) Titulaire: Centre National de la Recherche Scientifique (C.N.R.S), 75016 Paris (FR); Université d'Orleans, 45067 Orleans (FR)
(72) Inventeur: QUESNIAUX RYFFEL, Valerié, F-45560 Saint Denis En Val (FR); MARTIN, Olivier, F-45160 Saint Hilaire Saint Mesmin (FR); FRONT, Sophie, 45560 Saint Denis En Val (FR)
(74) Mandataire: Novagraaf Technologies
(86) Numéro de dépôt international: PCT/FR2009/000595
(87) Numéro de publication internationale: WO 2009/153434

(56) Documents cités:
- EP-A- 0 241 376
- WO-A-03/011336
- WO-A-2005/049631
- WO-A-2008/075983
- FR-A- 2 908 658
- US-A1- 2004 097 465
- AINGE ET AL: "Phosphatidylinositol mannosides: Synthesis and adjuvant properties of phosphatidylinositol di- and tetramannosides" BIOORGANIC & MEDICINAL CHEMISTRY, ELSEVIER SCIENCE LTD, GB, vol. 14, no. 22, 30 octobre 2006 (2006-10-30), pages 7615-7624, XP005839081 ISSN: 0968-0896
- AINGE ET AL: "Phosphatidylinositol mannosides: Synthesis and suppression of allergic airway disease" BIOORGANIC & MEDICINAL CHEMISTRY, ELSEVIER SCIENCE LTD, GB, vol. 14, no. 16, 15 août 2006 (2006-08-15) , pages 5632-5642, XP005534889 ISSN: 0968-0896
- NIGOU J ET AL: "Mannosylated lipoarabinomannans inhibit IL-12 production by human dendritic cells: evidence for a negative signal delivered through the mannose receptor" JOURNAL OF IMMUNOLOGY, AMERICAN ASSOCIATION OF IMMUNOLOGISTS, US, vol. 166, no. 12, 15 juin 2001 (2001-06-15), pages 7477-7485, XP002434004 ISSN: 0022-1767
- G.D. AINGE ET AL.: "Phosphatidylinositol mannoside ether analogues: Synthesis and interleukin-12-inducing properties" J. ORG. CHEM., vol. 72, 2007, pages 5291-5296, XP002513133
- B.S. DYER ET AL.: "Synthesis and structure of phosphatidylinositol dimannoside" J. ORG. CHEM., vol. 72, 2007, pages 3282-3288, XP002513134
- Y. WATANABE ET AL.: "Regiospecific synthesis of 2,6-di-O-(alfa-d-mannopyranosyl)phosphatid yl-d-myo-inositol" J. ORG. CHEM., vol. 61, 1996, pages 14-15, XP002513135
- X. LIU ET AL.: "Total synthesis of phosphatidylinositol mannosides of Mycobacterium tuberculosis" J. AM. CHEM. SOC., vol. 128, 2006, pages 3638-3648, XP002513136
- COTTAZ S ET AL: "PARASITE GLYCOCONJUGATES. PART 3. SYNTHESIS OF SUBSTRATE ANALOGUES OF EARLY INTERMEDIATES IN THE BIOSYNTHETIC PATHWAY OF GLYCOSYLPHOSPHATIDYLINOSITOL MEMBRANE ANCHORS" JOURNAL OF THE CHEMICAL SOCIETY, PERKIN TRANSACTIONS 1, CHEMICAL SOCIETY. LETCHWORTH, GB, no. 13, 1 janvier 1995 (1995-01-01), pages 1673-1676, XP009051535 ISSN: 0300-922X
- STADELMAIER A ET AL: "Synthesis of serine-linked phosphatidylinositol mannosides (PIMs)" EUROPEAN JOURNAL OF ORGANIC CHEMISTRY, WILEY-VCH VERLAG, WEINHEIM, DE, no. 15, 1 janvier 2004 (2004-01-01), pages 3292-3303, XP002419500 ISSN: 1434-193X
- STADELMAIER A ET AL: "Synthesis of phosphatidylinositol mannosides (PIMs)" CARBOHYDRATE RESEARCH, ELSEVIER SCIENTIFIC PUBLISHING COMPANY. AMSTERDAM, NL, vol. 338, no. 23, 14 novembre 2003 (2003-11-14), pages 2557-2569, XP004479528 ISSN: 0008-6215
- ELIE C J J ET AL: "Synthesis of 1-O-(1,2-di-O-palmitoyl-SN-glycero-3-phosp horyl)-2-O-alf a-D-mannopyranosyl-D-myo-inositol: fragment of mycobacterial phospholipids" TETRAHEDRON, ELSEVIER SCIENCE PUBLISHERS, AMSTERDAM, NL, vol. 45, no. 11, 1 janvier 1989 (1989-01-01), pages 3477-3486, XP002176136 ISSN: 0040-4020

## Description

### Domaine technique

La présente invention se rapporte à de nouveaux dérivés synthétiques des phosphatidyl-myo-inositol mannosides (ci-après PIM), leur procédé de préparation et leur utilisation dans la prévention ou le traitement d'une maladie associée à la surexpression de cytokines ou de chimiokines, notamment du TNF et/ou de l'IL-12.

L'invention concerne également une composition pharmaceutique comprenant au moins un dérivé synthétique de PIM.

Dans la description ci-dessous, les références entre crochets [] renvoient à la liste des références présentée à la fin du texte.

### Etat de la technique

L'inhibition de l'expression des cytokines pro-inflammatoires telles que le TNF et l'interleukine 12 (ci-après IL-12) p40 impliquées dans de nombreuses réponses inflammatoires constitue un réel besoin medical. Les indications liées à l'expression de ces cytokines incluent les maladies inflammatoires, allergiques et autoimmunes dont le polyarthrite rhumatoïde, la maladie de Crohn, la sclérose en plaque, le psoriasis, le diabète mellitus, le lupus erythematosus, le choc sceptique et les inflammations pulmonaires chroniques et aigues.

Les molécules classiquement utilisées dans ces indications sont des stéroïdes. Plusieurs agents bloquants le TNF (anticorps ou récepteurs solubles) sont commercialisés depuis 2000. Quatre classes de molécules inhibant la sécrétion d'IL-12 dont des anticorps font l'objet d'un brevet/demande de brevet [1].

Toutefois, les stéroïdes ont des effets secondaires qui en limitent l'application. Les anticorps monoclonaux ou récepteurs solubles sont des protéines coûteuses nécessitant des injections sous-cutanées. De plus, ces anticorps peuvent avoir des effets indésirables sur la résistance aux infections, comme cela a été montré pour les anticorps anti TNF qui peuvent induire la réactivation de la tuberculose latente. Le hyaluronan, décrit comme inhibiteur de la production d'IL-12, a un très haut poids moléculaire, est peu défini moléculairement et difficilement synthétisable [2].

Il subsiste donc un réel besoin médical pour trouver de nouveaux inhibiteurs des cytokines pro-inflammatoires telles que le TNF et l'IL-12 palliant ces défauts.

Les phosphatidyl-*myo*-inositol mannosides (ci-après PIM) sont des précurseurs de lipoglycanes complexes comme, par exemple, les lipoarabinomannanes (LAM) et les lipomannanes (LM), extraits des parois des mycobactéries. Selon leur structure, les PIM peuvent avoir des activités immunomodulatoires différentes.

Les PILAM, provenant d'une espèce avirulente à croissance rapide telle que M. *smegmatis,* sont des molécules pro-inflammatoires stimulant la production du TNF et de IL-12. En revanche, les LAM couverts avec des résidus mannosyle (ManLAM), provenant de myobactéries à croissance lente telles que *M*. *tuberculosis* and *M. bovis* BCG, sont des molécules anti-inflammatoires capables d'inhiber la production d'IL-12 et de TNF et d'augmenter la production d'IL-10 par les cellules dendritiques ou par des lignées de monocytes [8].

Les PILAM stimulent les macrophages par l'intermédiaire de récepteurs Toll-like (TLR)-2, qui sont des récepteurs impliqués dans l'immunité innée, en stimulant la voie de signalisation du NF-kB [9]. Les effets anti-inflammatoires de ManLAM ont été attribués à leur liaison aux récepteurs du mannose [10] ou à DC-SIGN [11].
Les LM, précurseurs biosynthétiques des LAM, sont composés d'un squelette de carbohydrates comportant un noyau constitué de D-mannane et d'un point d'ancrage de mannosyl-phosphatidylinositol (ci-après PIM) à l'extrémité réduite en mannane dudit noyau. Les LM sont dépourvus de domaine D-arabinane et des coiffes trouvées dans les LAM [12]. Les LM sont pro-inflammatoires mais les inventeurs ont récemment décrit que les LM de différentes origines bactériennes, y compris *M. bovis* BCG, *M. tuberculosis, M. chelonae* and *M. kansasii,* présentent également des propriétés anti-inflammatoires importantes [13]. En particulier, les LM tri- et tetra-acylés de *Mycobacterium bovis* BCG induisent la stimulation des macrophages et l'expression de cytokines pro-inflammatoires par l'intermédiaire de TLR2, de TLR4 et de la protéine adaptatrice MyD88 [14], alors que les LM di-acylés et, jusqu'à un certain niveau les LM tri-acylés, inhibent la production des cytokines inflammatoires par les macrophages stimulés à travers la voie TLR4, de manière indépendante de TLR2 [15]. Par conséquent, le degré d'acylation influence l'effet modulateur des LM de *M*. *bovis.* Les auteurs ont proposé que l'acylation différentielle des LM de la paroi myobactérienne peut représenter un moyen additionnel pour réguler la réponse inflammatoire de l'hôte.

Les PIM sont des molécules de faible poids moléculaire (environ 1000-2500) comprenant en général de 1 à 4 chaînes acylées, un résidu phosphatidyl-*myo*-inositol et 1 à 6 résidus mannose. Ainsi, les PIM naturels peuvent se présenter sous différentes formes avec un nombre variable de résidus mannose et acyle.

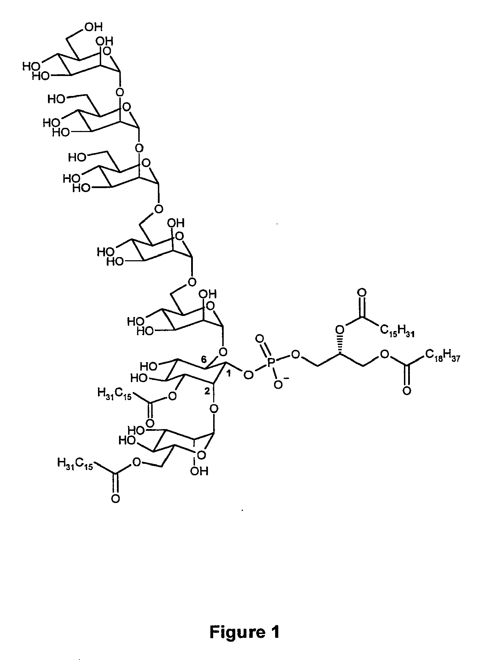

Parmi les PIM naturels, les phosphatidyl-*myo*-inositol dimannoside (ci-après PIM₂) et phosphatidyl-*myo*-inositol hexamannoside (ci-après PIM₆) sont les plus couramment trouvés chez *Mycobacterium bovis* BCG et *Mycobacterium tuberculosis* H37Rv. Un exemple de structure de PIM₆ à l'état naturel est représentée dans la Figure 1. On note que dans ces structures naturelles, les résidus mannosyles sont portés par le D-myo-inositol en positions 2 et 6.

Plusieurs fonctions ont été récemment attribuées aux PIM.

Il a été démontré que les PIM sont des agonistes de TLR2 [3] et sont reconnus par les cellules T CD4⁻CD8⁻ α/β humaines dans le contexte de cellules présentatrices d'antigènes exprimant CD1b [4]. L'interaction de haute affinité entre les protéines CD1b et les chaînes latérales acylées de PIM₂ a été établie [5]. La fraction phosphatidyl-inositol semble jouer un rôle central dans le processus de liaison des PIM aux protéines CD1b. Par ailleurs, il a été démontré que les PIM₂ pouvaient augmenter le recrutement des cellules NKT (Natural Killer T cells), qui jouent un rôle primordial dans la réponse granulomateuse [6], [7].

Il a été trouvé que les souches *Mycobacterium bovis* BCG, *Mycobacterium tuberculosis* H37Rv et *Mycobacterium smegmatis* 607 contenaient essentiellement deux familles de PIM, dimannosylés (PIM₂) et hexamannosylatés (PIM₆) [7]. PIM₁, PIM₃, PIM₄ and PIM₅ ont été observés en faibles quantités suggérant que ce sont des intermediaires biosynthétiques. Les PIM sont synthétisés à partir de phosphatidyl-inositol par addition séquentielle de résidus de mannose à des positions spécifiques. Les trois gènes codant pour les mannosyl transférases impliquées dans l'addition des trois premières unités d'α-Manp sont maintenant connus. L'étape d'initiation, catalysée par l'enzyme pimA [16], consiste à transférer un résidu α-Manp au *myo*-inositol du phosphatidylinositol pour former PIM₁. L'addition d'un second résidu α-Manp au *myo-*inositol du phosphatidyl-inositol pour générer PIM₂ est catalysée par l'enzyme- pimB [17]. L'élongation se fait grâce au pimC [18] pour créer PIM3 par addition d'un troisième résidu α-Manp.

Différentes formes acylées de PIM, en particulier des PIM₂ et PIM₆, ont été purifiés et caractérisées [7]. Quatre formes acylées majeures (mono- au tétra-acylés) ont été décrites pour PIM₂ et PIM₆ (Ac₁-à Ac₄-PIM₂ et PIM₆ ; voir Tableau 1). Leur activité biologique consistant à stimuler les macrophages à produire des cytokines a également été mise en évidence.

**Tableau 1 : Les formes majeures acylées de PIM₆ mis en évidence M. bovis BCG.**

| | m/z | Fragment Acyle | Gro | | Manp | Myo*lns* | % |
|---|---|---|---|---|---|---|---|
| | | | 1 | 2 | 6 | 3 | |
| Ac₁PIM₆ | 1543.6 | C₁₆ | C₁₆ | | | | |
| | 1585.7 | C₁₉ | C₁₉ | | | | |
| Ac₂PIM₆ | 1781.8 | C₁₆, C₁₆ | C₁₆ | C₁₆ | | | 35 |
| | 1823.9 | C₁₆, C₁₉ | C₁₆ | C₁₉ | | | 65 |
| Ac₃PIM₆ | 2062.1 | 2C₁₆, C₁₉ | C₁₆ | C₁₉ | C₁₆ | | 100 |
| Ac₄PIM₆ | 2300.3 | 3C₁₆, C₁₉ | C₁₆ | C₁₉ | C₁₆ | C₁₆ | 56 |
| | 2342.4 | 2C₁₆, 2C₁₉ | C₁₆ | C₁₉ | C₁₆ | C₁₉ | 44 |

L'abondance relative des différentes espèces pour chaque forme acylée a été déterminée à partir de l'intégration des signaux monoisotopiques correspondants [19].

Les inventeurs ont précédemment montré que PIM₂ et PIM₆ induisent une activation faible des macrophages pour sécréter TNF par TLR2 et l'adaptateur MyD88, quelque soit leur structure acylée [7],[19].

Toutefois, comme les PIM sont des précurseurs de LM, et en vue de la récente démonstration par les inventeurs selon laquelle le LM di-acylé et tri-acylé possède un effet anti-inflammatoire fort, ils ont déterminé que certains PIM naturels, y compris les PIM₆ di- et tri-acylés, sont des inhibiteurs de la libération, par les macrophages, de cytokine pro-inflammatoire. Ce travail fait l'objet de la demande de brevet français no. 06/10136 du 20 novembre 2006 publiée sous le numéro FR 2908658.

Les synthèses totales de PIM₂ et de PIM₆ ont été décrites [20]. Les inventeurs ont déjà réalisé la synthèse des PIM₁ di-acylés et ont montré l'inhibition de la libération de cytokines pro-inflammatoires.

Comme indiqué précédemment, un réel besoin médical existe pour trouver de nouveaux inhibiteurs des cytokines pro-inflammatoires telles que le TNF et l'IL-12 palliant aux défauts, inconvénients et obstacles des inhibiteurs de l'art antérieur tels que les stéroïdes ou les anticorps monoclonaux.

Les dérivés synthétiques de PIM peuvent constituer une alternative intéressante pour répondre à ce besoin.

Ainsi, il existe donc un réel besoin de nouveaux dérivés synthétiques des PIM à la fois simple à synthétiser, facile à mettre en oeuvre et de coût réduit, ayant des propriétés inhibitrices de l'expression de cytokines pro-inflammatoires, voire supérieurs, aux inhibiteurs de l'art antérieur.

### Description de l'invention

La présente invention a précisément pour but de répondre à ce besoin en fournissant de nouveaux composés de formule générale (I) : 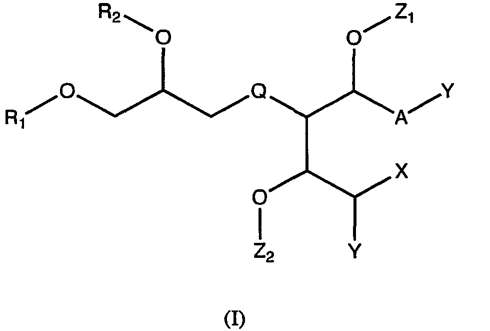 dans laquelle :
■ R₁ et R₂ représentent, indépendemment l'un de l'autre, un atome d'hydrogène, un radical alkyle en C₁-C₂₀, un radical acyle en C₁-C₂₀, étant entendu que lorsque l'un des substituants R₁ ou R₂ est un atome d'hydrogène l'autre substituant est différent d'hydrogène ;
■ Z₁ et Z₂ représentent, indépendemment l'un de l'autre, un atome d'hydrogène, au moins un sucre choisi dans le groupe comprenant le mannose, le glucose, le galactose, étant entendu que lorsque l'un des substituants Z₁ ou Z₂ est un atome d'hydrogène l'autre substituant est différent d'hydrogène ;
■ Q représente -OP(O)₂O-, -OCO₂-, -NHCO₂-, -NHCONH- ;
■ Y représente un atome d'hydrogène, un radical hydroxyl, un radical alkoxy en C₁-C₆, -(CH₂)ₙ-OH avec n est nombre entier égal à 1, 2 ou 3, étant entendu que lorsque Y est un radical hydroxyl, Z₁ et Z₂ ne représentent pas tous les deux un atome d'hydrogène ;
■ A représente -CH₂- ;
■ X représente un atome d'hydrogène ;
■ ou A et X forment ensemble une liaison pour conduire à un cycle à 6 chaînons dans lequel
   - A représente un -CH-,
   - X représente un -CH₂-, -CH(OH)-, un atome oxygène, un
      -NR₃- dans lequel R₃ est un atome d'hydrogène, un radical alkyle en C₁-C₆ ou un radical acyle en C₁-C₂₀,
      étant entendu que, lorsque
   - A et X forment une liaison pour conduire à un cycle à 6 chaînons,
   - X = -CH(OH)-,
   - Y =-OH, et
   - Z₁ et Z₂ représentent, indépendemment l'un de l'autre, au moins un sucre choisi dans le groupe comprenant le mannose, le glucose, le galactose,
      le cycle à 6 membres est de configuration *myo*-inositol avec Z₁ ou Z₂ en position 1 et représentant, au moins un sucre;
ou un de leurs sels pharmaceutiquement acceptables.

Dans le cadre de l'invention, on entend par myo-inositol la structure suivante avec la numérotation indiquée selon les recommandations de l'International Union of Biochemistry (1988).

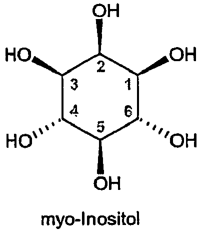

Selon un mode de réalisation de l'invention, les composés de l'invention dans lesquels A et X forment ensemble une liaison pour conduire à un cycle à 6 chaînons, peuvent être de formule (Ia) 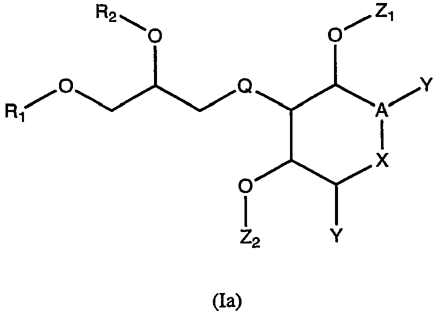 dans laquelle
■ R₁ et R₂, Z₁ et Z₂, Q et Y sont tels que définis précédemment ;
■ A représente un -CH- ;
■ X représente un -CH₂-, -CH(OH)-, un atome oxygène, un -NR₃- dans lequel R₃ est un atome d'hydrogène, un radical alkyle en C₁-C₆ ou un radical acyle en C₁-C₂₀,
étant entendu que, lorsque
- X = -CH(OH)-
- Y = -OH, et
- Z₁ et Z₂ représentent, indépendemment l'un de l'autre, au moins un sucre choisi dans le groupe comprenant le mannose, le glucose, le galactose,
   le cycle à 6 chaînons est de configuration *myo*-inositol avec Z₁ ou Z₂ en position 1 et représentant au moins un sucre ;
ou un de leurs sels pharmaceutiquement acceptables.

Un exemple de ce mode de réalisation peut être un composé dans lequel A et X forment ensemble une liaison pour conduire à un cycle à 6 chaînons, ledit composé pouvant alors être un composé dans lequel
■ R₁ et R₂ représentent, indépendemment l'un de l'autre, un radical acyle en C₁-C₂₀ ;
■ Z₁ représente un atome d'hydrogène;
■ Z₂ représente le mannose;
■ Q représente -OP(O)₂O- ;
■ A représente un -CH- ;
■ X représente un -CH(OH)-
■ Y représente un radical hydroxyl ;
■ le cycle à 6 chaînons est de configuration *myo*-inositol avec Z₁ en position 1 ;
ou un de ses sels pharmaceutiquement acceptables.

Un autre exemple de ce mode de réalisation peut être un composé dans lequel A et X forment ensemble une liaison pour conduire à un cycle à 6 chaînons, ledit composé pouvant alors être un composé dans lequel
■ R₁ et R₂ représentent, indépendemment l'un de l'autre, un radical acyle en C₁-C₂₀ ;
■ Z₁ représente le mannose ;
■ Z₂ représente un atome d'hydrogène ;
■ Q représente -OP(O)₂O- ;
■ A représente un -CH- ;
■ X représente un -CH(OH)-
■ Y représente un radical hydroxyl ;
■ le cycle à 6 chaînons est de configuration *myo*-inositol avec Z₁ en position 1 ;
ou un de ses sels pharmaceutiquement acceptables.

Lorsque A et X forment ensemble une liaison pour conduire à un cycle à 6 chaînons, les composés de l'invention peuvent également être, par exemple, des composés dans lesquels
■ R₁ et R₂, sont tels que définis précédemment ;
■ Q représente -OP(O)₂O- ;
■ A représente un -CH- ;
■ X représente -CH(OH)-,
■ Y représente un radical hydroxyl ;
■ Z₁ et Z₂ représentent, indépendemment l'un de l'autre, au moins un sucre choisi dans le groupe comprenant le mannose, le glucose, le galactose, le cycle à 6 chaînons est de configuration *myo*-inositol avec Z₁ ou Z₂ en position 1 et représentant au moins un sucre ;
ou un de leurs sels pharmaceutiquement acceptables.

Toujours, dans le cas où A et X forment ensemble une liaison pour conduire à un cycle à 6 chaînons, les composés de l'invention peuvent encore être, par exemple, des composés dans lesquels
■ R₁ et R₂, Z₁ et Z₂ et Y sont tels que définis précédemment ;
■ Q représente -OP(O)₂O- ;
■ A représente un -CH- ;
■ X représente un -NR₃- dans lequel R₃ est un atome d'hydrogène, un radical alkyle en C₁-C₆ ou un radical acyle en C₁-C₂₀ ;
ou un de leurs sels pharmaceutiquement acceptables.

Selon un autre mode de réalisation de l'invention, les composés de l'invention peuvent être de formule (Ib) 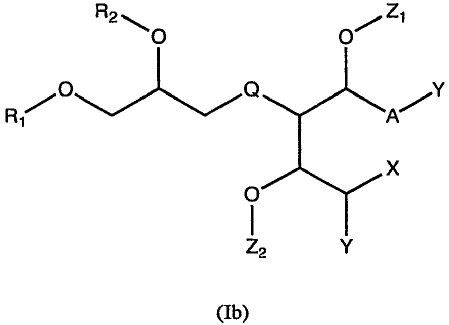 dans laquelle
■ R₁ et R₂, Z₁ et Z₂, Q et Y sont tels que définis précédemment ;
■ A représente -CH₂- ;
■ X représente un atome d'hydrogène ;
ou un de leurs sels pharmaceutiquement acceptables.

De manière tout à fait inattendue, il a été observé que les dérivés synthétiques des PIM conformément à l'invention permettent de séparer la propriété de ces dérivés à agir comme agoniste des récepteurs TLRs impliqués dans la réponse immunitaire, et ainsi de séparer l'activité pro- et anti-inflammatoire de ces nouveaux PIM.

Par ailleurs, les dérivés de PIM selon l'invention sont de faibles poids moléculaires (environ 1000). Ils sont bien définis moléculairement et leur synthèse est plus aisée que les PIM naturels. Ils sont en outre, non cytotoxiques.

De plus, ces dérivés possédent une activité immunomodulatrice au moins comparable, de préférence supérieure à celle des PIM naturels.

On entend par « alkyle » au sens de la présente invention, un radical carboné linéaire, ramifié ou cyclique, saturé ou insaturé, éventuellement substitué, comprenant 1 à 20 atomes de carbone, par exemple 1 à 15 atomes de carbone, par exemple 1 à 6 atomes de carbone. A titre indicatif, on peut citer les radicaux méthyle, éthyle, propyle, butyle, isobutyle, pentyle, hexyle, octyle, nonyle, décyle, undécyle, dodécanyl, tridécanyle, tétradécanyle, pentadécanyle, hexadécanyle, heptadécanyle, octadécanyle octadécanyle, nonadécanyle, eicosanoyle et leurs isomères ramifiés.

On entend par « acyle » au sens de la présente invention, un radical -COR' dans lequel R' est un radical alkyle tel que défini ci-dessus. Le groupe acyle peut également signifier le groupe tuberculostéaryle.

On entend par « alkoxy» au sens de la présente invention, un radical -OR' dans lequel R' est un radical alkyle tel que défini ci-dessus.

Dans le cadre de la présente invention, le terme « sels pharmaceutiquement acceptables » comprend les sels préparés avec des acides ou bases, non toxiques, en fonction des substitituants présents sur les composés. Lorsque les composés de l'invention comportent des fonctions acides, les sels correspondant; peuvent être obtenus par addition d'une base organique ou inorganique sur le composé sous forme neutralisée en présence éventuellement d'un solvant de préférence inerte. Des exemples de sel d'addition d'une base peuvent être les sels de sodium, potassium, calcium, ammonium, amino (organique), ou magnésium. Lorsque les composés de l'invention comportent des fonctions basiques, les sels correspondants peuvent être obtenus par addition d'un acide organique ou inorganique éventuellement dans un solvant de préférence inerte. Des exemples de sels d'addition d'acide inorganique peuvent être les sels hydrochlorique, hydrobromique, nitrique, carbonique, monohydrogencarbonique, phosphorique, monohydrogenphosphorique, dihydrogenphosphorique, sulfurique, monohydrogensulfurique, hydriodique. Des exemples de sels d'addition d'acide organique peuvent être les sels acétique, propionique, isobutyrique, maléique, malonique, benzoïque, succinique, subérique, fumarique, lactique, mandélique, phthalique, benzènesulfonique, p-tolylsulfonique, citrique, tartarique, méthanesulfonique. Sont également couverte par cette invention, des sels d'acides aminés tels que l'arginate, et des sels d'acides organiques tels que les acides glucuronique ou galactunorique.

Les composés de formule générale (I), (Ia) et (Ib) peuvent être sous forme racémique ou enrichie en un énantiomère ou enrichie en un stéréoisomère.

Un des composés préférés de l'invention est le composé de formule (la) de structure suivante (isoPIM₁-C16C18) :

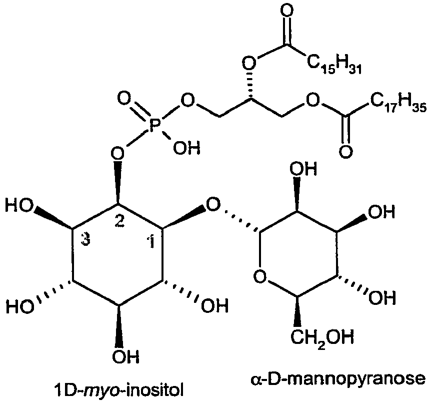

L'invention concerne également le procédé de préparation de composés selon l'invention tels que définis précédemment, dans lequel :
a) on condense un dérivé di-O-acylé ou di-O-alkylé du glycérol de formule (III) 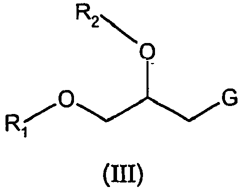 dans laquelle
   - G = OH ou NH₂
   - R₁ et R₂ ont les mêmes définitions que précédemment avec un agent de phosphitylation, de phosphorylation ou de carbonylation pour donner un intermediaire de formule (IV) 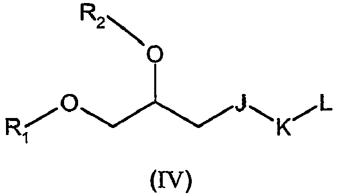 dans laquelle
      - J=O ou NH,
      - K = P-OBn , P(O)-OBn ou C=O,
      - L = groupe sortant
      - R₁ et R₂ ont les mêmes définitions que précédemment ;
b) on condense l'intermédiaire (IV) avec un dérivé d'un polyol ou aminopolyol de formule générale (V) 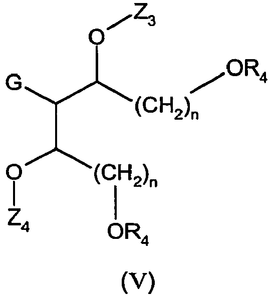 dans laquelle
   - G = OH ou NH₂
   - Z₃ et Z₄ représentent indépendamment l'un de l'autre un hexopyranose de configuration *manno, gluco* ou *galacto* et portant des groupes protecteurs acétyle ou methoxyacétyle, ou un groupement benzyle, étant entendu que l'un au moins des groupes Z₃ et Z₄ représente un sucre protégé
   - R₄ représente un groupe protecteur choisi dans le groupe compenant un groupe benzyle, ou un radical alkoxyacétyle en C₁-C₆,
   ou on condense l'intermédiaire (IV) avec un composé cyclique de formule générale (VI) 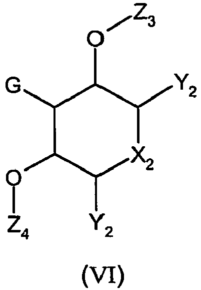 dans laquelle
   - G = OH, NH₂
   - Z₃ et Z₄ ont les mêmes définitions que précédemment
   - Y₂ = H ou un groupe -OR₄ ou un groupe -(CH₂)ₙOR₄ dans lequel R₄ a la même définition que précédemment
   - X₂ = -CH₂, -CHOR₄, -NR₃ ou O dans lequel R₃ est un atome d'hydrogène, un radical alkyle en C₁-C₆ ou un radical acyle en C₁-C₂₀ et R₄ représente un groupe protecteur, comme un groupe benzyle, ou un radical alkoxyacétyle en C₁-C₆
   éventuellement en présence d'un agent de couplage ;
c) on soumet, éventuellement, le composé obtenu à l'étape b) à une réaction d'oxydation, pour donner un composé de formule générale (VII) 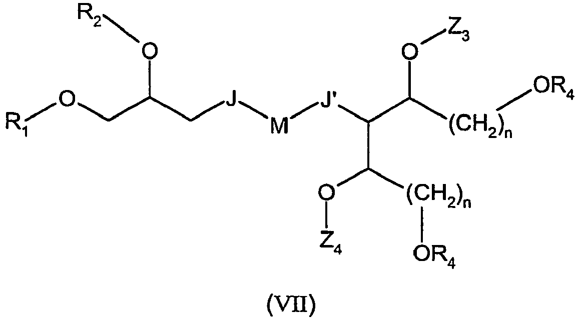 dans laquelle
   - J et J' = O ou NH
   - M = P(O)OBn ou C=O
   - Z₃, Z₄, R₁, R₂, R₄ ont les mêmes définitions que précédemment
   ou un composé de formule générale (VIII) 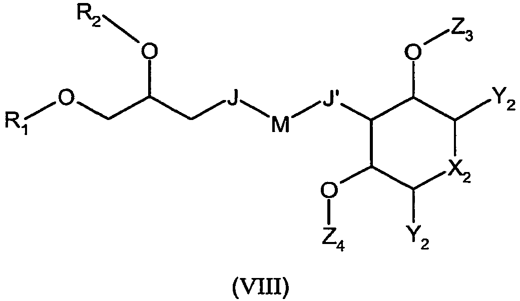 dans laquelle
   - J et J' = O ou NH
   - M = P(O)OBn ou C=O
   - X₂, Y₂, Z₃, Z₄, R₁, R₂, R₄ ont les mêmes définitions que précédemment
d) on soumet le produit de formule générale (VU) ou de formule générale (VIII) à une déprotection en deux étapes qui consiste en ce que l'on les traite d'abord avec une alkylamine choisi dans le groupe comprenant la t-butylamine pour couper sélectivement les groupes acétyles ou méthoxyacétyles puis soumet les produits désacylés à une hydrogénation catalytique pour éliminer les groupements benzyles.

Comme exemple d'agent de phosphitylation, on peut citer la chlorobis(diisopropylamino)phosphine, la benzyloxydichlorophosphine, la bis(benzyloxy)-N,N-diethylaminophosphine et composés apparentés.

Comme exemple d'agent de phosphorylation, on peut citer le diphenylchlorophosphate, le dibenzyl phosphorochloridate, le xylylenechlorophosphate et composés apparentés.

Comme exemple d'agent de carbonylation, on peut citer le carbonyldiimidazole, le triphosgène et les composés apparentés.

Par « groupe protecteur » on entend un groupe qui permet la transformation d'un groupe fonctionnel en un groupe qui sera inerte dans les conditions réactionnelles choisies afin d'empêcher des réactions secondaires de se produire dans la suite de la synthèse. A ce titre on peut citer, par exemple, le groupe benzyle, le groupe t-butyldiméthylsilyle, le groupe acétyle ou le groupe méthoxyacétyle.

Par « groupe sortant ou partant », on entend un groupe susceptible d'être substitué par un réactif nucléophile, comme par exemple, les halogénures ou les tosylates.

Dans le cadre de la présente invention, on entend par « agent de couplage ou agent de liaison », un agent permettant la condensation d'un dérivé d'acide phosphorique ou d'un acide carboxylique avec un alcool ou une amine pour former les liaisons esters ou amides correspondantes. A ce titre on peut citer, par exemple, la dicyclohexylcarbodiimide (DCC), la N-ethyl N-dimethylamino-propylcarbodiimide (EDCI).

Dans un mode de réalisation de l'invention, les composés de formule (I) dans laquelle Q représente -OP(O)₂O-, peuvent également être préparés selon un procédé dans lequel :
a) on condense un dérivé di-O-acylé ou di-O-alkylé du glycérol de formule (IX) 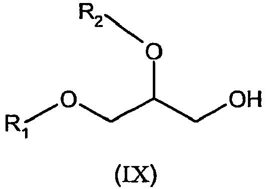 dans laquelle R₁ et R₂ ont les mêmes définitions que précédemment avec un agent de phosphitylation, pour donner un intermédiaire de formule (X) 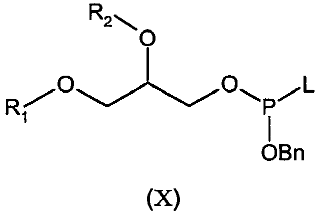 dans laquelle L = groupe sortant
b) on condense alors l'intermédiaire (X) avec un dérivé d'un polyol de formule générale (XI) 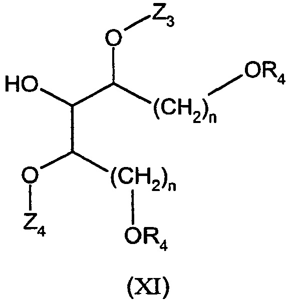 dans laquelle
   - Z₃ et Z₄ représentent indépendamment l'un de l'autre un hexopyranose de configuration *manno, gluco* ou *galacto* et portant des groupes protecteurs acétyle ou methoxyacétyle, ou un groupement benzyle
      avec la condition que l'un au moins des groupes Z₃ et Z₄ represente un sucre protégé
   - R₄ représente un groupe protecteur, comme un groupe benzyle, ou un radical alkoxyacétyle en C₁-C₆
   ou avec un composé cyclique de formule générale (XII) 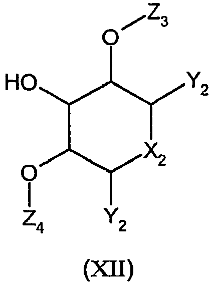 dans laquelle
   - Z₃ et Z₄ ont les mêmes définitions que précédemment
   - Y₂ = H ou un groupe -OR₄ ou un groupe -(CH₂)ₙOR₄ dans lequel R₄ a la même définition que précédemment et n peut prendre des valeurs de 1 à 3
   - X₂ = -CH₂, -CHOR₄, -NR₃ ou O dans lequel R₃ et R₄ ont la même définition que précédemment
   en présence d'un agent de couplage choisi dans le groupe comprenantle 1 H-tetrazole,
c) on soumet ensuite le phosphite intermédiaire à une réaction d'oxydation, pour donner un composé de formule générale (XIII) 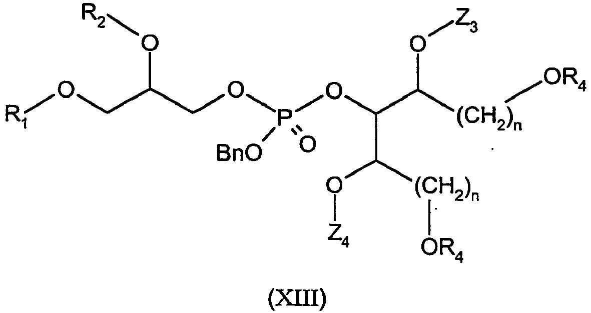 dans laquelle Z₃, Z₄, R₁, R₂, R₄ ont les mêmes définitions que précédemment
   ou un composé de formule générale (XIV) 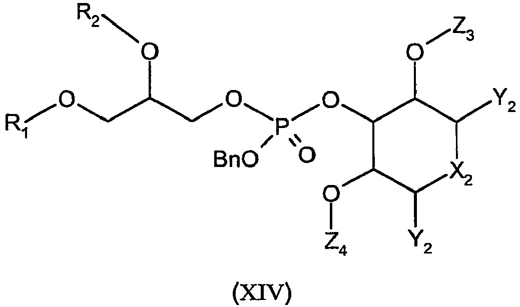 dans laquelle X₂, Y₂, Z₃, Z₄, R₁, R₂, ont les mêmes définitions que précédemment
d) on soumet alors les composés (XIII) et (XIV) obtenus à l'étape c) à une déprotection en deux étapes qui consiste en ce que l'on les traite d'abord avec une alkylamine telle que la t-butylamine pour couper sélectivement les groupes acétyles ou méthoxyacétyles puis soumet les produits désacylés à une hydrogénation catalytique pour éliminer les groupements benzyles.

Un autre objet de la présente invention est une composition pharmaceutique comprenant au moins un composé selon l'invention tel que défini précédemment et tout excipient pharmaceutiquement acceptable.

La composition selon l'invention peut comprendre, en outre, des composants bien connus de l'homme du métier dans le domaine pharmaceutique, comme des agents stabilisants, des agents émulsifiants, des agents de tonicité, des agents conservateurs, des colorants, des excipients, des liants, des lubrifiants notamment.

La composition pharmaceutique selon l'invention peut être utilisée pour la prévention ou le traitement de désordres ou de maladies inflammatoires ou autoimmunes.

Un autre objet de l'invention consiste en l'utilisation d'une composition telle que décrite précédemment pour la fabrication d'un médicament destiné à la prévention ou au traitement d'une maladie associée à la surexpression de cytokines ou de chimiokines, notamment du TNF et/ou de l'IL-12 chez un sujet.

Par sujet, on entend un mammifère, de préférence un humain.

La maladie associée à la surexpression cytokines ou de chimiokines, notamment du TNF et/ou de l'IL-12 comprend:
A) les maladies immunes ou auto-immunes choisies dans le groupe comprenant la polyarthrite rhumatoïde, le diabète sucré, le lupus érythémateux disséminé ou la maladie de Basedow ;
B) le rejet de greffe,
C) les infections virales et/ou parasitaires ;
D) les chocs résultant d'une infection chronique ou aiguë d'origine bactérienne, virale et/ou parasitaire ;
E) les maladies inflammatoires choisies dans le groupe comprenant les maladies inflammatoires chroniques (la sarcoïdose, l'affection abdominale inflammatoire, l'arthrite rhumatoïde, la rectocolite hémorragique, la maladie de Crohn) et les maladies inflammatoires vasculaires (le syndrome de défibrination, l'arhtérosclérose, la maladie de Kawazaki) ;
F) les maladies neurodégénératives choisies dans le groupe comprenant les maladies démyélinisantes (la sclérose en plaques et la myélite transverse aiguë), les maladies extrapyramidales et cérébelleuses (les lésions du système corticospinal ou les désordres des noyaux gris centraux) ;
G) les pathologies malignes impliquant des tumeurs sécrétant du TNF ou impliquant le TNF choisies dans le groupe comprenant la leucémie (aiguë, myélocytique, lymphocytique ou myélodispastique chronique), le lymphome (Hodgkin ou malin (Burkitt) et
H) l'hépatite induite par l'alcool.

L'invention concerne encore l'utilisation d'un composé tel que défini précédemment, pour la fabrication d'un médicament destiné à la prévention ou au traitement d'une maladie associée à la surexpression de cytokines ou de chimiokines, notamment du TNF et/ou de l'IL-12, ladite maladie comprenant :
A) les maladies immunes ou auto-immunes choisies dans le groupe comprenant la polyarthrite rhumatoïde, le diabète sucré, le lupus érythémateux disséminé ou la maladie de Basedow ;
B) le rejet de greffe,
C) les infections virales et/ou parasitaires ;
D) les chocs résultant d'une infection chronique ou aigue d'origine bactérienne virale et/ou parasitaire ;
E) les maladies inflammatoires choisies dans le groupe comprenant les maladies inflammatoires chroniques et les maladies inflammatoires vasculaires ;
F) les maladies neurodégénératives choisies dans le groupe comprenant les maladies démyélinisantes, les maladies extrapyramidales et cérébelleuses ;
G) les pathologies malignes impliquant des tumeurs sécrétant du TNF ou impliquant le TNF choisies dans le groupe comprenant la leucémie, le lymphome ; et
H) l'hépatite induite par l'alcool.

De préférence, ledit médicament est destiné à la prévention ou au traitement d'une maladie inflammatoire chez un sujet.

Ledit médicament peut être administré par injection (intraveineuse, intramusculaire, sous-cutané, intracutané, etc.), administration nasale, orale, percutanée ou par inhalation. En fonction du mode d'administration, ledit médicament peut être préparé sous forme de solutions, d'émulsions, de cachets, de poudres, d'onguents, de lotions, de gels, de suppositoires ou de sprays.

Dans ledit médicament, la concentration en composé de formule (I) ou de son sel pharmaceutiquement acceptable n'est pas limitée et est comprise de préférence entre 0,1 et 100% (p/p) et de manière particulièrement préférée entre 0,5 et 20%.

D'autres avantages pourront encore apparaître à l'homme du métier à la lecture des exemples ci-dessous, illustrés par les figures annexées, donnés à titre illustratif.

### Brève description des figures

- La figure 1 représente une structure de PIM₆ naturel comportant trois groupes acyle dont la chaîne alkyle est linéaire et en C₁₆ et un groupe acyle dont la chaîne alkyle est ramifiée et en C₁₉.
- Les figures 2a, 2b et 2c représentent le schéma synthétique du composé de l'exemple 1 isoPIM₁PIM1-2C₁₆ (SFPIM91; figure 2a), du composé de l'exemple 2 isoPIM₁-C₁₆C₁₈ (SFPIM219; figure 2b) et des composés des exemples 3 et 4 PIM-2-mimNCOCF₃ (SFPIM324-t4) et PIM-2-mimNH (SFPIM324-t8) (figure 2c).
- La figure 3 représente l'inhibition de l'expression de TNF par les macrophages primaires stimulés avec le LPS en présence de la forme « isomère » de PIM₁ isoPIM₁-2C₁₆ (SFPIM91), en comparaison avec les PIM naturels Ac2PIM₆ et Ac3PIM₆ , et le PIM₁ synthétique (SFPIM135), titrés à 1,3 et 10 µg/mL.
- La figure 4 représente l'absence de cytotoxicité de l'isoPIM₁-2C₁₆ (SFPIM91), les PIM naturels Ac2PIM₆ et Ac3PIM₆, et le PIM₁ synthétique (SFPIM135), titrés à 1, 3 et 10 µg/mL.
- La figure 5 représente l'inhibition de l'expression d'IL-6 par les macrophages primaires stimulés au LPS, avec ou sans IFNγ, en présence de l'isoPIM₁-2C₁₆ (SFPIM91), en comparaison avec les PIM naturels Ac2PIM₆ et Ac2PIM₆, et le PIM₁ synthétique (SFPIM135), à 10 µg/mL.
- La figure 6 représente l'inhibition de l'expression d'IL-12 p40 par les macrophages primaires stimulés avec le LPS en présence de l'isoPIM₁-2C₁₆ (SFPIM91), en comparaison avec les PIM naturels Ac2PIM₆ et Ac3PIMₛ, et le PIM₁ synthétique (SFPIM135), à 1µg/mL.
- La figure 7 représente l'inhibition de l'expression de KC par les macrophages stimulés avec le LPS en présence de PIM₁ synthétique (SFPIM135) et de l'isoPIM₁-2C₁₆ (SFPIM91), à 10µg/mL.
- La figure 8 représente l'inhibition par le PIM₁ synthétique (SFPIM135) et l'isoPIM₁-2C₁₆ (SFPIM91) de la réponse des voies respiratoires à une administration locale d'endotoxine. L'abbréviation « AUC » signifie la zone sous la courbe.
- La figure 9 représente l'inhibition par le PIM₁ synthétique (SFPIM135) et l'isoPIM₁-2C₁₆ (SFPIM91) du recrutement de cellules inflammatoires, majoritairement des neutrophiles, dans l'espace broncho-alvéolaire en réponse à l'endotoxine.
- La figure 10 représente l'inhibition par le PIM₁ synthétique (SFPIM135) et l'isoPIM₁-2C₁₆ (SFPIM91) de la sécrétion de chimiokine KC, qui participe au recrutement des neutrophiles, dans_ le lavage broncho-alvéolaire en réponse à l'endotoxine.
- La figure 11 représente l'inhibition par le PIM₁ synthétique (SFPIM135) et l'isoPIM₁-2C₁₆ (SFPIM91) de la sécrétion de TNF dans le lavage broncho-alvéolaire en réponse à l'endotoxine.
- La figure 12 représente l'inhibition de l'expression de TNF par les macrophages primaires stimulés avec le LPS en présence de la forme « isomère » de PIM₁ isoPIM₁-C₁₆C₁₈(SFPIM219), titré à 1, 3 et 10µg/mL.
- La figure 13 représente l'absence de cytotoxicité de l'isoPIM₁-C₁₆C₁₈ (SFPIM219), titré à 1, 3 et 10µg/mL.
- La figure 14 représente l'inhibition de l'expression d'IL-12 p40 par les macrophages primaires stimulés avec le LPS en présence de la forme « isomère » de PIM₁ isoPIM₁-C₁₆C₁₈ (SFPIM219), titré à 1, 3 et 10µg/mL.
- La figure 15 représente l'inhibition de l'expression de TNF par les macrophages primaires stimulés avec le LPS en présence de PIM₁ synthétique (SFPIM145), l'isoPIM₁-C₁₆C₁₈ (PIM219), et les composés PIM-2-mimNCOCF3 (SFPIM324 t2) et PIM-2-mimNH (SFPIM324 t8), titrés à 1, 3, 10 et 30µg/mL.
- La figure 16 représente la viabilité des macrophages primaires stimulés avec le LPS en présence de PIM₁ synthétique (SFPIM145), l'isoPIM₁-C₁₆C₁₈ (PIM219), et les composés PIM-2-mimNCOCF3 (SFPIM324 t2) et PIM-2-mimNH (SFPIM324 t8), titrés à 1, 3, 10 et 30µg/mL.
- La figure 17 représente l'inhibition de l'expression d'IL-12 p40 par les macrophages primaires stimulés avec le LPS en présence de PIM₁ synthétique (SFPIM145), l'isoPIM₁-C₁₆C₁₈ (PIM219), et les composés PIM-2-mimNCOCF3 (SFPIM324 t2) et PIM-2-mimNH (SFPIM324 t8), titrés à 1, 3, 10 et 30 µg/mL.

### EXEMPLES

### Solvants et réactifs

Le dichlorméthane (CH₂Cl₂) et le toluène sont distillés, sous atmosphère d'argon, sur CaH₂, le tétrahydrofurane (THF) est distillé, sous atmosphère d'argon, sur sodium et benzophénone. Le diéthyl éher est distillé, sous atmosphère d'argon, sur CaH₂ et conservé à 0-4°C sous argon sur Tamis moléculaire 4Å. Les autres solvants utilisés proviennent du fournisseur Carlo-Erba.

### Résonance Magnétique Nucléaire (RMN)

Les spectres ¹H et ¹³C sont effectués sur un appareil Bruker DPX250 ou sur un appareil Bruker AV400. Les déplacements chimiques (δ) des spectres RMN ¹H sont calibrés selon le témoin tétraméthylsilane (TMS) ayant pour valeur de δ 0.00 ppm. Les δ des spectres de RMN ¹³C sont calibrés sur la valeur de référence du solvant tel que décrit dans l'article Gottlieb et al, J. Org. Chem., 1997, 62, 7512. Les spectres RMN ³¹P sont réalisés sur un appreil Bruker AV400 et sont calibrés selon une référence externe contenant 80% d'acide phosphorique (H₃PO₄) (δ = 0.00 ppm). Les spectres ¹⁹F sont enregistrés sur un appareil Bruker AV400 équippé d'une sonde multinoyaux automatique et sont calibrés selon une référence externe contenant du BF₃ étherate (δ = 0.00 ppm). Les mesures sont effectuées à 25°C dans des tubes de 5 mm de diamètre.
Le spectres sont effectués dans des solvants deutérés provenant des fournisseur Aldrich ou SDS.

### Chromatographie

Les chromatographies sur couche mince (CCM) sont réalisées sur des plaques d'aluminium « TLC Silica gel 60F₂₅₄ » de Merck . Les composés sont révéles sous lampe UV ou/et sont trempés dans le révélateur à l'acide phosphomolybdique dans l'acide sulfurique et l'éthanol suivi d'un chauffage avec un décapeur thermique.
Les colonnes chromatographiques sont réalisées avec un gel de silice (Silica gel 60 (40-63 µm) de Merck.

### Spectrométrie de masse

Masse ESI : Les échantillons sont analysés sur un spectromètre Perkin Elmer Sciex API 300 dans des solvant de qualité « pour Analyse ». Masse haute résolution : les échantillons sont analysés au centre de mesures physique de l'université Blaise Pascal à Aubière.

### EXEMPLE 1 :

### 1) Synthèse de l'iso PIM₁- 2C₁₆ (SFPIM91):

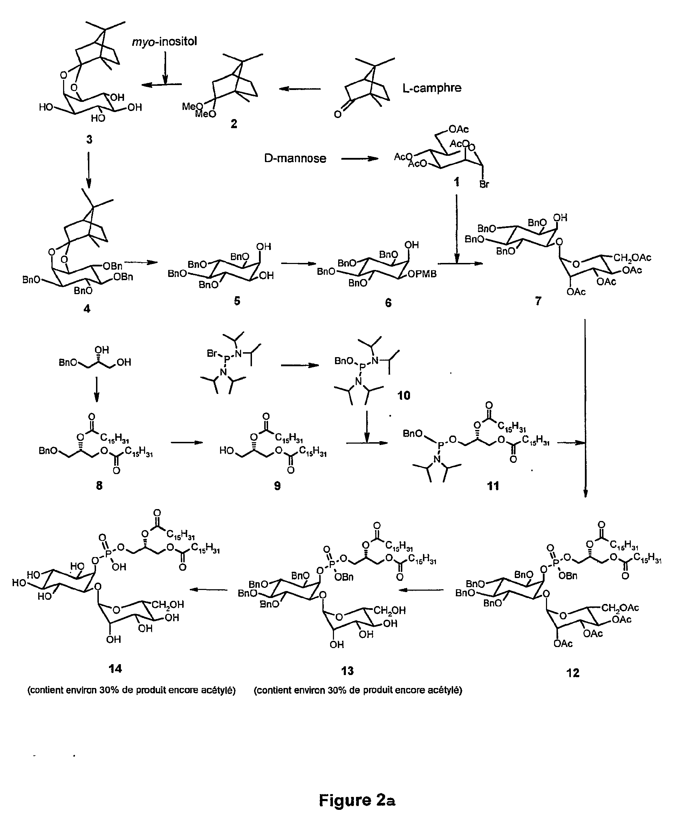

### Diméthyl acétal du L-camphre (composé 2, figure 2a)

### SFPIM-97

Préparation selon Lindberg et al. Tetrahedron, 2002, 58, 1387-1398 [24]. De l'acide sulfurique (H₂SO₄) (194 µL) est ajouté à une solution de L-camphre commercial (>95%, Fluka) (20 g, 0.131 mol) dans un mélange de triméthyl orthoformate (95 mL, 0.908 mol, 6.9 éq) et de méthanol (20 mL). Après 48h d'agitation, le mélange est neutralisé par ajout de méthoxyde de sodium (NaOMe) (400 mg) et les solvants sont évaporés. Le résidu est collecté par distillation sous un vide (25 mbar) à 125°C pour donner le composé 2 (21 g, 81%) sous forme d'un liquide incolore. Spectre de ¹H-RMN conforme.

### 1,2-O-(L-1,7,7-triméthylbicyclo[2,2,1]hept-6-ylidène)-D-myo-inositol (composé 3, figure 2a)

### SFPIM-10

Préparation selon Lindberg et al. Tetrahedron, 2002, 58, 1387-1398 [24]. De l'acide sulfurique H₂SO₄ (173 µL) est ajouté à une solution de composé **2** (8.06 g, 0.041 mol, 2.4 éq) et de *myo*-inositol commercial >99% (Aldrich) (3.1 g, 0.017 mol) dans du diméthylsulfoxide (DMSO) (34 mL). Le mélange résultant est agité 3h à 75°C, puis neutralisé par addition de Et₃N (1 mL) et concentré sous vide à 80°C. Au résidu sont ajoutés du DMSO (3 mL), du chloroforme (CHCl₃) (52 mL), du méthanol (MeOH) (3.2 mL), de l'eau (H₂O) (1 mL), et de l'acide p-toluène sulfonique (APTS) (11.6 mg). Le mélange réactionnel est agité 18h puis neutralisé par addition de Et₃N (0.4 mL). Le précipité ainsi formé est filtré sur verre fritté et lavé avec du CHCl₃ (2 x 40 mL). Le produit brut est recristallisé dans du méthanol (MeOH) (contenant 0.1% NEt₃) pour donner le composé **3** (1.738 g, 32%) sous forme d'un solide blanc. Spectre de ¹H-RMN conforme.

### 3,4,5,6-Tétra-O-benzy)-1,2-O-(L-1,7,7-triméthylbicyclo[2,2,1]hept-6-ylidène)-D-myo-inositol (composé 4, figure 2a)

### SFPIM-72

De l'hydrure de sodium (NaH) (1.16 g, 0.048 mol, 12 eq, 60% en dispersion dans l'huile minérale) est ajouté, sous atmosphère d'argon, à une solution précédemment refroidie à 0°C de composé **3** (1.266 g, 4.02 mmol) dans du DMF anhydre (30 ml). Après 15 min d'agitation à 0°C, du bromure de benzyle (2.9 mL, 0.024 mmol, 6 éq) est ajouté goutte à goutte, puis le mélange réactionnel est agité 24h à température ambiante (environ 20°C). Il est ensuite refroidit à 0°C, puis l'excès de NaH est détruit par addition de MeOH (3 mL) et le milieu est dilué avec du toluène (250 mL). La phase organique est lavée avec de l'eau (100 mL), puis avec une solution de NaCl saturée (3 x 100 mL), puis séchée sur MgSO₄. Les solvants sont évaporés et une purification par colonne chromatogaphique sur gel de silice (Hexane/éther diéthylique 8/1) conduit au composé **4** (2.15 g, 80%) sous forme d'un sirop incolore.
C₄₄H₅₀O₆ (M = 674.89 g/mol).
RMN ¹H (400 MHz, CDCl₃ ) δ 0.86 (s, 3H, CH₃), 0.87 (s, 3H, CH₃), 1.09 (s, 3H, CH₃), 1.22-1.33 (m, 3H), 1.34-1.45 (b, 1 H), 1.47 (s, 0.5H), 1.50 (s, 0.5H), 1.68-1.76 (m, 2H), 1.9-2.02 (m, 2H), 3.44 (dd, 1 H, H5, *J*₅₋₆= 8.2 Hz, *J*₄₋₅= 9.6 Hz), 3.74 (dd, 1H, H4, *J*₄₋₃ = 7.2 Hz), 3.77 (t, 1 H, H1, *J*₁₋₂ = 4.2 Hz), 3.84 (dd, 1 H, H6, *J*₁₋₆ = 8.4Hz), 3.96 (dd, 1H, H3, *J*₂₋₃= 6.2 Hz), 3.44 (dd, 1 H, H5, *J*₅₋₆ = 8.2 Hz, *J*₄₋₅ = 9.6 Hz), 4.30 (dd, 1 H, H2), 7.20-7.45 (m, 20H, 4Ph).
RMN ¹³C (100 MHz, CDCl₃) δ 10.27 (CH₃), 20.52 (CH₃), 20.75 (CH₃), 27.76 (CH₂), 29.89 (CH₂), 45.08 (CH₂), 45.31 (CH), 48.08 (Cq), 51.67 (Cq), 72.55 (CH₂, CH₂Ph), 73.19 (CH, C2), 74.00 (CH₂, CH₂Ph), 75.08(CH₂, CH₂Ph), 75.25 (CH₂, CH₂Ph), 76.32 (CH, C3), 77.50 (CH, C1), 80.86 (CH, C6), 82.22 (CH, C5), 83.31 (CH, C4), 117.77 (Cq), 127.55-128.44 (CH, Ph), 138.55-138.89 (Cq, Ph, 4 lines).
SM-IS: M calculée 674.36; trouvée: 675.5 [M+H]⁺, 692.5 [M+NH₄]⁺, 697.5 [M+Na]⁺, 713.5 [M+K]⁺.

### 3,4,5,6-Tétra-O-benzyl-D-myo-inositol (composé 5, figure 2a)

### SFPIM-17

Le composé **4** (1.53 g, 2.26 mmol) est mis en suspension dans une solution aqueuse d'acide acétique à 80% (75 mL) et le milieu est agité à 100°C pendant 4h. Les solvants sont évaporés sous vide puis coévaporés avec du toluène. Le résidu est purifié par colonne chromatogaphique sur gel de silice (toluène/acétone 35/1 avec un gradient jusqu'à 10/1) pour conduire au composé **5** (1.029 g, 84%) sous forme d'un solide blanc. C₃₄H₃₆O₆ (M = 540.66 g/mol).
RMN ¹H (400 MHz, CDCl₃ ) δ 2.53 (d, 1H, OH-1, *J*_{1-OH} = 4.8 Hz), 2.64 (s, 1 H, OH-2), 3.42-3.51 (m, 3H, H3, H1, H5), 3.83 (dd, 1 H, H6, *J*₆₋₅ = 9.6 Hz, *J*₁₋₆ = 9.6 Hz), 3.97 (dd, 1 H, H4, *J*₃₋₄ = 9.6 Hz, *J*₄₋₅ = 9.6 Hz), 4.17 (dd, 1 H, H2, *J*₂₋₃ = 2.8 Hz, *J*₂₋₁ = 2.8 Hz), 4.65-4.96 (m, 8H, 4 CH₂Ph), 7.25-7.4 (m, 20 H, 4x5 CH_{Ar}).
RMN ¹³C (100 MHz, CDCl₃) δ 69.33 (CH, C2), 71.87 (CH, C1), 72.84 (CH₂, C*H*₂Ph), 75.71 (CH₂, C*H*₂Ph), 75.82 (CH₂, C*H*₂Ph), 76.07 (CH₂, C*H*₂Ph), 80.11 (CH, C3), 81.45 (CH, C6), 81.74 (CH, C4), 83.33 (CH, C5), 127.73-128.67 (CH, Ph, 9 lines), 137.89 (Cq, Ph), 138.62 (2Cq, Ph), 138.73 (Cq, Ph).
MS-IS : M calculée 540.25; trouvée : 541.5 [M+H]⁺, 558.5 [M+NH₄]⁺, 563.5 [M+Na]⁺, 579.5 [M+K]⁺.

### 3,4,5,6-Tétra-O-benzyl-1-O-p-méthoxybenzyl-D-myo-inositol (composé 6, figure 2a)

### SFPIM104

Une suspension de composé **5** (301 mg, 0.557 mmol) et d'oxyde de dibutylétain (139 mg, 0.557 mmol, 1 éq) dans du toluène (5 mL) est chauffée 18h à 140°C sous argon en utilisant un piège de Dean-Stark pour capter l'eau formée. Le milieu est concentré sous vide et le résidu est dissout dans du DMF anhydre contenant du fluorure de césium (CsF) (172 mg, 1.11 mmol) et du chlorure de 4-méthoxybenzyle (76 µL, 0.557 mmol, 1 éq). La réaction est agitée 7h30 et les solvants sont ensuite évaporés à sec pour laisser un résidu solide blanc qui est partiellement dissout dans de l'acétate d'éthyle (20 mL). Le solide résiduel est retiré par filtration sur verre fritté et le filtrat est évaporé à sec. Le résidu est purifié par colonne chromatogaphique sur gel de silice (éther de pétrole/acétate d'éthyle 7/3) pour conduire au composé **6** (262 mg, 71%) sous forme d'un solide blanc. C₄₂H₄₄O₇ (M = 660.81 g/mol).
RMN ¹H (400 MHz, CDCl₃) δ 2.43 (s, 1H, OH), 3.27 et 3.28 (2 dd, 2H, H1 et H3, *J*₁₋₆ = *J*₃₋₄ = 9.6 Hz, *J*₁₋₂ = *J*₂₋₃ = 2.8 Hz), 3.36 (dd, 1 H, H5 , *J*₅₋₆ = *J*₅₋₄ = 9.2 Hz), 3.69 (s, 3H, CH₃), 3.89 et 3.91 (2 dd, 2H, H4 et H6), 4.09 (dd, 1 H, H2, *J*₂₋₃ = *J*₂₋₁ = 2.8Hz), 4.53 (s, 2H, C*H*₂Ph), 4.61 (s, 2H, C*H*₂Ph), 4.72-4.83 (m, 63H, 3 x CH₂Ph), 6.74 et 6.76 (2s, 2H, CH_{Ar} PMB), 7.1-7.3 (m, 23H, CH_{Ar}).
RMN ¹³C (100 MHz, CDCl₃) δ 55.33 (CH₃), 67.59 (CH, C2), 72.44 (CH₂), 72.76 (CH₂), 75.95 (CH₂), 75.98 (2xCH₂), 79.55 et 79.86 (2 CH, C1 et C3), 81.27 et 81.29 (2 CH, C4 et C6), 83.26 (CH, C5), 127.61-129.59 (CH_{Ar}, 8 lines), 130.10 (Cq), 138.05 (Cq), 138.77 (Cq), 138.83 (Cq), 138.88 (Cq), 159.42 (Cq)

### 3,4,5,6-Tétra-O-benzyl-1 -O-(2,3,4,6-tétra-acetyl-α-D-mannopyranosyl)-D-myo-inositol (composé 7, figure 2a)

### SFPIM63

Une solution du compose 6 (457 mg, 0.69 mmol, 1 éq) dans du CH₂Cl₂ (10 mL) est cannulée sous argon sur une solution du composé 1 (845 mg, 2.08 mmol, 3 éq) dans du CH₂Cl₂ (10 mL). Le mélange réactionnel est agité en présence de tamis moléculaire 4Å pendant 20 min, puis est refroidit à -20°C. Le triflate d'argent est ajouté (1.066 g, 4.15 mmol, 6 éq) et le milieu réactionnel est agité en laissant remonter la température à 20°C pendant 1 h. La réaction est neutralisée par addition de triethylamine (1 mL), puis diluée avec CH₂Cl₂. La phase organique est lavée avec une solution saturée de NaHCO₃ (30 mL) puis de NaCl (30 mL) et est séchée sur MgSO₄. Le solvant est évaporé sous vide et le résidu est purifié par colonne chromatogaphique sur gel de silice (éther de pétrole/acétate d'éthyle 3/1) pour conduire au composé 7 (309 mg, 21 %).
C₄₈H₅₄O₁₅ (M = 870.96 g/mol).
RMN ¹H (400 MHz, CDCl₃) δ 7.38-7.20 (m, 20H, CH_{Ar}), 5.43 (dd, 1 H, H3', J_{3'-2'} = 2.6Hz, J_{3'-4'} = 10 Hz), 5.34 (dd, 1 H, H2', J_{2'-1'} = 1 Hz), 5.27 (dd, 1 H, H4', J_{4'-5'} = 10 Hz), 5.07 (d, 1 H, H1', J_{1-2'} = 1 Hz), 4.86 (s, 4H, 2 CH₂Ph), 4.85, (d, 1H, CH₂Ph, J = 10.8 Hz), 4.80 (d, 1H, CH₂Ph, J = 10.8 Hz), 4.75 (d, 1 H, CH₂Ph, J = 11.2Hz), 4.68 (d, 1 H, CH₂Ph, J = 11.2 Hz), 4.29 (m, 3H, H2, H5', H6A'), 4.03 (m, 2H, H6B', H4 or H6), 3.95 (dd, 1 H, J₅₋₄ = J₅₋₆ = 9.6 Hz), 3.54 (dd, 1 H, H1 or H3, J = 2.8 et 10 Hz), 3.45 (dd, 1 H, H4 or H6), 3.43 (dd, 1H, H3 or H1), 2.08 (s, 3H, CH₃ Ac), 2.06 (s, 3H, CH₃ Ac), 2.03 (s, 3H, CH₃ Ac), 2.00 (s, 3H, CH₃ Ac).
RMN ¹³C (100 MHz, CDCl₃) δ 170.74, 169.94, 169.91, 169.72, 138.68, 138.64, 138.33, 137.87, 128.65-127.75, 99.85 (C1'), 83.12, 81.27, 81.07, 80.42, 80.13, 76.28, 76.07, 76.01, 73.15, 69.64 (C2'), 69.54 (C5'), 69.20 (C3'), 68.92 (C2), 66.65 (C4'), 63.16 (C6'), 20.86, 20.78.
MS-IS : M calculée 870.35; trouvée 888.5 [M+NH₄]⁺, 893.5 [M+Na]⁺, 909.0 [M+K]⁺.

### 3-O-Benzyl-1,2-O-dipalmitoyl-sn-glycérol (composé, 8 figure 2a)

### SFPIM77

Le chlorhydrate de [(dimethylamino)propyl]carbodiimide (EDCI) (847 mg, 4.42 mmol, 3 éq) et la DMAP (54 mg, 0.44 mmol, 0.3 éq) sont ajoutés, sous atmosphère d'argon, à une solution de 3-O-benzyl-sn-glycerol (268 mg, 1.47 mmol, 1 éq) et d'acide palmitique (943 mg, 3.68 mmol, 2.5 éq) dans du CH₂Cl₂ anhydre (20 mL). Le mélange réactionnel est agité 18h à température ambiante (environ 20°C) puis dilué avec du CH₂Cl₂ (100 mL). La phase organique est lavée avec une solution d'HCl 1N (50 mL), de l'eau (50 mL), puis une solution de NaCl saturée (50 mL) et séchée sur MgSO₄. Le solvant est évaporé sous vide et le résidu est purifié par colonne chromatogaphique sur gel de silice (éther de pétrole/acétate d'éthyle 6/1) pour conduire au composé 8 (909 mg, 93%) sous forme d'un solide blanc. C₄₂H₇₄O₅ (M = 659.06 g/mol).
RMN ¹H (400 MHz, CDCl₃) δ 0.88 (t, 3H, CH₃), 1.25 (b, 48H, 24 CH₂), 1.59 (b, 4H, 2x2H3"), 2.28 (t, 2H, *J*=7.5Hz), 2.32 (t, 2H, *J*=7.5Hz), 3.59 (d, 2H, 2H3a), 4.19 (dd, 1H, H1a_{A}, *J*_{1aA-1aB} = 12Hz, *J*_{1A-2a} = 6.4Hz), 4.35 (dd, 1 H, H1a_{B}, *J*_{1aB-2a} = 3.6Hz), 4.53 (AB, 2H, CH₂Ph, J = 12.2Hz), 5.24 (m, 1 H, H2a), 7.32 (m, 5H, CH_{Ar}).
MS-IS : M calculée 658.55; trouvée : 660.0 [M+H]⁺, 677.0 [M+NH₄]⁺, 682.0 [M+Na]⁺.

### 1,2-O-Dipalmitoyl-sn-glycérol (composé 9, figure 2a)

### SFPIM82

Le composé 8 (890 mg, 1.35 mmol) est dissous dans un mélange CH₂Cl₂/EtOH (1/1.5, 25 mL). Un large excès de palladium sur charbon (Pd/C 10%) est ajouté et la réaction est agitée 4h à température ambiante (environ 20°C) sous pression atmosphérique d'hydrogène (ballon de baudruche). Le mélange réactionnel est chauffé à 40°C pour une meilleure dissolution du produit attendu puis le catalyseur est retiré par filtration sur membrane millipore. Le solide est rincé 3 fois avec 20 mL de mélange CH₂Cl₂/EtOH (1/1) précédemment chauffé à 40°C. Les solvants résiduels sont évaporés sous vide pour conduire au composé attendu **9** (757 mg, 99%) sous forme de solide blanc.
C₃₅H₆₈O₅ (M = 568.93 g/mol).
RMN ¹H (250 MHz, CDCl₃) δ 0.88 (t, 6H, 2 CH₃, *J* = 6.8 Hz), 1.26 (m, 48H, 24 CH₂), 1.62 (m, 4H, 2H3"), 1.99 (t, 1 H, OH, *J* = 6.5 Hz), 2.32 (t, 2H, *J* = 7.7Hz), 2.35 (t, 2H, *J* = 7.5Hz), 3.73 (dd, 2H, 2H3a, *J* = 5 et 6.2 Hz), 4.23 (dd, 1H, H1a_{A}, *J*_{1aA-1aB} = 12 Hz, *J*_{1A-2a} = 5.5 Hz), 4.35 (dd, 1 H, H1a_{B}, *J*_{1aB-2a} = 4.5Hz), 5.08 (m, 1 H, H2a).
MS -IS : M calculée, 568.51; trouvée: 570.0 [M+H]⁺, 587.0 [M+NH₄]⁺, 592.0 [M+Na]⁺.

### (S)-2,3-Dipalmitoyloxypropyl benzyl (N,N-diisopropylamino)phosphoramidite (composé 11, figure 2a)

### SFPIM83

Le composé **9** (200 mg, 0.352 mmol), précédemment séché 18h sous vide sur P₂O₅, est dissout dans du CH₂Cl₂ anhydre (15 mL) et une solution de bis(*N*,*N*-diisopropylamino)benzyloxyphosphine (0.84M, 938 µL, 0.788 mmol, 2.24 éq) (composé **10**) y est a outée. Le mélange réactionnel est refroidi à 0°C et du 1*H*-tétrazole solide (32 mg, 0.458 mmol, 1.3 éq) est ajouté. Après 1 h30 d'agitation, le solvant est évaporé sous vide et le résidu est purifié par colonne chromatographique sur gel de silice (éther de pétrole/acétate d'éthyle 9/1 contenant 3% de Et₃N) pour conduire au composé **11** (259 mg, 92%) sous forme d'un sirop incolore.
C₄₈H₈₈NO₆P (M = 806.21 g/mol).
RMN ¹H (250 MHz, CDCl₃) δ 0.88 (app t, 6H, 2CH₃), 1.16-1.30 (m, 60H, 24 CH₂, 4xCH₃ isopropyl), 1.60 (m, 4H, 2CH₂), 2.29 (t, 4H, 2CH₂, *J* = 7.5 Hz), 3.55-3.85 (m, 4H, 2CH isopropyl, 2H3a), 4.17 (ddd, 1H, H1a_{B}, *J*_{1aB-1aA} = 12 Hz, *J*_{1aB-2a} = 6.3Hz, *J*_{1aB-P} = 3Hz), 4.34 (ddd, 1 H, H1 a_{A}, *J*_{1aA-2a} = *J*_{1aA-P} = 4.5Hz), 4.71 (m, 2H, CH₂Ph), 5.19 (m, 1 H, H2a), 7.2-7.4 (m, 5H, 5CH_{Ar}).

### 3,4,5,6-Tétra-O-benzyl-1-O-(2,3,4,6-tétra-O-acetyl-α-D-mannopyranosyl)-2-O-[((S)-2,3-dipalmitoyloxypropyl)(benzyl)phosphoryl]-D-myo-inositol (composé 12, figure 2a)

### SFPIM87

Le composé **11** (259 mg, 0.321 mmol, 2.8 éq) et le composé **7** (100 mg, 0.115 mmol) sont coévaporés ensemble avec du toluène anhydre (2 x 10 mL) puis séchés 30 min sous vide poussé avant d'être dissous, sous atmosphère d'argon, dans du CH₂Cl₂ anhydre (12 mL). Du 1*H*-tétrazole solide (26 mg, 0.368 mmol, 3.2 éq) est ajouté à 0°C puis après 1 h30 d'agitation à température ambiante, le mélange réactionnel est refroidi à - 40°C. Une solution d'acide m-chloroperbenzoique (m-CPBA) (50%, 79 mg, 0.230 mmol, 2 éq) dans du CH₂Cl₂ (5 mL) est ajouté goutte à goutte. Après 2h d'agitation en laissant remonter le milieu réactionnel à température ambiante (environ 20°C), la réaction est stoppée par addition d'une solution aqueuse à 10% de Na₂S₂O₃ (65 mL) et le mélange est extrait avec du diéthyl éther (Et₂O) (130 mL). La phase organique est lavée avec une solution aqueuse à 5% de NaHCO₃ (3 x 65 mL) puis séchée sur MgSO₄. Le solvant est évaporé sous vide et le résidu est purifié par colonne chromatogaphique sur gel de silice (éther de pétrole/acétate d'éthyle 4/1) pour donner une fraction du premier *P*-stéréoisomère du composé **12** (42 mg), une fraction de mélange d'isomères (23 mg) et une fraction du deuxième isomère du composé **12** (38 mg) (rendement global 56%).
C₉₀H₁₂₇O₂₂P (M = 1591.88 g/mol).
RMN ¹³C (100 MHz, CDCl₃) δ 173.36, 172.97, 170.72, 170.15, 169.89, 169.53, 138.36, 138.33, 137.90, 137.30, 136.00 (d), 135.67 (d), 128.82-172.08, 83.22, 81.12, 80.89, 78.75 (d), 76.11, 76.07, 76.01, 75.91, 72.99, 69.90, 69.82, 69.39, 69.33, 69.16, 67.45, 67.39, 65.91, 65.43, 62.89, 62.28, 34.33, 34.16, 32.06, 29.84, 29.79, 29.65, 29.50, 29.46, 29.28, 29.27, 24.98, 22.82, 20.96, 20.83, 20.74, 14.25.

### 3,4,5,6-Tétra-O-benzyl-1-O-α-D-mannopyranosyl-2-O-[((S)-2,3-dipalmitoyloxypropyl)(benzyl)phosphoryl]-D-myo-inositol (composé 13, figure 2a)

### SFPIM90

Une solution d'hydroxide de sodium à 0.1 M fraîchement préparée (1 mL, 0.1 mmol, 4 éq) est ajoutée à une solution de composé **12** (40 mg, 0.025 mmol) dans le tétrahydrofurane (THF) (3 mL). Le mélange réactionnel est agité 4h à température ambiante puis il est dilué avec du CH₂Cl₂ (10 mL). La phase organique est lavée avec H₂O puis séchée sur MgSO₄. Le solvant est évaporé pour conduire au composé attendu **13** (36 mg, 100%) Les spectres RMN du produit brut indiquent la présence d'environ 30% de produit encore acétylé.

### 1-O-α-D-mannopyranosyl-2-O-[((S)-2,3-dipalmitoyloxypropyl) phosphoryl]-D-myo-inositol (composé 14, figure 2a)

### SFPIM91

Le composé **13** (36 mg, 0.025 mmol) obtenu ci-dessus est dissous dans un mélange de CH₂Cl₂/EtOH/acide acétique (4/5/0.5 mL). Un large excès de palladium sur charbon (Pd/C 10%) est ajouté et la réaction est agitée 6h à température ambiante (environ 20°C) sous pression atmosphérique d'hydrogène (ballon de baudruche). Le catalyseur est retiré par filtration sur membrane millipore et rincé 3 fois avec 20 mL de mélange CH₂Cl₂/EtOH (1/1). Les solvants résiduels sont évaporés sous vide et coévaporés avec du toluène pour conduire au composé **14** (25 mg, 100%) sous forme de solide blanc.

### 2) Préparation des cultures primaires de macrophages :

Des cellules murines de moelle osseuse ont été obtenues à partir de fémurs de lignées de souris sauvages. Les cellules obtenues ont été cultivées (10⁶/ml) pendant 7 jours dans un milieu DMEM (Dulbecco's modified Eagle's medium) complémenté avec 20% de sérum de cheval et 30% de milieu cellulaire conditionné L929 [22]. Trois jours après renouvellement du milieu, la préparation cellulaire comprend une population homogène de macrophages.

### 3) Stimulation des macrophages de souris sauvages par le LPS en présence et en l'absence d'isomère de PIM isoPIM₁-2C₁₆(SFPIM91)

Les macrophages dérivés de la moelle osseuse de souris sauvages ont été cultivés sur des plaques de culture 96 puits à raison de 10⁵ cellules par puits puis stimulés par du LPS (100 ng/ml, *Escherichia. coli,* sérotype O111:B4, SIGMA) avec ou sans PIM analogue (1-10 µg/ml). Toutes les préparations de PIM analogue lyophilisées utilisées sont solubilisées dans du DMSO et additionnées aux cultures à une concentration finale maximale non cytotoxique de 1%.

Après une stimulation de 24 heures, les sumageants de culture ont été collectés et analysés pour leur contenu en cytokines TNF-α, IL-6, IL-12p40 et KC par ELISA (DUOSET R&D) et pour leur contenu en nitrite par la réaction de Griess.

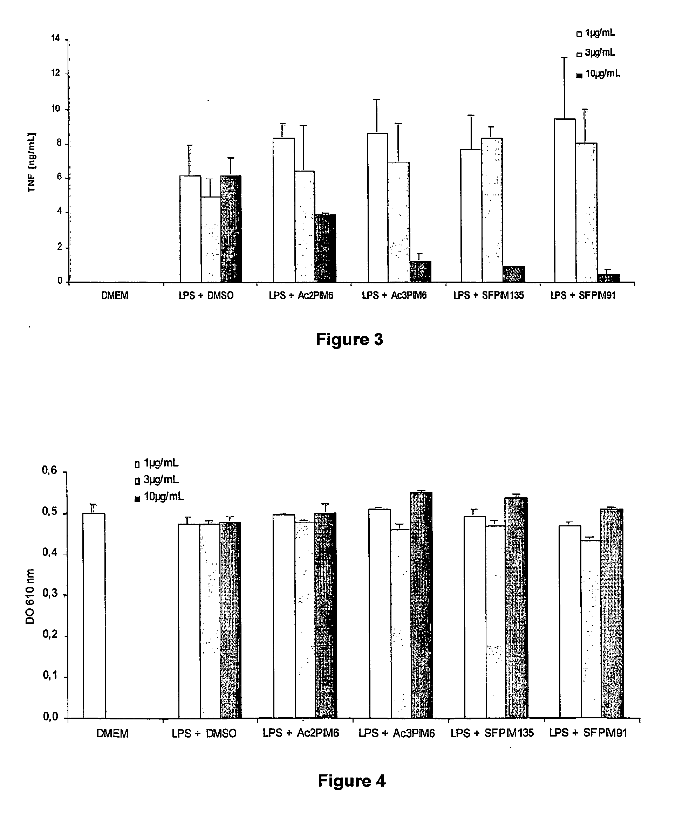

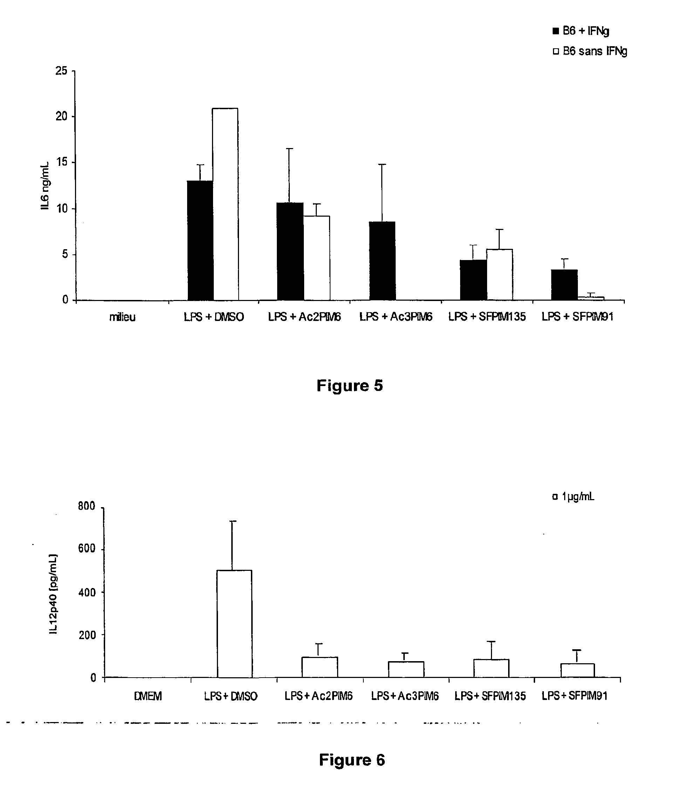

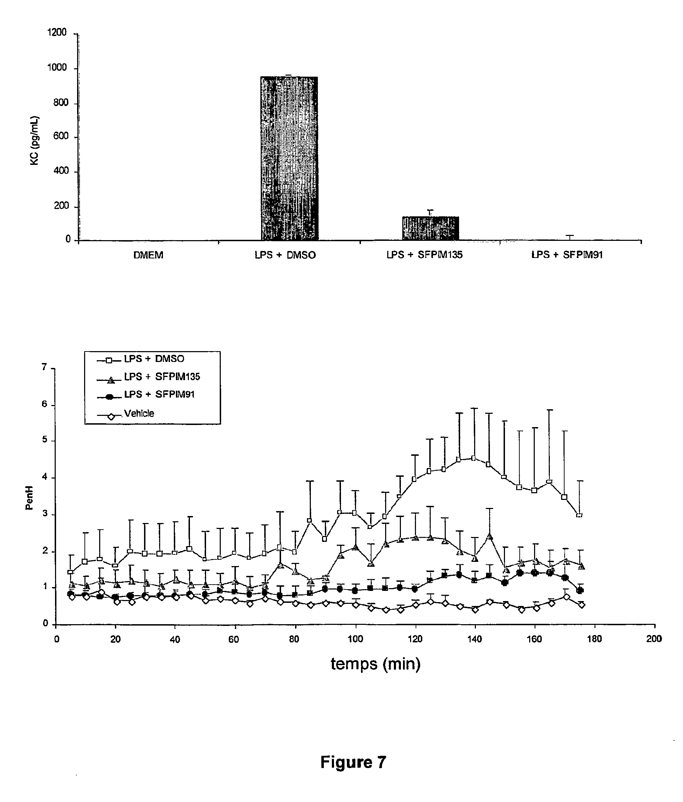

Les résultats montrent que la forme « isomère » de PIM₁ isoPIM₁-2C₁₆ (SFPIM91) inhibe fortement la synthèse de TNF-α induite chez les macrophages stimulés au LPS, en comparaison avec les PIM naturels Ac2PIM₆ et Ac2PIM₆, et le PIM₁ synthétique (SFPIM135) (figure 3). Des résultats similaires ont été obtenus pour le NO. Un test de cytotoxicité MTT réalisé sur les mêmes macrophages en présence des différentes fractions de PIM a permis de montrer l'absence de cytotoxicité des différentes préparations pour les cellules (figure 4). L'expression de l'IL-6 par les macrophages stimulés au LPS en présence ou en absence d'Interféron γ est aussi inhibée (figure 5). La sécrétion d'IL-12p40 en réponse au LPS est déjà fortement inhibée à des concentration de 1ug/mL de PIM ou d'isoPIM (figure 6). De même, la production de chimiokine KC, impliquée dans le recrutement des cellules inflammatoires comme les neutrophiles, est aussi fortement réduite en présence de PIM₁ (SFPIM135) ou plus fortement d' isoPIM₁-2C₁₆ (SFPIM91) (figure 7).

Dans la mesure où des préparations de PIM₂ et de PIM₆ ont été identifiées initialement comme étant des stimulateurs de la sécrétion de TNF et d'IL-12p40 par des cultures primaires de macrophages, les préparations d'isomère de PIM ont été testées pour leur capacité à induire une réponse pro-inflammatoire à des concentrations jusqu'à 20 µg/ml. Les résultats obtenus n'ont montré aucune stimulation de la réponse inflammatoire (TNF-α et IL-12p40) des cultures primaires de macrophages induite par les préparations d'isomère de PIM.

### 4) Activité anti-inflammatoire in vivo des dérivés synthétiques de PIM

### Modèle in vivo de détresse respiratoire

Les souris C57BU6 ont reçu le véhicule seul (saline avec 1.25% DMSO) ou du LPS (1 µg par souris) *d'Escherichia coli* (serotype O111:B4; Sigma, St Louis, MO, USA) en l'absence ou en présence de PIM₁ (SFPIM135) ou d'isoPIM₁-2C₁₆ (SFPIM91) (50µg/souris), appliqués par instillation nasale dans un volume de 40 µL sous une anesthésie légère à la kétamine-xylasine.

La résistance des voies respiratoires a été évaluée par la plethysmographie non-invasive sur une période de 3 heures après l'application de LPS. Des souris vigiles ont été placées dans des chambres de plethysmographie (EMKA Technologies, Paris, France). L'augmentation de la pause respiratoire (Penh) en tant que mesure d'inconfort respiratoire a été enregistrée et analysée en utilisant le logiciel. "Datanalyst. Software (EMKA Technologies, Paris, France) et exprimée en moyenne ± SEM de Penh de n=2-3 souris individuelles par groupe [23].

### Lavage bronchoalvéolaire (BAL)

Le fluide BAL est collecté en canulant la trachée et en lavant les poumons quatre fois avec 0.5 mL de PBS froid. Après centrifugation à 400 x g pendant 10 min à 4°C, le surnageant du premier lavage est conservé à -70°C pour l'analyse des cytokines. Des pools de culots cellulaires sont comptés au bleu Trypan (Sigma) dans une cellule haematocytométrique. Pour le comptage différentiel, les cellules sont marquées avec Diff-Quik Staining (Merz & Dade AG., Dudingen, Switzerland). Deux fois cent cellules sont comptées.

### Détermination des cytokines

Les concentrations de TNF et de KC ont été évaluées par enzyme-linked immunosorbent assay (ELISA) conformément aux instructions du fabricant (R&D Duoset, Minneapolis, MO).

### Résultats

Le TNF est essentiel pour le dysfonctionnement respiratoire aigu induit par le LPS comme cela a été montré chez les souris déficientes en TNF [23]. Afin de déterminer leur activité potentielle *in vivo,* le PIM₁ (SFPIM135) et l'isoPIM₁-2C₁₆ (SFPIM91) ont été testés pour leur activité inhibitrice dans un modèle murin d'inflammation pulmonaire aigue et de détresse respiratoire induites par l'application intranasale de LPS (1 µg/souris).

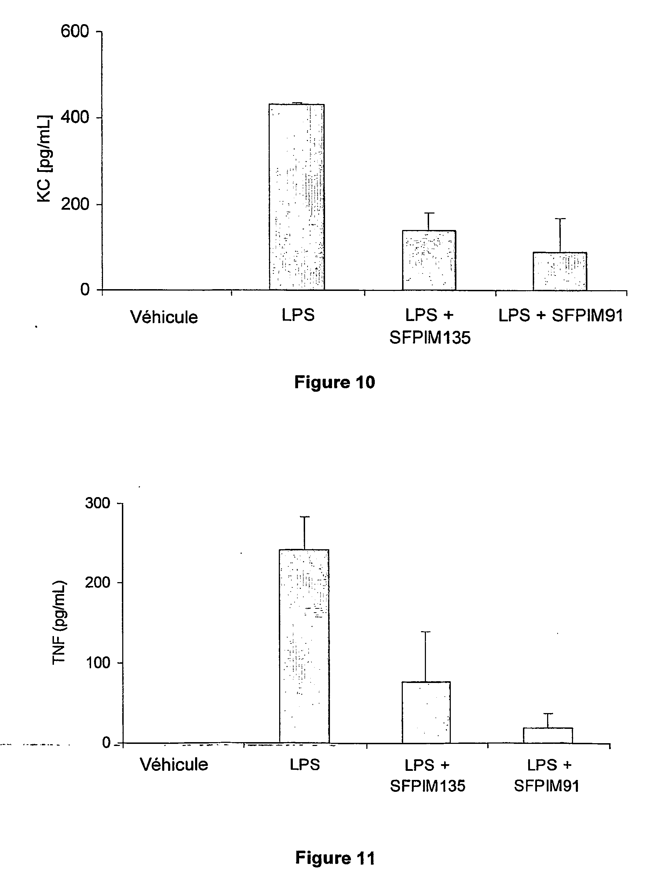

Les souris C57BU6 ont reçu une application par voie intranasale de 1 µg de LPS et Penh a été enregistré pour 200 minutes utilisant la plethysmographie non-invasive. Le graphique de la figure 11 représente l'aire sous la courbe (de 70 à 175 min). Les valeurs représentent la moyenne ± SEM de n=2-3 souris_par groupe.

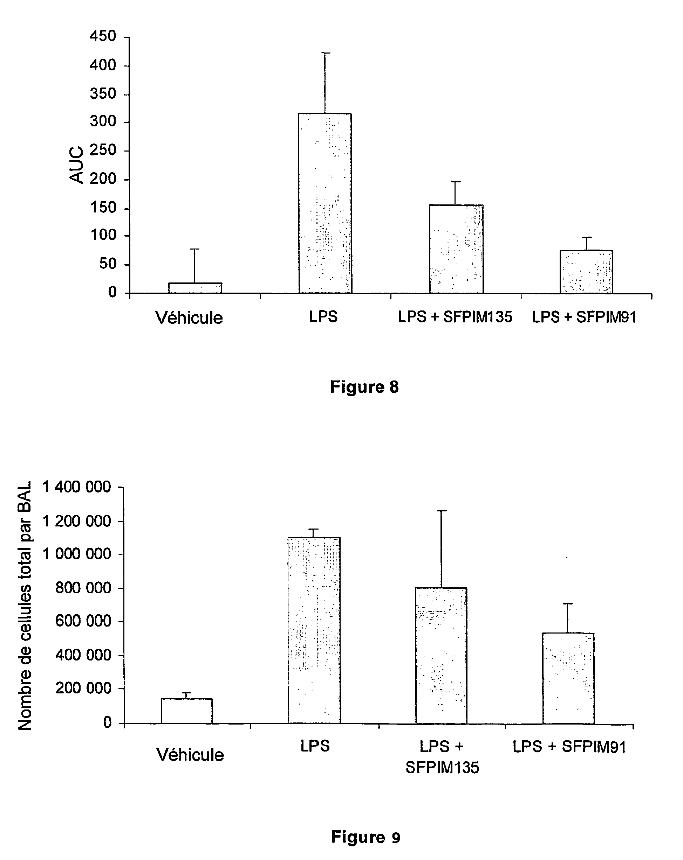

Typiquement, les souris recevant une application intranasale de LPS développent une augmentation aiguë de Penh (accroissement de la pause repiratoire ou enhanced respiratory pause), mesure du dysfonctionnement respiratoire, débutant 90 minutes après l'application de LPS (figure 8). L'addition de PIM₁ (SFPIM135) cause une réduction partielle dans l'accroissement de Penh induit par le LPS. Cette inhibition est encore plus prononcée après l'addition de l'isoPIM₁-2C₁₆ (SFPIM91) (figure 8).

Le LPS provoque un recrutement des cellules inflammatoires dans les voies respiratoires, mesuré en nombre total de cellules dans le fluide bronchoalvéolaire (BAL) des souris traitées par le LPS. Ici, le nombre de cellules inflammatoire détecté dans le BAL 18 heures après l'application de LPS, est partiellement réduit en présence d'isoPIM₁-2C₁₆ (SFPIM91), et moins en présence de PIM₁ (SFPIM135) (figure 9). Les neutrophiles constituent la plupart des cellules inflammatoires recrutées après exposition au LPS. Les macrophages sont essentiellement inchangés. Dans cette première expérience une réduction du nombre des neutrophiles jusqu'à 50 ou 70 % est observée dans 2 souris sur 3 ayant reçu le PIM₁ (SFPIM135) ou l'isoPIM₁-2C₁₆ (SFPIM91), respectivement.

Le recrutement des neutrophile dépend de différents facteurs dont la chimiokine KC, même s'il n'est pas dépendant de manière critique du TNF [23]. Dans la figure 10, les souris traitées sont sacrifiées 24 heures après l'application intranasale de 1 µg de LPS et le fluide bronchoalvéolaire est analysé pour déterminer le contenu en chimiokine KC. La sécrétion de KC dans l'espace broncho-alvéolaire est fortement diminuée par la co-administration de PIM₁ (SFPIM135) ou d'isoPIM₁-2C₁₆ (SFPIM91) (figure 10). De même, le relargage de TNF dans l'espace broncho-alvéolaire est diminuée par la co-administration de PIM₁ (SFPIM135) ou plus fortement par l'isoPIM₁-2C₁₆ (SFPIM91) (figure 11). Les valeurs représentent la moyenne ± SEM de n=3 souris par groupe, d'une expérience représentative de 3 expériences indépendantes.

Des expériences complémentaires indiquent que l'isoPIM₁-2C₁₆ (SFPIM91) cause une diminution d'expression de plusieurs autres cytokines et chimiokines inflammatoires dans les poumons exposés aux endotoxines.

Dans ces expérience l'isoPIM₁-2C₁₆ (SFPIM91) cause une inhibition marquée de l'inflammation pulmonaire et de la résistance des voies respiratoires en réponse aux endotoxines locales.

### EXEMPLE 2 :

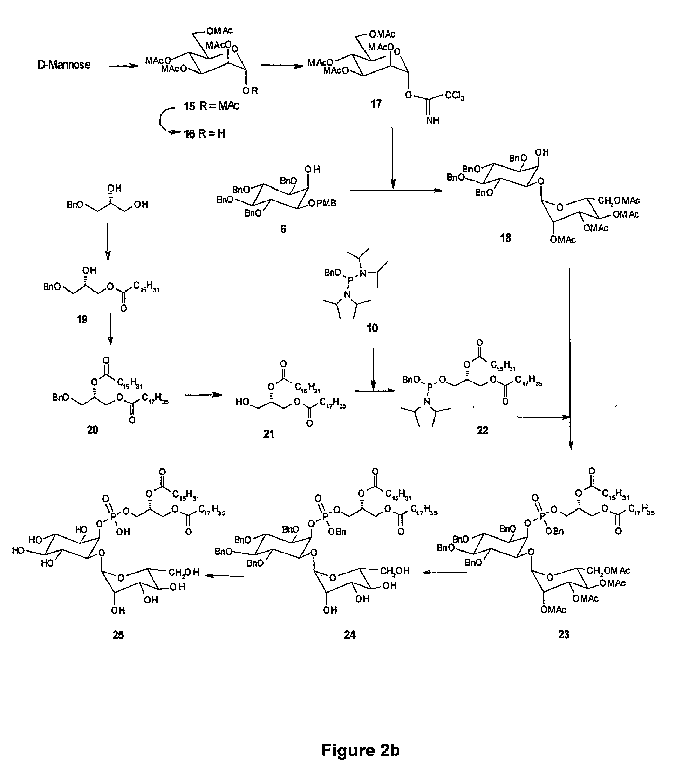

### 1) Synthèse du composé 25 (IsoPIM1-C₁₆C₁₈)(Figure 2b)

### Per-O-méthoxyacétyl-D-mannopyranose (composé 15, figure 2b)

### SFPIM-93

Le chlorure de méthoxyacétyle (660 µl, 7.22 mmol, 6.5 éq) est ajouté goutte à goutte à une solution de D-Mannose (200 mg, 1.11 mmol) dans de la pyridine (6 ml) à température ambiante (environ 20°C) et le mélange est agité 18h. Les solvants sont évaporés et le résidu est dilué dans de l'acétate d'éthyle (30 mL). La solution est ensuite lavée avec une solution aqueuse de HCl 1N (10 mL), puis une solution de NaCl saturée (10 mL) et séchée sur sulfate de magnésium (MgSO₄). Les solvants sont évaporés et une purification par colonne chromatographique sur gel de silice (éther de pétrole/acétate d'éthyle 2/1 puis 1/1) conduit au composé **15** pur (518 mg, 86%) sous forme d'un sirop jaune.
C₂₁H₃₂O₁₆ (M = 540.47 g/mol).
RMN ¹H (250 MHz, CDCl₃) δ 3.41 (s, 6H), 3.45 (s, 3H), 3.49 (s, 6H), 3.85-4.26 (m, 14H), 5.25-5.53 (m, 3H), 6.21 (s, 1 H) ;
RMN ¹³C (62.9 MHz, CDCl₃) δ 59.34, 59.42, 59.50, 61.83, 65.50, 68.35, 68.82, 69.14, 69.26, 69.37, 70.44, 90.49, 167.61, 169.08, 169.22, 169.33, 169.90.
MS IS : M calculée 540.17; trouvée : 558.5 [M+NH₄]⁺, 563.5 [M+Na]⁺, 579.5 [M+K]⁺.

### 2,3,4,6-Tétra-O-méthoxyacétyl-D-mannopyranose (composé 16, figure 2b)

### SFPIM-114

De l'acétate d'hydrazine (500 mg, 5.48 mml, 1.6 éq) est ajouté à une solution du composé **15** (1.850 g, 3.4 mmol) dans du diméthyl formamide (DMF) (15 mL) précédemment refroidie à -20°C. Après 1h d'agitation à - 20°C, le mélange est dilué avec de l'acétate d'éthyle (150 mL), puis lavé avec une solution de NaCl saturée (5 x 50 mL), séché sur MgSO₄ et concentré sous vide pour donner le composé **16** (1.331 g, 83%) sous forme d'un sirop jaune.
C₁₈H₂₈O₁₄ (M = 468.42 g/mol).
RMN ¹H (400 MHz, CDCl₃) δ 3.34 (s, 3H,), 3.35 (s, 3H), 3.39 (s, 3H), 3.41 (s, 3H)(4 OMe), 3.87 (AB, 2H, C*H*₂OMe), 3.95 (s, 2H, C*H*₂OMe), 4.02 (s, 2H, C*H*₂OMe), 4.08 (AB, 2H, C*H*₂OMe), 4.2-4.35 (m, 3H, H5', H6'A, H6'B), 5.19 (d, 1H, H1', *J*₁₋₂ = 1.8 Hz), 5.27 (t, 1 H, H4', *J*₄₋₃ = 10 Hz), 5.3 (dd, 1 H, H2', *J*₂₋₃ = 3.2 Hz), 5.49 (dd, 1H, H3').
RMN ¹³C (100 MHz, CDCl₃ ) δ 59.45 (CH₃), 59.48 (CH₃), 59.50 (CH₃), 62.62 (C6'), 66.53 (CH₂, C4'), 68.00 (CH, C5'), 69.29 (CH, C3'), 69.34 (CH₂), 69.4 1 (CH₂), 69.49 (CH₂), 69.52 (CH₂), 70.50 (CH, C2'), 91.98 (CH, C1').

### 2,3,4,6-Tétra-O-méthoxyacetyl-1-O-trichloroacétimidoyl-α-D-manno-pyranose (composé 17, figure 2b)

### SFPIM-125

Du trichloroacétonitrile (1.28 mL, 12.80 mmol, 12 éq) est ajouté à un mélange de composé **16** (500 mg, 1.067 mmol) et de 1,8-diazabicyclo[5.4.0]undec-7-ène (DBU) (45 µL, 0.299 mmol, 0.28 éq) dans du dichlorométhane (CH₂Cl₂) anhydre (10 mL) à température ambiante (20°C) sous une atmosphère d'argon. Après 10 min d'agitation, le mélange réactionnel est purifié par colonne chromatographique sur gel de silice (éther de pétrole/acétate d'éthyle 1/2 contenant 0.2% de triéthylamine (Et₃N)) et conduit ainsi au composé **17** (479 mg, 73%) sous forme d'un sirop jaune.
C₂₀H₂₈O₁₄NCl₃ (M = 614.81 g/mol).
RMN ¹H (250 MHz, CDCl₃) δ 3.41 (s, 3H, CH₃), 3.42 (s, 3H, CH₃), 3.45 (s, 3H, CH₃), 3.50 (s, 3H, CH₃), 3.95 (dd, 2H, C*H*₂OMe), 4.03 (s, 2H, C*H*₂OMe), 4.08 (s, 2H, C*H*₂OMe), 4.19 (dd, 2H, C*H*₂OMe), 4.22-4.33 (m, 2H, H5',H6'A), 4.40 (dd, 1H, *J* = 5.3 et 13.3 Hz, H6'B), 5.45 (t, 1 H, J = 9.8 Hz, H4'), 5.55 (dd, 1 H, J = 5.2 et 10.0 Hz, H3'), 5.59 (dd, 1 H, H2'), 6.32 (d, 1 H, *J* = 1.5 Hz, H1'), 8.84 (s, 1 H, NH).

### 3,4,5,6.Tétra-O-benzyl-1.O-(2,3,4,6-tetra-méthoxyacetyl-α-D-mannopyranosyl)-D-myo-inositol (composé 18, figure 2b)

### SFPIM-207

Le composé accepteur **6** (940 mg, 1.42 mmol, 1 éq) et le composé donneur **17** (1.16 g, 1.89 mmol, 1.3 éq) sont rassemblés dans un même ballon et sont placés sous vide, sur P₂O₅ pendant 18h. Le mélange est ensuite placé sous argon et du tamis 4Å y est ajouté. Le ballon est laissé sous flux d'argon pendant 10 min, puis du CH₂Cl₂ anhydre (7 mL) est ajouté. Après 30 min supplémentaires d'agitation sous argon, le milieu réactionnel est refroidi à 0°C et le TMSOTf (68 µL, 0.38 mmol) est ajouté goutte à goutte. Après 5 min d'agitation à 0°C et 1h à température ambiante (environ 20°C) sous atmosphère d'argon, le mélange réactionnel est refroidit à 0°C et la réaction est stoppée par addition de Et₃N (1 ml). Le tamis est filtré sur verre fritté, les solvants sont évaporés sous vide et le résidu est purifié par colonne chromatogaphique sur gel de silice (éther de pétrole/acétate d'éthyle 1/1) pour conduire au composé **18** (296 mg, 21%). (On isole également une fraction de 350 mg du composé **18** en mélange avec l'isomère initialement attendu ayant perdu le PMB mais glycosylé en position 2 de l'inositol)
C₅₂H₆₂O₁₉ (M = 991.06 g/mol).
RMN ¹H (400 MHz, CDCl₃) δ 2.89 (m, 1 H, OH), 3.34 (s, 3H, CH₃), 3.41 (s, 3H, CH₃), 3.42 (s, 3H, CH₃), 3.44 (s, 3H, CH₃), 3.41-3.48 (m, 2H, 2CHᵢₙₛ), 3.45 (dd, 1 H, CHᵢₙₛ, J = 2.4, 6.0 Hz), 3.86-4.16 (m, 8H, 4CH₂OMe), 4.15 (dd, 1 H, H6'A, J_{6'A-5'} = 2.4 J_{6'A-6'B} = 12 Hz), 4.29 (br s, 1 H, H2), 4.34 (dd, 1 H, H6'B, J_{6'B-5'} = 4.8 Hz), 4.43 (ddd, 1 H, H5'), 4.65-4.91 (m, 8H, 4CH₂Ph), 5.07 (d, 1 H, H1', J_{1'-2'} = 2 Hz), 5.31 (t, 1H, H4', J_{4'-3} = J_{4'-5'} = 10.0 Hz), 5.41 (dd, 1H, H2', J_{2'-3} = 3.2 Hz), 5.56 (dd, 1H, H3'), 7.15-7.4 (m, 20H, 4x5CH_{Ar}).
RMN ¹³C (62.9 MHz, CDCl₃ ) δ 59.42, 59.48, 59.52, 63.12 (C6'), 66.65 (4'), 68.83 (C5'), 69.26 (C2), 69.38 (C3'), 69.46 (2CH₂OMe), 69.49 (CH₂OMe), 69.74 (CH₂OMe), 69.87 (C2'), 73.17 (CH₂Ph), 76.0(CH₂Ph), 76.03(CH₂Ph), 76.28(CH₂Ph), 80.27, 80.31, 81.15, 81.53 et 83.12 (CHᵢₙₛ), 99.47 (C1'), 127.69-128.62 (11 pics, CH_{Ar}), 137.88, 138.26, 138.57, 138.63, 169.25 (CO), 169.31 (CO), 169.49 (CO), 169.86 (CO).

### 3-O-Benzyl-1-O-octadëcanoyl-sn-glycerol (composé 19, figure 2b)

### SFPIM1

La (diméthylamino)pyridine (DMAP) (5.6 mg, 0.05 mmol, 0.1 éq) puis le dicyclohexylcarbodümide (DCC) (190 mg, 0.92 mmol, 2 éq) sont ajoutés à une solution, précédemment refroidie à 0°C, d'acide stéarique (130 mg, 0.46 mmol) et de 3-O-benzyl-sn-glycerol (100 mg, 0.55 mmol, 1.2 éq) dans du dichlorométhane anhydre (CH₂Cl₂) (5 mL). Le mélange réactionnel est agité 1h à 0°C puis 18h à temperature ambiante (environ 20°C) puis est filtré sur cotton afin de retirer une partie de la dicyclohéxylurée formée. Le solvant est évaporé sous vide et le résidu est purifié par colonne chromatogaphique sur gel de silice (éther de pétrole/acétate d'éthyle 6/1) pour conduire au composé **19** (129 mg, 63%) sous forme d'un solide blanc.
C₂₈H₄₈O₄ (M = 448.69 g/mol)
RMN ¹H (250 MHz, CDCl₃) δ 0.88 (t, 3H, CH₃, *J* = 6.5 Hz), 1.25 (b, 28H, 14CH₂), 1.60 (m, 2H, 2H3"), 2.31 (t, 2H, 2H2", *J* = 7.5 Hz), 2.64 (d, 1 H, OH, *J* = 3.5 Hz), 3.48 (dd, 1H, H3a_{A}, *J*_{3aA-3aB} = 9.75Hz, *J*_{3aA-2a} = 6 Hz), 3.55 (dd, 1H, H3a_{B}, *J*_{3aB-2a} = 4.5 Hz), 4.02 (m, 1 H, H2a), 4.12 (dd, 1 H, H1a_{A}, *J*_{1aA-2a} ~ 4.8 Hz), 4.19 (dd, 1H, H1a_{B}, *J*_{1aB-1aA} = 11.5Hz, *J*_{1aB-2a} 5 Hz), 4.55 (s, 2H, CH₂Ph), 7.33 (m, 5H, CH_{Ar}).
RMN ¹³C (62.9 MHz, CDCl₃ ) δ 14.22 (CH₃), 22.19 (CH₂), 25.00 (CH₂, C3"), 29.23, 29.36, 29.46, 29.66, 29.71, 29.76, 29.80 (7 CH₂), 32.02 (CH₂), 34.24 (CH₂, C2"), 65.43 (CH₂, C1a), 68.80 (CH, C2a), 71.00 (CH₂, C3a), 73.58 (CH₂Ph), 127.82 (CH_{Ar}), 127.94 (CH_{Ar}), 128.54 (CH_{Ar}), 137.77 (Cq_{Ar}), 174.01 (Cq, C1").
MS-IS : calculée 448.36; trouvée : 449.5 [M+H]⁺, 466.5 [M+NH₄]⁺, 471.5 [M+Na]⁺.

### 3-O-Benzyl-2-O-hexadecanoyl-1-O-octadécanoyl-sn-glycerol (composé 20, figure 2b)

### SFPIM34

Le chlorhydrate de 1-éthyl-3-[3-(dimethylamino)propylcarbodiimide (EDCI) (662 mg, 3.45 mmol, 2.5 éq) et la DMAP (34 mg, 0.28 mmol, 0.2 éq) sont ajoutés à une solution du composé **19** (620 mg, 1.38 mmol) et d'acide palmitique (708 mg, 2.76 mmol, 2 éq) dans du CH₂Cl₂ anhydre (25 mL). Le mélange réactionnel est agité 18h à température ambiante (environ 20°C) puis dilué avec CH₂Cl₂ (100 mL). La phase organique est lavée avec une solution d'HCl 1 N (40 mL), de l'eau (40 mL), puis une solution de NaCl saturée (40 mL) et séchée sur MgSO₄. Le solvant est évaporé sous vide et le résidu est purifié par colonne chromatogaphique sur gel de silice (éther de pétrole/acétate d'éthyle 25/1 puis 20/1) pour conduire au composé **20** (899 mg, 95%) sous forme d'un solide blanc.
C₄₄H₇₈O₅ (M = 687.11 g/mol)
RMN ¹H (250 MHz, CDCl₃ ) δ 0.88 (t, 3H, CH₃, *J* = 6.5 Hz), 1.26 (m, 52H, 26CH₂), 1.60 (m, 4H, 2H3', 2H3"), 2.27 (t, 2H, *J* = 7.5. Hz) et 2-31 (t, 2H, *J* = 7.5 Hz)(2H2' et 2H2"), 3.59 (d, 2H, 2H3a, *J* = 5 Hz), 4.19 (dd, 1H, H1a_{A}, *J*_{1aA-1aB} = 11.75 Hz, *J*_{1A-2}a = 6.3 Hz), 4.35 (dd, 1 H, H1_{aB}, *J*_{1aB-2a} = 3.8 Hz), 4.53 (AB, 2H, CH₂Ph, *J* = 12.2Hz), 5.24 (quint., 1H, H2a), 7.32 (m, 5H, CH_{Ar}).
RMN (100 MHz, CDCl₃) δ 14.23 (CH₃), 22.82 (CH₂), 25.01 et 25.08 (2CH₂, C3', C3''), 29.22-29.83 (CH₂, 8 lines), 32.06 (CH₂), 34.23 et 34.44 (2CH₂), 62.16 (CH2, C1a), 68.38 (CH₂, C3a), 70.12 (CH, C2a), 73.42 (CH₂Ph), 127.72 (CH_{Ar}), 127.87 (CH_{Ar}), 128.51 (CH_{Ar}), 137.83 (Cq_{Ar}), 173.16 (C_{q}, CO), 173.45 (Cq, CO).
SM-IS M calculée : 686.58; trouvée : 687.5 [M+H]⁺, 704.5 [M+NH₄]⁺, 709.5 [M+Na]⁺.

### 2-O-Hexadecanoyl-1-O-octadécanoyl-sn-glycerol (composé 21, figure 2b)

### SFPIM29

Le composé **20** (250 mg, 0.36 mmol) est dissous dans un mélange CH₂Cl₂/EtOH (1/2.5, 14 mL). Un large excès de palladium sur charbon (Pd/C 10%) est ajouté et la réaction est agitée 4 h à température ambiante (environ 20°C) sous pression atmosphérique d'hydrogène (ballon de baudruche). Le mélange réactionnel est chauffé à 30°C pour une meilleure dissolution du produit attendu et le catalyseur est retiré par filtration sur membrane millipore. Il est rincé 3 fois avec 20 mL de mélange CH₂Cl₂/EtOH (1/1) précédemment chauffé à 30°C. Le solvants résiduels sont évaporés sous vide pour conduire au composé **21** (215 mg, 99%) attendu sous forme de solide blanc.
C₃₇H₇₂O₅ (M = 596.98 g/mol).
RMN ¹H (250 MHz, CDCl₃ ) δ 0.88 (t, 6H, 2CH₃), 1.26 (m, 52H, 26CH₂), 1.62 (m, 4H), 1.99 (s très large, 1 H, OH), 2.32 (t, 2H, *J* = 7.7 Hz), 2.34 (t, 2H, *J* = 7.5 Hz), 3.73 (d, 2H, 2H3a, *J* = 5 Hz), 4.23 (dd, 1H, H1a_{A}, *J*_{1aA-1aB} = 11.75 Hz, *J*_{1A-2a} = 5.75 Hz), 4.35 (dd, 1H, H1a_{B}, *J*_{1aB-2a} = 4.5 Hz), 5.08 (quint, 1H, H2a, J = 4.5 Hz).
RMN ¹³C (62.9 MHz, CDCl₃) δ 14.27 (CH₃), 22.84 (CH₂), 25.04 et 25.09 (2CH₂), 29.25-29.85 (CH₂, 8 lines), 32.08 (CH₂), 34.26 et 34.44 (2CH₂), 61.68, 62.16, 72.25, 173.57 (Cq, CO), 173.93 (Cq, CO).

### (S)-2-O-Hexadecanoyloxy-1-O-octadécanoyloxypropyl benzyl (N,N-diisopropylamino)phosphoramidite (composé 22, figure 2b)

### SFPIM47

Du 1H-tetrazole solide (11 mg, 0.151 mmol, 0.6 éq) et le composé **21** (150 mg, 0.251 mmol) sont séparément séchés sous vide sur P₂O₅ pendant 1 h avant d'être rassemblés et dissous dans le CH₂Cl₂ anhydre (2 mL). La solution stock de composé **10** (0.84M, 358 µL, 0.301 mmol, 1.2 éq) est ajoutée et après 30 min d'agitation à température ambiante (environ 20°C), le mélange réactionnel est dilué avec du CH₂Cl₂ (50 mL), refroidi à 0°C et la phase organique est lavée avec une solution saturée de NaHCO₃ à 0°C (10 mL), puis séchée sur MgSO₄. Le solvant est évaporé et une purification rapide sur colonne chromatographique de gel de silice (éther de pétrole/acétate d'éthyle 6/1 contenant 1 % Et₃N) permet d'obtenir le composé attendu **22** (100 mg, 48%) sous forme d'une huile incolore.
C₅₀H₉₂NO₆P (M = 834.27 g/mol).
RMN ¹H (400 MHz, CDCl₃ ) δ 0.88 (t, 6H, 2CH₃), 1.18 (d, 6H, CH₃ isopropyl), 1.19 (d, 6H, CH₃ isopropyl), 1.25 (m, 52H, 26 CH₂), 1.60 (m, 4H, 2CH₂), 2.29 (2t, 4H, 2CH₂),3.56-3.9 (2 m, 4H, 2CHMe₂, 2H3a), 4.17 (ddd, 1H, H1a_{B}, J = 4.8, 6.4 et 12 Hz), 4.34 (ddd, 1H, H1a_{A}, J = 4, 8 et 12 Hz), 4.65 et 4.66 (2 dd, 1 H, POCH_{A}Ph, J_{A,B} = 12.4 Hz, J_{A,P} = 8.4 Hz), 4.732 et 4.735 (2 dd, 1 H, POCH_{B}Ph, J_{A,B} = 12.4 Hz, J_{A,P} = 8.4 Hz), 5.19 (m, 1 H, H2a), 7.3-7.4 (m, 5H, 5CH_{Ar}).
³¹P NMR (162 MHz, CDCl₃) δ 148.70, 148.80.

### 3,4,5,6-Tétra-O-benzyl-1-O-(2,3,4,6-tetra-O-methoxyacetyl-α-D-mannopyranosyl)-2-O-[((S)-2-O-hexadecanoyl-3-O-octadécanoyl-propyl)(benzyl)phosphoryl)-D-myo-inositol (composé 23, figure 2b)

### SFPIM214

Le composé **22** (290 mg, 0.348 mmol, 3 éq) et le composé **18** (115 mg, 0.116 mmol) sont coévaporés ensemble avec du toluène anhydre (2 x 10 mL) puis séchés 30 min sous vide poussé avant d'être dissous, sous atmosphère d'argon, dans du CH₂Cl₂ anhydre (8 mL). Le 1H-tetrazole solide (41 mg, 0.58 mmol, 5 éq) est ajouté à 0°C puis après 1h30 d'agitation à température ambiante, le mélange réactionnel est refroidi à - 40°C. Une solution d'acide m-chloroperbenzoique (m-CPBA) (50%, 120 mg, 0.348 mmol, 3éq) dans le CH₂Cl₂ (8 mL) est ajoutée goutte à goutte. Après 2h d'agitation en laissant remonter le milieu réactionnel à température ambiante (environ 20°C), la réaction est stoppée par addition d'une solution aqueuse à 10% de Na₂S₂O₃ (50 mL) et le mélange est extrait avec du diéthyl éther (Et₂O) (100 mL). La phase organique est lavée avec une solution aqueuse à 5% de NaHCO₃ (3 x 50 mL) puis séchée sur MgSO₄. Le solvant est évaporé sous vide et le résidu est purifié par colonne chromatogaphique sur gel de silice (éther de pétrole/acétate d'éthyle 2/1 avec un gradient jusqu'à 1/1) pour donner une fraction d'un premier P-stéréoisomère du composé **23** (55 mg) et une fraction de mélange d'isomères (76 mg contenant environ 30% du premier isomère) (rendement global 65%).
C₉₆H₁₃₉O₂₆P (M = 1740.14 g/mol).
SM-IS *m*/*z* M calculée : 1738.92; trouvée : 1-40.5 [M+H]⁺, 1763.5 [M+Na]⁺. HRMS calculée pour [M+H]⁺ : C₉₆H₁₄₀O₂₆P : 1739.9370 ; trouvée : 1739.9386.

### 3,4,5,6-Tétra-O-benzyl-1-O-α-D-mannopyranosyl-2-O-[((S)-2-O-hexadecanoyl-3-O-octadécanoyl-propyl)(benzyl)phosphoryl)-D-myo-inositol (composé 24, figure 2b)

### SFPIM218

Le composé 23 (129 mg, 0.074 mmol) est dissout dans un mélange de CHCl₃/MeOH (4/1, 1 mL). Le mélange réactionnel est refroidi à 0°C et de la t-butylamine (160 µL) y est ajoutée. Après 10 min d'agitation à 0°C puis 1h en laissant le milieu réactionnel remonter à temperature ambiante, les solvants sont évaporés sous vide poussé à temperature ambiante (environ 20°C) et le résidu est purifié par deux colonnes chromatogaphique successives sur gel de silice (CH₂Cl₂/MeOH 20/1) avec une grande quantité de silice pour donner le composé attendu **24** (67 mg, 63%) sous forme d'un solide blanc homogène (RMN : présence des deux *P**-stéréoisomères).
C₈₄H₁₂₃O₁₈P (M = 1415.85 g/mol).
HRMS calculée pour [M+H]⁺ : C₈₄H₁₂₄O₁₈P : 1451.8525; trouvée: 1451.8521.

### 1-O-α-D-Mannopyranosyl-2-O-[((S)-2-O-hexadecanoyl-3-O-octa-décanoylpropyl)phosphoryl]-D-myo-inositol (composé 25, figure 2b)

### SFPIM219

Le composé 24 (63 mg, 0.043 mmol) est dissous dans un mélange de CH₂Cl₂/EtOH (0.6/1, 16 mL). Un large excès de palladium sur charbon (Pd/C 10%) est ajouté et la réaction est agitée 18h à température ambiante (environ 20°C) sous pression atmosphérique d'hydrogène (ballon de baudruche). Le catalyseur est retiré par filtration sur membrane millipore et rincé 3 fois avec un mélange CH₂Cl₂/EtOH (1/1) (20 ml). Les solvants résiduels sont évaporés sous vide pour conduire au composé **25** (43 mg, 100%) sous forme de solide blanc très homogène (RMN).
C₄₉H₉₃O₁₈P (M = 1001.25 g/mol).
RMN ¹H (400 MHz, CD₃OD/CDCl₃ 0.7/0.3 mL) δ 0.88 (t, 6H, 2CH₃), 1.26 (m, 52H, 26 CH₂), 1.60 (m, 4H), 2.32 (t, 2H, *J* = 7.6 Hz), 2.35 (t, 2H, *J* = 7.6 Hz), 3.22 (t, 1 H, H5, J₄₋₅ = J₅₋₆ = 8.8Hz), 3.52 (br d, 1 H, H3), 3.55 (t, 1 H, H4', J_{4'-5'} = 10.0 Hz), 3.56 (t, 1 H, H4, J₄₋₃ = 8.8 Hz), 3.66 (m, 1H, H1), 3.67-3.72 (m, 2H, H6, H6'A), 3.81 (dd, 1 H, H3', J_{3'-4'} = 9.4, J_{3'-2'} = 3.2 Hz), 3.83 (m, 1 H, H5'), 3.89 (dd, 1 H, H6'B , J_{6'B-5'} = 2, J_{6'B}-_{6'A} = 11.6 Hz), 3.97 (dd, 1 H, H2', J_{2'-1'} = 1.6 Hz), 4.16-4.23 (m, 3H, H3aA et 2H1a), 4.42 (dd, 1 H, H3aB, J_{3aB-2a} = 3.2, J_{3aB-3aA} = 12 Hz), 4.75 (ddd, 1 H, H2, J₂₋₃ ~ 10, J₂₋₁ = 2.2 Hz), 5.16 (d, 1 H, H1', J = 1.2 Hz), 5.26 (m, 1 H, H2a).
RMN ¹³C (100 MHz, CD₃OD/CDCl₃ 0.7/0.3 mL) δ 14.40 (CH₃), 23.43, 25.67, 25.70, 29.90, 29.93, 30.15, 30.16, 30.33, 30.36, 30.44, 30.46, 30.48, 32.74, 34.75, 34.88, 62.83 (C6'), 63.23 (C3a), 65.87 (d, C1a), 68.77 (C4'), 70.89 (d, C2a), 71.48 (C3 et C2'), 73.58 (C4), 73.96 (C6), 74.70 (C5'), 75.79 (C5), 76.37 (d, C1), 81.37 (d, C1), 102.95 (C1'), 174.43 (CO), 174.81 (CO).
³¹P NMR (162 MHz, CD₃OD/CDCl₃ 0.7/0.3 mL) δ -1.71 ppm.
HRMS calculée pour [M+H]⁺ : C₄₉H₉₄O₁₈P: 1001.6178 ; trouvée: 1001.6172.

### 2) Stimulation des macrophages de souris sauvages par le LPS en présence d'isomère de PIM₁ isoPIM₁-C₁₆C₁₈ (SFPIM219)

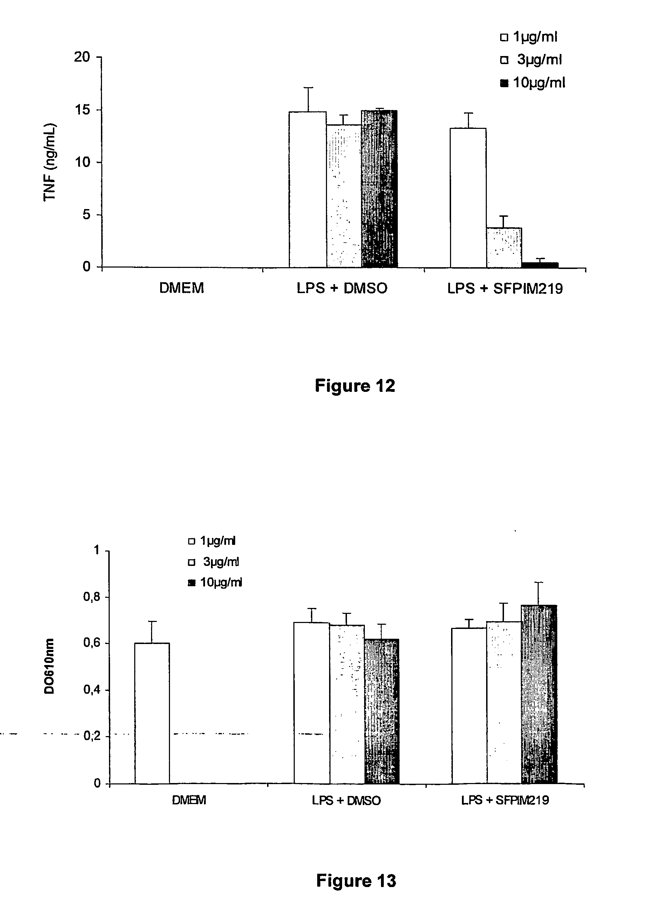

Un nouvel isomère de PIM, l'isoPIM₁C₁₆C₁₈ (SFPIM219) a été testé pour son activité inhibitrice sur les macrophages stimulés par du LPS (figure 12).

Les macrophages dérivés de la moelle osseuse de souris sauvages ont été cultivés sur des plaques de culture 96 puits à raison de 10⁵ cellules par puits puis stimulés par du LPS (100 ng/ml, *Escherichia. coli*, sérotype O111:B4, SIGMA) avec l'isoPIM₁C₁₆C₁₈ (SFPIM219) (1-10 µg/ml) ou un contrôle DMSO. La préparation de SFPIM219 lyophilisée utilisée est solubilisée dans du DMSO et additionnée aux cultures à une concentration finale maximale non cytotoxique de 1 %.

Après une stimulation de 24 heures, les surnageants de culture ont été collectés et analysés pour leur contenu en cytokines TNF-α ou IL-12 p40 par ELISA (R&D Duoset, Minneapolis, MO). Les résultats correspondent à la moyenne +SD de n=2 souris par génotype.

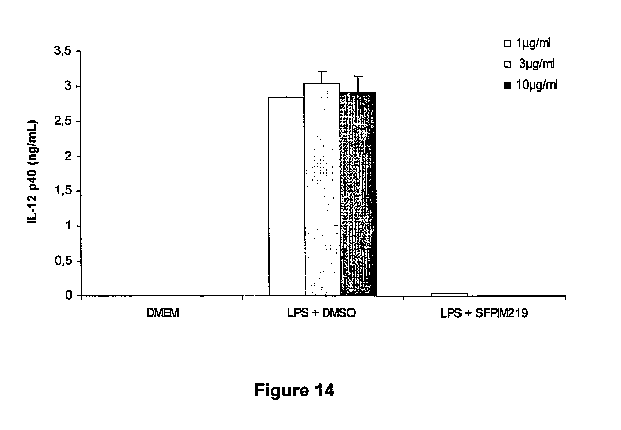

L'isoPIM₁C₁₆C₁₈ (SFPIM219) inhibe fortement la sécrétion de TNF (figure 12). Un test de cytotoxicité MTT réalisé sur les mêmes macrophages incubés en présence d'isoPIM₁C₁₆C₁₈ (SFPIM219) a permis de montrer son absence de cytotoxicité sur les macrophages (figure 13). La sécrétion d'IL-12p40 en réponse au LPS est déjà fortement inhibée à des concentration de 1ug/mL d'isoPIM₁C₁₆C₁₈ (SFPIM219) (figure 14).

### EXEMPLES 3 et 4 :

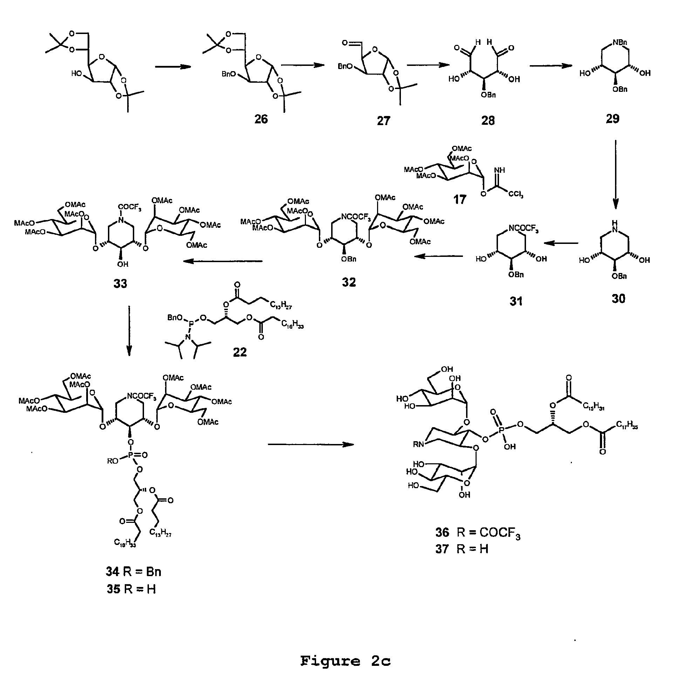

### 1) Exemple 3 : Synthèse du composé 36 de la figure 2c ou PIM-2-mimNCOCF₃

### SFPIM324-t2

### Exemple 4: Synthèse du composé 37 de la figure 2c ou PIM-2mimNH

### SFPIM324-t 8

### 3-O-Benzyl-1,2:5,6-di-O-isopropylidène-α-D-glucofuranose (composé 26, figure 2c)

### SFPIM311

A une solution de 1,2:5,6-di-*O*-isopropylidène-α-D-glucofuranose commerciale (13.5 g ; 52 mmol) dans du THF anhydre (100 mL) est ajouté à 0°C, sous atmosphère d'azote, l'hydrure de sodium (sous la forme d'une dispersion à 60% dans de l'huile minérale) (2.5 g ; 62 mmol ; 1.2 éq.). Le mélange est agité 20 minutes à 0°C. Après addition à température ambiante (environ 20°C) d'iodure de tétrabutylammonium (149 mg ; 0.4 mmol ; 0.008 éq.), le bromure de benzyle (9 mL ; 76 mmol ; 1.3 éq.) est ajouté goutte à goutte. Le milieu est chauffé à reflux pendant 2h, puis du méthanol (10 mL) est ajouté lentement. Le mélange est dilué avec du dichlorométhane (100 mL) et de l'eau (40 mL). La phase aqueuse est extraite 3 fois au dichlorométhane (50 mL). Les phases organiques sont rassemblées, séchées et concentrées sous vide. Le produit brut est purifié par chromatographie sur gel de silice (cyclohexane/Et₂O 4/1 + 0.4% de Et₃N) pour donner le composé **26** sous la forme d'une huile jaune.
C₁₉H₂₆O₆ (M = 350,42 g/mol).
RMN ¹H (400 MHz, CDCl₃): δ 1.31 (s, 3H, CH₃), 1.37 (s, 3H, CH₃), 1.43 (s, 3H, CH₃), 1.49 (s, 3H, CH₃), 4.00 (d, 1 H, H6A, *J*_{6A-5} = 5.6, *J*_{6A-8B} = 8.4Hz), 4.02 (d, 1 H, H3, *J*₄₋₃ = 3.0 Hz), 4.11 (d, 1H, H6B, *J*_{6B-5} = 6.0 Hz), 4.15 (d, 1 H, H4, *J*₄₋₅ = 7.6Hz), 4.37 (m, 1 H, H5), 4.58 (d, 1 H, H2, *J*₂₋₁ = 3.6Hz), 4.66 (AB, 2H, CH₂Ph), 5.90 (d, 1 H, H1), 7.27.35 (m, 5H, CHar).
RMN ¹³C (101 MHz, CDCl₃): δ 25.57 (CH₃), 26.37 (CH₃), 26.92 (CH₃), 26.97 (CH₃), 67.53 (C6'), 72.50 (CH₂Ph), 72.65 (C5'), 81.45 (C4'), 81.83 (C3'), 82.79 (C2'), 105.42 (C1'), 109.10 (Cq), 111.90 (Cq), 127.77, 127.96, 128.52, 137.77.
SM-IS *m*/*z* M calculée : 350.17; trouvée 351.0 [M+H]⁺, 373.0 [M+Na]⁺.

### 3-O-Benzyl-1,2-O-isopropylidène-α-D-xylo-pentodialdo-1,4-furanose (composé 27 figure 2c)

### SFPIM268A

Le composé **27** est synthétisé selon [25]. Une solution de **26** (1.03 g ; 2.9 mmol) dans un mélange acide acétique-eau (21/9 mL) est agitée à 45°C pendant 2 heures. Le mélange réactionnel est ensuite refroidie à 0°C, et une solution de périodate de sodium (692 mg ; 3.23 mmol ; 1.1 éq.) dans l'eau (7 mL) est ajoutée. Le milieu est agité à température ambiante (environ 20°C) pendant environ 18h. Du dichlorométhane (15 mL) est ajouté et la phase aqueuse est extraite 3 fois au dichlorométhane (15 mL). Les phases organiques sont rassemblées, puis lavées 2 fois à l'eau (20 mL), séchées sur MgSO₄ et concentrées sous vide. Le résidu est coévaporé 2 fois avec du toluène. L'aldéhyde **27** est utilisé sans purification dans l'étape suivante.
RMN ¹H (250 MHz, CDCl₃): δ 1.33 (s, 3H, CH₃), 1.47 (s, 3H, CH₃), 4.33 (d, 1 H, H3, *J*₃₋₁ = 3.7 Hz), 4.54 (AB, 2H, CH₂Ph), 4.61 (dd, 1 H, H4, *J*₄₋₅ = 1.5, *J*₄₋₃ = 3.7 Hz), 4.64 (d, 1 H, H2, *J*₂₋₁ = 3.7 Hz), 6.12 (d, 1 H, H1), 7.1-7.4 (m, 5H, CHar), 9.67 (d, 1 H, H5, *J*₅₋₄ = 1.8Hz).

### 3-O-Benzyl-xylo-pentodialdo-1,4-furanose (composé 28, figure 2c)

### SFPIM268

A une solution de **27** (2.9 mmol) dans un mélange dioxane/eau (10/4 mL) est ajoutée de la résine Dowex 50WX8 (3.4 g). Le milieu est agité doucement 18h à 75°C. La résine est filtrée sur verre fritté et rincée avec un mélange dioxane/eau. Les solvants sont évaporés sous pression réduite. Le résidu est coévaporé plusieurs fois avec du toluène jusqu'à obtenir le produit **28** sous la forme d'une mousse orange-rouge. Il est utilisé sans purification dans l'étape suivante.

### N-Benzyl-3-O-benzyl-1,5-dideoxy-1,5-imino-xylitol (composé 29, figure 2c)

### SFPIM272

A une solution de **28** (700 mg, 2.9 mmol) précédemment séché sur P₂O₅ une nuit, dans du méthanol anhydre (50 mL), est ajouté sous atmosphère d'azote le cyanoborohydrure de sodium (554 mg, 2.67 mmol, 3 éq.). Le mélange est agité en présence de tamis moléculaire 3Å pendant 10 minutes. Le milieu est refroidi à -78°C, puis l'acide acétique glacial (332.µL, 5.8 mmol, 2 éq.) et la benzylamine (292 µL, 2.67 mmol, 0.9 éq.) sont ajoutés. Après retour à température ambiante (environ 20°C), le milieu est agité 18h, puis filtré sur célite. La célite est rincée avec de l'acétate d'éthyle (3 x 10 mL). Les solvants sont évaporés sous pression réduite. Le résidu est repris dans l'acétate d'éthyle (60 mL) et lavé avec une solution saturée de NaHCO₃ (20 mL), puis 2 fois avec de l'eau (20 mL). La phase organique est séchée, puis concentrée sous vide pour donner le composé **29** sous la forme d'un solide blanc homogène. Le composé peut éventuellement être recristallisé dans l'acétate d'éthyle.
C₁₉H₂₃O₃N (M = 313.40 g/mol).
RMN ¹H (250 MHz, CDCl₃): δ 2.24 (dd, 2H, H1aA, H5a'A, *J*_{1aA-2a} = *J*_{5aA-4a} = 8, *J*_{1aA-1aB} = *J*_{5aA-5aB} = 11 Hz), 2.45 (m, 2H, OH), 2.85 (dd, 2H, H1aB, H5aB, *J*_{1aB-2a} = *J*_{5aB-4a} = 3.5 Hz), 2.25 (app t, 1 H, H3a, *J*₂ₐ₋₃ₐ = *J*₃ₐ₋₄ₐ = 7 Hz), 3.55 (s, 2H, CH₂Ph), 3.77 (ddd, 2H, H4a, H2a), 4.76 (s, 2H, CH₂Ph), 7.20-7.40 (m, 10H, 2x5 CHar).
RMN ¹³C (101 MHz, CDCl₃): δ 57.12 (C1a, C5a), 62.20 (CH₂Ph NBn), 69.91 (C2a, C4a), 73.98 (CH₂Ph OBn), 84.30 (C3a, signal de faible intensité), 127.39-129.14 (9 pics, CHar), 137.86 (Cqar), 138.68 (Cqar). SM-IS *m*/*z* M calculée : 313.17; trouvée 314.0 [M+H]⁺, 336.0 [M+Na]⁺.

### 3-O-Benzyl-1 ,5-dideoxy-1 ,5-imino-xylitol (composé 30, figure 2c)

### SFPIM314

Le composé **29** (300 mg ; 0.957 mmol) est dissout dans EtOH (10 mL). Un excès de d'hydroxyde de palladium sur charbon 20% est ajouté et le milieu réactionnel est agité à température ambiante (environ 20°C) sous pression atmosphérique d'hydrogène pendant 20h. De l'hydroxyde de palladium sur charbon 20% est à nouveau ajouté et la réaction est poursuivie 18 h supplémentaires. Le milieu réactionnel est filtré sur membranes de millipores, le catalyseur est rincé à l'EtOH (2x10 mL). Le filtrat est évaporé sous pression réduite pour conduire au composé **30** (172 mg, on observe environ 35% de produit O-débenzylé).
C₁₂H₁₇NO₃ (M = 223,27 g/mol)
RMN ¹H (400 MHz, CD₃OD): (contient 35% de composé O-débenzylé référencé*) δ *2.38 (dd, ∼0.4H, *H1aA, *H5aA, *J*_{1aA-2a} = *J*_{5aA-4a} = 10.1, *J*_{1aA-1aB} = *J*_{5aA-5aB} = 12.6 Hz), 2.45 (dd, ∼1.7H, H1aA, H5aA, *J*_{1aA-2a} = *J*_{5aA-4a} = 9.7, *J*_{1aA}-_{1aB} = *J*_{5aA}-_{5aB} = 12.8Hz), 2.85 (dd, 2H, H1aB, H5aB, *J*_{1aB-2a} = *J*_{5aB-4a} = 4.3 Hz, + composé *), 3,21 (app t, 1 H, *H3a, H3a, *J*₂ₐ₋₃ₐ = *J*₄ₐ₋₃ₐ = 8.3 Hz), *3.39 - 3.41 (2 dd, -0.4H, *H4a, *H2a), 3.55 - 3.57 (2 dd, ∼1.7H, H4a, H2a), 4.85 (s, CH₂Ph), 7.20-7.40 (m, 5H, CHar).
RMN ¹³C (101 MHz, CD₃OD): δ *51.39 (*C1a, *C5a), 51.47 (C1a, C5a), 71.92 (C2a, C4a), *72.45 (*C2a, *C4a), 75.55 (CH₂Ph OBn), *79.94 (*C3a), 87.12 (C3a), 127.93-128.98, 129.17 (CHar), 140.36 (Cqar).
SM-IS *m*/*z* M calculée : 223.12; trouvée 224.5 [M+H]⁺, 246.5 [M+Na]⁺.

### N-Trifluoroacétamido-3-O-benzyl-1,5-didéoxy-1,5-imino- xylitol (composé 31, figure 2c)

### SFPIM315

A une solution de composé **30** (172 mg ; 0.77 mmol) dans le dichlorométhane anhydre (5 mL) sont ajoutés la pyridine (100 µL ; 1.31 mmol ; 1.7 éq) et l'anhydride trifluoroacétique (160 µL ; 1.16 mmol ; 1.5 éq). Le milieu réactionnel est agité 18h à température ambiante (environ 20°C) puis est dilué dans le dichlorométhane. La phase organique est lavée deux fois avec une solution d'HCl 1 N, une fois avec H₂O, séchées sur MgSO₄ et concentrée sous vide. Le produit brut est purifié par chromatographie sur gel de silice (Ether de pétrole/acétate d'éthyle 3/1.5) pour donner le produit **31** (110 mg ; 45%).
C₁₄H₁₅NO₄F₃ (M = 318.28 g/mol)
RMN ¹H (400 MHz, CDCl₃): δ 3.50 (d, 1H, H1A ou H5A, *J* = 13.4Hz), 3.61 (m, 1H, H3), 3.66-3.78 (m, 3H, H1 ou H5, OH), 3.82 (m, 2H, H2 ou H4, OH), 3.96 (m, 1 H, H4 ou H2), 4.10 (dd, H1B ou H5B, *J* = 4.0, *J* = 13.6Hz), 4.66 (s, 2H, CH₂Ph), 7.25-7.38 (m, 5H, CHar).
RMN ¹³C (101 MHz, CDCl₃): δ 45.77 (C5 ou C1), 48.25 et 48.28 (C1 ou C5 rotamères), 67.80, (C2 ou C4), 67.97 (C4 ou C2), 73.04 (CH₂Ph), 76.52 (C3), 116.51 (q, CF₃, *J*_{C-F} = 289 Hz), 127.78, 128.23, 128.72 (3 pics CHar), 137.71 (Car), 157.72 (q, COCF₃, *J*_{C-F} = 36Hz).
RMN ¹⁹F (376 MHz, CDCl₃): δ -67.72.

### N-Trifluoroacétamido-3-O-benzyl-2,4 -bis-O-(2,3,4,5-tétra-O-méthoxyacétyl-α-D-mannopyranosyl)-1,5-didéoxy-1,5-imino-xylitol (composé 32, figure 2c)

### SFPIM318

Une solution de composé **17** (500 mg ; 0.813 mmol ; 2.5 éq) dans le dichlorométhane (2 mL) est canulée sous argon dans une solution de composé **31** (105 mg ; 0.33 mmol) dans le dichlorométhane (5 mL) contenant du tamis moléculaire 4Å. Le mélange réactionnel est agité 30 min à température ambiante (environ 20°C) et le triméthysilyl trifluorométhanesulfonate (30 µL ; 0.163 mmol ; 20% par rapport à l'imidate) est ajouté. Le milieu réactionnel est agité 2h à température ambiante (environ 20°C) puis la réaction est stoppée par addition de triéthylamine (0.8 mL) et filtrée sur célite. La célite est rincée au dichlorométhane, le solvant est évaporé à sec et le produit brut est purifié par chromatographie sur gel de silice (Dichlorométhane/acétone 6/1) pour donner le produit **32** (188 mg ; 47%) sous forme d'un sirop incolore.
C₅₀H₆₈NO₃₀F₃ (M = 1220.08 g/mol)
*Remarque : en RMN, on observe des rotamères dus à la présence du NCOCF₃.*
RMN ¹H (400 MHz, CDCl₃): δ 2.92 (dd, 0.5 H, H1aA ou H5aA, *J* = 9.9, 12.9Hz), 3.06 (dd, 0.5 H, H1aA ou H5aA, *J* = 10.1, 12.9Hz), 3.18 (dd, 0.5 H, H 1 aA ou H5aA, *J* = 9.8, 13.9Hz), 3.26 (dd, 0.5 H, H1aA ou H5aA, *J* = 10.3, 13.9Hz), 3.36, 3.38, 3.38, 3.40 3.41, 3.44, 3.45, 3.48 (8 CH₃), 3.64-4.22 (m, 25H, 8CH₂ MAc, 2H5, 1H1aB ou 1H5aB, 3H6, H3a, H2a, H1a), 4.40 (m, 1H, H6B), 4.53 (m, 1H, H1aB ou H5aB), 4.80-4.90 (m, 2H, CH2Ph), 4.94 (s, 0.5H, H1), 5.05 (s, 0.5H, H1), 5.14 (s, 0.5H, H1), 5.17 (s, 0.5H, H1), 5.24-5.45 (m, 6H, 2H2, 2H3, 2H4), 7.15-7.40 (m, 5H, CHar).
RMN ¹³C (101 MHz, CDCl₃) : δ 43.44, 45.52, 45.56, 45.85, 47.89, 47.92 (6 pics pour C1a et C5a), 59.32, 59.37, 59.39, 59.41, 59.44, 59.45, 59.53 (7 CH₃), 61.75, 62.23, 62.58 (3 pics pour les H6), 65.43, 65.56, 65.97, 66.07, 68.49, 68.62, 68.68, 68.94, 69.26, 69.49 (10 pics pour C2, C3, C4, C5), 69.14, 69.22, 69.26, 69.29, 69.39, 69.43, 69.46 (7 pics pour les CH₂ MAc), 71.17, 72.20, 76.56, 77.08, 81.88, 82.00 (6 pics pour C1a, C2a, C3a), 75.48, 75.57 (2 pics pour CH₂Ph), 94.41, 94.75 (2 pics pour C1), 98.82, 99.07 (2 pics pour C1), 116.14. (q, CF₃, *J*_{C-F} = 289Hz), 116.21 (q, CF₃, *J*_{C-F} = 289Hz), 125.32-129.06 (9 pics pour CHar), 137.09,169.12-170.18 (14 pics pour CO MAc).
RMN ¹⁹F (376 MHz, CDCl₃): ô -68.79, -68.55.

### N-Trifluoroacétamido-2,4-bis-O-(2,3,4,5-tétra-O-méthoxyacétyl-α-D-mannopyranosyl)-1,5-didéoxy-1,5-imino-xylitol (composé 33, figure 2c)

### SFPIM319

Le composé **32** (188 mg ; 0.154 mmol) est dissous dans un mélange EtOH/CH₂Cl₂ (6/6 mL). Un excès de palladium sur charbon 10 % est ajouté et le milieu réactionnel est agité à température ambiante (environ 20°C) sous pression atmosphérique d'hydrogène pendant 3h. Le milieu réactionnel est filtré sur membrane de millipores, le catalyseur est rincé avec un mélange EtOH/CH₂Cl₂ (1/1). Le filtrat est évaporé sous pression réduite pour conduire au composé 33 (168 mg ; 97%).
C₄₃H₆₂NO₃₀F₃ (M = 1129.96 g/mol)
*Remarque : en RMN, on observe des rotamères dus à la présence du NCOCF₃*.
RMN ¹H (400 MHz, CDCl₃): δ 2.74 (app t, 0.5 H, H1aA ou H5aA, *J* = 12.1Hz), 2.84 (app t, 0.5 H, H1aA ou H5aA, *J* = 11.6Hz), 3.12 (m, 1H, H1aA ou H5aA), 3.41, 3.42, 3.44, 3.45, 3.46, 3.47, 3.48, 3.49 (8 CH₃), 3.59 (m, 3H, H4a, H2a, OH), 3.74 (m, 1 H, H3a), 3.88-4.49 (m, 23H, 8CH₂ MAc, 2H5, 1H1aB ou 1H5aB, 4H6), 4.64 (m, 1 H, H1aB ou H5aB), 4.94 (s, 0.5H, H1), 5.01 (s, 0.5H, H1), 5.21 (s, 0.5H, H1), 5.25 (s, 0.5H, H1), 5.26-5.48 (m, 6H, 2H2, 2H3, 2H4).
RMN ¹³C (101 MHz, CDCl₃): δ 44.26, 45.61, 46.01, 47.69 (4 pics pour C1a et C5a), 59.22-59.43 (7 CH₃), 62.21, 62.24, 62.58 (3 pics pour les H6), 65.85, 65.90, 65.98, 66.04, 68.57, 68.62, 69.07, 69.13, 69.53 (9 pics pour C2, C3, C4, C5), 69.19, 69.22, 69.33, 69.42, 69.46 (4 CH₂ MAc), 74.05, 74.78, 75.64, 75.84, 76.54, 77.25 (6 pics pour C1a, C2a, C3a), 95.75, 98.99 (2 C), 116.10 (q, CE₃, *J*_{C-F} = 289Hz), 116.18 (q, CF₃, *J*_{C-F} = 289Hz), 155.58 (q, COCF₃, *J*_{C-F} = 36Hz), 155.62 (q, COCF₃, *J*_{C-F} = 36Hz), 169.16-170.18 (13 pics COMAc).
RMN ¹⁹F (376 MHz, CDCl₃): δ -68.91, -68.72.

### N-Trifluoroacétamido-2,4-bis-O-(2,3,4,5-tétra-O-méthoxyacétyl-α-D-mannopyranosyl)-(3-O-(((S)-2-O-hexadécanoyloxy-3-O-octadécanoyloxy-propyl)(benzyl)phosphoryl)- 1,5-didéoxy-1,5-imino-D-xylitol (composé 34, figure 2c)

### SFPIM322

Une solution à 0.26 M dans le CH₂Cl₂ anhydre du composé **22** (861 µL ; 0.223 mmol ; 1.5 éq) est ajoutée à une solution de composé **33** (168 mg ; 0.149 mmol) dans le CH₂Cl₂ anhydre (8 mL). Du tamis 3Å est ajouté et le milieu réactionnel est agité 30 min à température ambiante sous argon. Une solution commerciale de tétrazole (∼0.45 M dans acétonitrile), préalablement séchée sur tamis 3Å, est ajoutée à 0°C (1.65 mL ; 0.743 mmol ; 5 éq) et la réaction est poursuivie sous agitation à température ambiante pendant 2h. Le mélange réactionnel est refroidi à - 40°C et une solution d'acide m-chloro-perbenzoïque (m-CPBA) (50%, 154 mg ; 0.447 mmol ; 3 éq) dans le CH₂Cl₂ (2 mL) est ajouté goutte à goutte. Après 2h d'agitation en laissant remonter le milieu réactionnel à température ambiante (environ 20°C), la réaction est arrêtée par addition d'une solution aqueuse à 50% de Na₂S₂O₃ (20 mL) et le mélange est extrait avec du diéthyle éther (Et₂O) (80 mL). La phase organique est lavée 4 fois avec une solution aqueuse à 50% de Na₂S₂O₃ (3 x 20 mL), 1 fois avec une solution saturée de NaHCO₃, 1 fois avec H₂O, puis séchée sur MgSO₄. Le solvant est évaporé sous vide et le résidu est purifié par colonne chromatographique sur gel de silice (Toluène/acétone 4/1) pour donner le composé **34** (104 mg ; 37%) sous forme d'un sirop incolore.
C₈₇H₁₃₉NO₃₇F₃ (M = 1879.03 g/mol)
*Remarque : les spectres sont difficilement interprétables. En effet, on observe des pics démultipliés à cause des rotamères liés à la présence du NCOCF₃, des diastéréoisomères liés à la présence du phosphate benzylé et de la non-équivalence des sucres. On peut ainsi noter 4 pics pour un seul carbone anomérique. On remarque également 4 pics en RMN du Fluor.*
RMN ¹³C (101 MHz, CDCl₃) : δ 94.47, 94.50, 94.91, 95.00 (1C1), 98.69, 98.76, 99.18 (2C) (1C1).
RMN ³¹P (162 MHz, CDCl₃) : δ -0.92
RMN ¹⁹F (376 MHz, CDCl₃): δ -68.68, -68.67, -68.47, -68.44.
SM-IS *m*/*z* M calculée : 1877.87; trouvée 1901.0 [M+Na]⁺.

### N-Tritluoroacétamido-2,4-bis-O-(2,3,4,5-tétra-O-méthoxyacétyl-α-D-mannopyranosyl)-(3-O-(((S)-2-O-hexadécanoyloxy-3-O-octadécanoyloxy-propyl)phosphoryl)- 1,5-didéoxy-1,5-imino-D-xylitol (composé 35, figure 2c)

### SFPIM323

Le composé **34** (90 mg ; 0.048 mmol) est dissous dans un mélange EtOH/CH₂Cl₂ (6/4 mL). Un excès de palladium sur charbon 10 % est ajouté et le milieu réactionnel est agité à température ambiante (environ 20°C) sous pression atmosphérique d'hydrogène pendant 3h. Le milieu réactionnel est filtré sur membrane de millipores, le catalyseur est rincé avec un mélange EtOH/CH₂Cl₂ (1/1). Le filtrat est évaporé sous pression réduite pour conduire au composé **35** (82 mg ; 95%).
C₈₀H₁₃₃NO₃₇F₃P (M = 1788.91 g/mol)
*Remarque : les spectres sont difficilement interprétables. En effet, on observe des pics démultipliés à cause des rotamères liés à la présence du NCOCF3 de la non-équivalence des sucres. Cela dit, les diastéréoisomères ont disparu, le phosphate a été déprotégé. On peut encore noter 2 pics pour un seul carbone anomérique. On remarque également 2 pics en RMN du Fluor.*
RMN ¹³C (101 MHz, CDCl₃) : δ 94.54, 94.98 (1C1), 98.65, 99.17 (1C1).
RMN ³¹P (162 MHz, CDCl₃) : δ -1.36, -1.30.
RMN ¹⁹F (376 MHz, CDCl₃): ô -68.66, -68.45.

### Exemple 3 : N-Trifluoroacétamido-2,4-bis-O-(α-D-mannopyranosyl)-(3-O-(((S)-2-O-hexadécanoyloxy-3-O-octadécanoyloxy-propyl)phosphoryl)-1,5-didéoxy-1,5-imino-D-xylitol (composé 36, figure 2c)

### SFPIM324-t2

### et

### Exemple 4 : 2,4-bis-O-(α-D-mannopyranosyl)-(3-O-(((S)-2-O-hexadécanoyloxy-3-O-octadécanoyloxy-propyl)phosphoryl)-1,5-didéoxy-1,5-imino-D-xylitol (composé 37, figure 2c)

### SFPIM324-t8

Le composé **35** (80 mg ; 0.045 mmol) est dissous dans un mélange de CHCl₃/MeOH (0.2/0.8 mL). Le mélange réactionnel est refroidi à 0°C et la t-butylamine (164 µL) y est ajoutée. Après 10 min d'agitation à 0°C puis 1 h30 en laissant le milieu réactionnel remonter à température ambiante (environ 20°C), les solvants sont évaporés sous vide poussé à température ambiante et le résidu est purifié par colonne chromatogaphique sur gel de silice (CHCl₃/MeOH/H₂O 70/40/1) pour donner dans les premiers tubes le composé **36** (16 mg ; 32%) et dans les tubes suivant le composé **37** (21 mg ; 48%) sous forme de solides blancs.
Composé **36**
C₅₆H₁₀₁NO₂₁PF₃ (M = 1212.39 g/mol).
*Remarque ; on observe toujours les rotamères dus à la présence de NCOCF₃, surtout au niveau des H anomériques.*
RMN ¹H (400 MHz, CDCl₃/CD₃OD 0.4/0.2 mL): δ 0.89 (t, 6H, 2CH3, *J* = 6.7Hz), 1.27 (m, 52H), 1.61 (m, 4H), 2.32 (t, 2H, *J* = 7.6Hz), 2.34 (t, 2H, *J* = 7.6Hz), 3.20-4.22 (plusieurs massifs), 4.91 (s, 0.5H, H1), 5.03 (s, 0.5H, H1); 5.12 (s, 0.5H, H1), 5.17 (s, 0.5H, H1), 5.25 (m; 1H, H2a').
RMN ³¹P (162 MHz, CDCl₃/CD₃OD 0.4/0.2 mL) : δ 0.161.
RMN ¹⁹F (376 MHz, CDCl₃/CD₃OD 0.4/0.2 mL): δ -68.34, -67,83.
SM-IS (-) *m*/*z* M calculée : 1211.66; trouvée 1210.5 [M-H]⁻.
Composé **37**
C₅₄H₁₀₂NO₂₀P (M = 1116.38 g/mol)
*Remarque : on n'observe plus les rotamères au niveau des H anomériques.*
RMN ¹H (250 MHz, CDCl₃/CD₃OD/D₂O difficilement soluble): δ 0.89 (t, 6H, 2CH3, *J* = 6.8Hz), 1.27 (m, 52H), 1.60 (m, 4H), 2.31 (t, 2H, *J* = 7.3Hz), 2.34 (t, 2H, *J* = 7.3Hz), 3.00-4.5 (plusieurs massifs), 4.93 (s, 1 H, H1), 5.08 (s, 1 H, H1), 5.23 (m, 1 H, H2a').
RMN ³¹P(162 MHz, CDO₃/CD₃OD/D₂O difficilement soluble) : δ 0.670 SM-IS (-) *m*/*z* M calculée : 1115.67; trouvée 1116.0 [M+Na]⁺.

### 2) Stimulation des macrophages de souris sauvages par le LPS en présence des composés 36 et 37

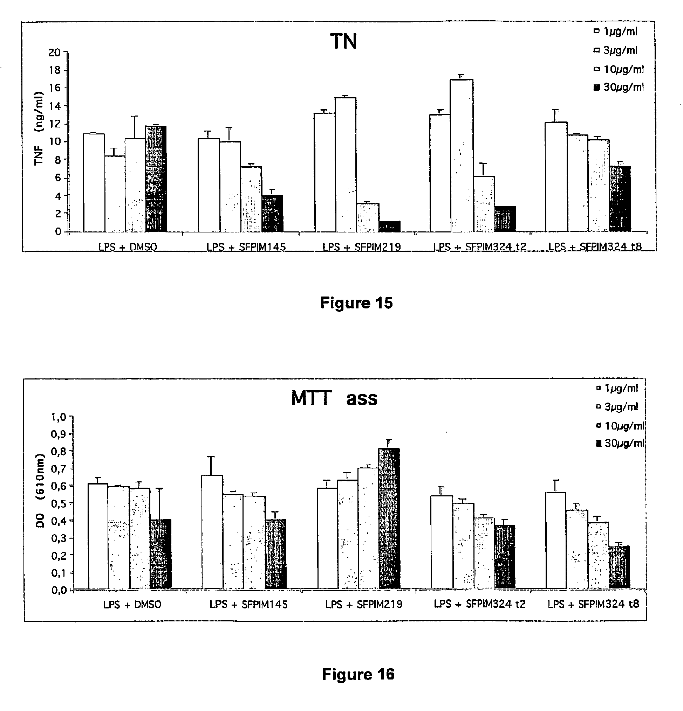

Les composés **36** de la figure 2c ou PIM-2-mimNCOCF₃ (SFPIM324 t2) et **37** de la figure 2c ou PIM-2-mimNH (SFPIM324 t8) ont été testés pour leur activité inhibitrice sur les macrophages stimulés par du LPS (figures 15 à 17).

Les macrophages dérivés de la moelle osseuse de souris sauvages ont été cultivés sur des plaques de culture 96 puits à raison de 10⁵ cellules par puits puis stimulés par du LPS (100 ng/ml, *Escherichia. coli*, sérotype O111:B4, SIGMA) avec le PIM₁ (SFPIM145), L'isoPIM₁C₁₆C₁₈ (SFPIM219) ou les composés PIM-2-mimNCOCF₃ (SFPIM324 t2) et PIM-2-mimNH (SFPIM324 t8) (titrés à 1, 3, 10, 30 µg/ml) ou des contrôles DMSO. Les préparations de SFPIM145, SFPIM219, SFPIM324 t2 et SFPIM324 t8 lyophilisées utilisées sont solubilisées dans du DMSO et additionnées aux cultures à une concentration finale maximale non cytotoxique de 1 %.,
Après une stimulation de 24 heures, les surnageants de culture ont été collectés et analysés pour leur contenu en cytokines TNF-α ou IL-12 p40 par ELISA (R&D Duoset, Minneapolis, MO). Les résultats correspondent à la moyenne +SD de n=2 souris par génotype.

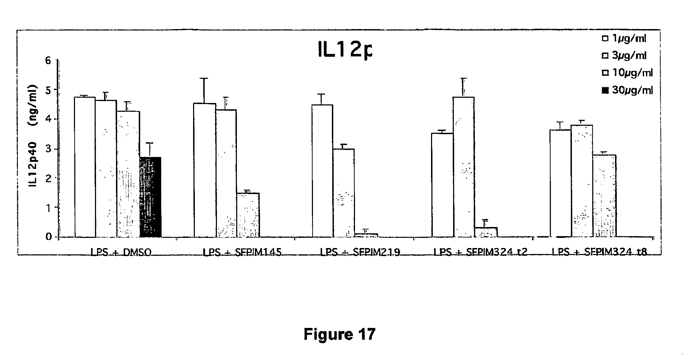

Le PIM-2-mimNCOCF₃ (SFPIM324 t2) et l'isoPIM₁C₁₆C₁₈ (SFPIM219) inhibent fortement la sécrétion de TNF, alors que le PIM₁ (SFPIM145) et PIM-2-mimNH (SFPIM324 t8) inhibent moins fortement (figure 15). Un test de viabilité des cellules au MTT réalisé sur les mêmes macrophages indique une certaine cytotoxicité à la plus forte concentration, notamment en présence de PIM-2-mimNH (SFPIM324 t8) (figure 16). La sécrétion d'IL-12p40 en réponse au LPS est pratiquement abolie à des concentrations de 10ug/mL de PIM-2-mimNCOCF₃ (SFPIM324 t2) et d'isoPIM₁C₁₆C₁₈ (SFPIM219), alors que le PIM₁ (SFPIM145) et PIM-2-mimNH (SFPIM324 t8) inhibent partiellement à cette concentration (figure 17).

### Listes des références

[1] Anticorps anti IL-12 : US2003228311(WO 9816248); Hyaluronan : US 2004097465 ; inhibiteurs IL-12 : US 2005049262 (WO 030475) ; anti sense RNA : US 2004241843.
[2] Keane J. (2005). TNF-blocking agents and tuberculosis: new drugs illuminate an old topic. Rheumatology (Oxford) ; Keane J., Gershon S., Wise R. P., Mirabile-Levens E., Kasznica J., Schwieterman W. D., Siegel J. N., and Braun, M. M. (2001), Tuberculosis associated with infliximab, a tumor necrosis factor alpha-neutralizing agent. N. Engl. J. Med. 345, 1098-1104; Mohan A. K., Cote T. R., Block J. A., Manadan A. M., Siegel J. N., and Braun M. M. (2004), Tuberculosis following the use of etanercept, a tumor necrosis factor inhibitor, Clin. Infect. Dis. 39, 295-299.
[3] Jones B. W., Means T. K., Heldwein K. A., Keen M. A., Hill P. J., Belisle J. T., and Fenton M. J. (2001), J. Leukoc. Biol. 69, 1036-1044
[4] Sieling P. A., Chatterjee D., Porcelli S. A., Prigozy T. 1., Mazzaccaro R. J., Soriano T., Bloom B. R., Brenner M. B., Kronenberg M., Brennan P. J. (1995) Science 269, 227-230.
[5] Ernst W. A., Maher J., Cho S., Niazi K. R., Chatterjee D., Moody D. B., Besra G. S., Watanabe Y., Jensen P. E., Porcelli S. A., Kronenberg M., and Modlin R. L. (1998) Immunity 8, 331-340.
[6] Apostolou 1., Takahama Y., Belmant C., Kawano T., Huerre M., Marchal G., Cui J., Taniguchi M., Nakauchi H., Fournie J. J., Kourilsky P., Gachelin G. (1999) Proc. Natl. Acad. Sci. USA 96, 5141-5146.
[7] Gilleron, M., Ronet, C., Mempel, M., Monsarrat, B., Gachelin, G., and Puzo, G. (2001) J Biol Chem 276, 34896-34904.
[8] Knutson, K. L., Z. Hmama, P. Herrera-Velit, R. Rochford, and N. E. Reiner. 1998. Lipoarabinomannan of Mycobacterium tuberculosis promotes protein tyrosine dephosphorylation and inhibition of mitogen-activated protein kinase in human mononuclear phagocytes. Role of the Src homology 2 containing tyrosine phosphatase 1. J Biol Chem 273:645 ; Nigou, J., Zelle-Rieser, C., Gilleron, M., Thurnher, M., and Puzo, G. (2001) J lmmunol 166, 7477-7485 ; Geijtenbeek, T. B., S. J. Van Vliet, E. A. Koppel, M. Sanchez-Hemandez, C. M. Vandenbroucke-Grauls, B. Appelmelk, and Y. Van Kooyk. 2003, Mycobacteria target DC-SIGN to suppress dendritic cell function. J Exp Med 197:7.
[9] Means TK, Wang S, Yoshimura A, Golenbock DT and Fenton MJ. Human Toll-like receptors mediate cellular activation by Mycobacterium tuberculosis. J Immunol 163: 3920 - 3927 (1999).
[10] Nigou, J., Zelle-Rieser, C., Gilleron, M., Thurnher, M., and Puzo, G. (2001) J Immunol 166, 7477-7485.
[11] Maeda, N., J. Nigou, J. L. Herrmann, M. Jackson, A. Amara, P. H. Lagrange, G. Puzo, B. Gicquel, and O. Neyrolles, 2003, The cell surface receptor DC-SIGN discriminates between Mycobacterium species through selective recognition of the mannose caps on lipoarabinomannan, J Biol Chem 278:5513 ; Geijtenbeek, T. B., S. J. Van Vliet, E. A. Koppel, M. Sanchez-Hernandez, C. M. Vandenbroucke-Grauls, B. Appelmelk, and Y. Van Kooyk. 2003..
[12] Besra, G. S., C. B. Morehouse, C. M. Rittner, C. J. Waechter, and P. J. Brennan. 1997. Biosynthesis of mycobacterial lipoarabinomannan. J Biol Chem 272:18460 ; Nigou, J., M. Gilleron, and G. Puzo. 2003. Lipoarabinomannans: from structure to biosynthesis, Biochimie 85:153.
[13] Quesniaux, V.J., Nicolle, D.M., Torres, D., Kremer, L., Guerardel, Y., Nigou, J., Puzo, G., Erard, F., and Ryffel, B. (2004). Toll-like receptor 2 (TLR2)-dependent-postives and TLR2-independent-negative regulation of proinflammatory cytokines by mycobacterial lipomannans. J Immunol 172, 4425-4434.
[14] Gilleron M, Nigou J, Nicolle D, Quesniaux V and Puzo G. The acylation state of mycobacterial lipomannans modulates innate immunity response through Toll-like receptor 2. Chem Biol 13:39-47 (2006) ; Doz E., Rose S., Nigou J., Gilleron M., Puzo G., Erard F., Ryffel B. and Quesniaux V.F.J. (2007) Acylation determines the TLR-dependent positive versus mannose receptor- and SIGNR1-independent negative regulation of proinflammatory cytokines by mycobacterial lipomannan. J. Biol. Chem. 282:26014-25.
[15] Doz E., Rose S., Nigou J., Gilleron M., Puzo G., Erard F., Ryffel B. and Quesniaux V.F.J. (2007) Acylation determines the TLR-dependent positive versus mannose receptor- and SIGNR1-independent negative regulation of proinflammatory cytokines by mycobacterial lipomannan. J. Biol. Chem. 282:26014-25.
[16] Kordulakova, J., Gilleron, M., Mikusova, K., Puzo, G., Brennan, P. J., Gicquel, B., and Jackson, M. (2002) J Biol Chem
[17] Schaeffer, M. L., Khoo, K. H., Besra, G. S., Chatterjee, D., Brennan, P. J., Belisle, J. T., and Inamine, J. M. (1999) J Biol Chem 274, 31625-31631.
[18] Kremer, L., Gurcha, S. S., Bifani, P., Hitchen, P. G., Baulard, A., Morris, H. R., Dell, A., Brennan, P. J., and Besra, G. S. (2002) Biochem J 363, 437-447.
[19] Gilleron, M., Quesniaux, V.F., and Puzo, G. (2003). Acylation state of the phosphatidyl inositol hexamannosides from mycobacterium bovis BCG and mycobacterium tuberculosis H37Rv and its implication in TLR response. J Biol Chem 278, 29880-29889.
[20] Stadelmaier A, Schmidt RR. (2003) Synthesis of phosphatidylinositol mannosides (PIMs). Carbohydr Res. 338:2557-69 ; Liu X, Stocker BL, Seeberger PH. (2006) Total synthesis of phosphatidylinositol mannosides of Mycobacterium tuberculosis. J Am Chem Soc. 128:3638-48.
[21] Figueroa-Perez, I., Stadelmaier, A., Morath, S., Hartung, T., Schmidt, Richard R. (2005) Synthesis of structural variants of Staphylococcus aureus lipoteichoic acid (LTA) Tetrahedron Asymmetry 16, 493-506 ; Dyer, B. S., Jones, J. D., Ainge, G. D., Denis, M., Larsen, D. S. and Painter, G. F. (2007). Synthesis and structure of phosphatidylinositol Dimmanoside. J. Org. Chem. 72, 3282-3288.
[22] Muller, M., Eugster, H. P., Le Hir, M., Shakhov, A., Di Padova, F., Maurer, C., Quesniaux, V. F., and Ryffel, B. (1996) Mol Med 2, 247-255.
[23] Togbe D., Noulin N., Grivennikov SI., Couillin I, Maillet I, Jacobs M, Maret M, Fick L, Nedospasov SA, Quesniaux VFJ, Schnyder B and Schnyder-Candrian S. (2007) T cell derived TNF downregulates acute airway response to endotoxin Eur.J.Immunol. 37:768-79
[24] Lindberg et al. Tetrahedron, 2002, 58, 1387-1398.
[25] Miculka C. (1999), Synlett, 948-950.

## Revendications

1. Composé de formule générale (I) : 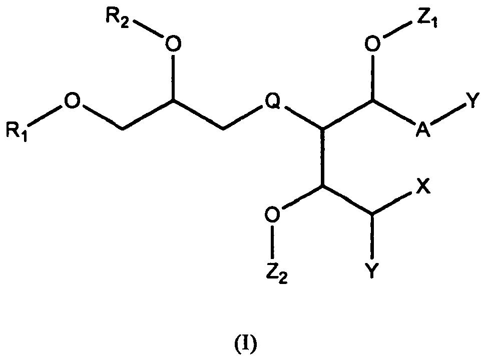 dans laquelle :
■ R₁ et R₂ représentent, indépendemment l'un de l'autre, un atome d'hydrogène, un radical alkyle en C₁-C₂₀, un radical acyle en C₁-C₂₀, étant entendu que lorsque l'un des substituants R₁ ou R₂ est un atome d'hydrogène l'autre substituant est différent d'hydrogène ;
■ Z₁ et Z₂ représentent, indépendemment l'un de l'autre, un atome d'hydrogène, au moins un sucre choisi dans le groupe comprenant le mannose, le glucose, le galactose, étant entendu que lorsque l'un des substituants Z₁ ou Z₂ est un atome d'hydrogène l'autre substituant est différent d'hydrogène ;
■ Q représente -OP(O)₂O-, -OCO₂-, -NHCO₂-, -NHCONH- ;
■ Y représente un atome d'hydrogène, un radical hydroxyl, un radical alkoxy en C₁-C₆, -(CH₂)ₙ-OH avec n est nombre entier égal à 1, 2 ou 3, étant entendu que lorsque Y est un radical hydroxyl, Z₁ et Z₂ ne représentent pas tous les deux un atome d'hydrogène ;
■ A représente -CH₂- ;
■ X représente un atome d'hydrogène ;
■ ou A et X forment ensemble une liaison pour conduire à un cycle à 6 chaînons dans lequel
• A représente un -CH-,
• X représente un -CH₂-, -CH(OH)-, un atome oxygène, un
-NR₃- dans lequel R₃ est un atome d'hydrogène, un radical alkyle en C₁-C₆ ou un
radical acyle en C₁-C₂₀,
étant entendu que, lorsque
• A et X forment une liaison pour conduire à un cycle à 6 chaînons,
• X = -CH(OH)-,
• Y = -OH, et
• Z₁ et Z₂ représentent, indépendemment l'un de l'autre, au moins un sucre choisi dans le groupe comprenant le mannose, le glucose, le galactose,
le cycle à 6 membres est de configuration myo-inositol avec Z₁ ou Z₂ en position 1 et représentant au moins un sucre;
sachant que « alkyle » signifie un radical carboné linéaire, ramifié ou cyclique, saturé ou insaturé, éventuellement substitué, comprenant 1 à 20 atomes de carbone sachant que « acyle » signifie un radical -COR' dans lequel R' est un radical alkyle tel que défini ci-dessus sachant que « alkoxy » signifie un radical -OR' dans lequel R' est un radical alkyle tel que défini ci-dessus ;
ou un de ses sels pharmaceutiquement acceptables ;
sachant que « sels pharmaceutiquement acceptables » signifie des sels préparés avec des acides ou bases, non toxiques.

2. Composé selon la revendication 1 dans lequel A et X forment ensemble une liaison pour conduire à un cycle à 6 chaînons, de formule (Ia) 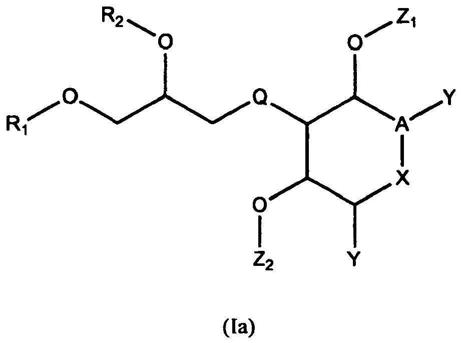 dans laquelle
■ R₁ et R₂, Z₁ et Z₂, Q et Y sont tels que définis à la revendication 1 ;
■ A représente un -CH- ;
■ X représente un -CH₂-, -CH(OH)-, un atome oxygène, un -NR₃- dans lequel R₃ est un atome d'hydrogène, un radical alkyle en C₁-C₆ ou un radical acyle en C₁-C₂₀,
étant entendu que, lorsque
• X = -CH(OH)-
• Y = -OH, et
• Z₁ et Z₂ représentent, indépendemment l'un de l'autre, au moins un sucre choisi dans le groupe comprenant le mannose, le glucose, le galactose,
le cycle à 6 chaînons est de configuration myo-inositol avec Z₁ ou Z₂ en position 1 et représentant au moins un sucre ;
ou un de ses sels pharmaceutiquement acceptables.

3. Composé selon l'une des revendications 1 ou 2, dans lequel A et X forment ensemble une liaison pour conduire à un cycle à 6 chaînons dans lequel
■ R₁ et R₂, sont tels que définis à la revendication 1 ;
■ Q représente -OP(O)₂O- ;
■ A représente un -CH- ;
■ X représente -CH(OH)-,
■ Y représente un radical hydroxyl ;
■ Z₁ et Z₂ représentent, indépendemment l'un de l'autre, au moins un sucre choisi dans le groupe comprenant le mannose, le glucose, le galactose, le cycle à 6 chaînons est de configuration *myo*-inositol avec Z₁ ou Z₂ en position 1 et représentant au moins un sucre ;
ou un de ses sels pharmaceutiquement acceptables.

4. Composé selon selon l'une des revendications 1 ou 2, dans lequel A et X forment ensemble une liaison pour conduire à un cycle à 6 chaînons dans lequel
■ R₁ et R₂, Z₁ et Z₂ et Y sont tels que définis à la revendication 1 ;
■ Q représente -OP(O)₂O- ;
■ A représente un -CH- ;
■ X représente un -NR₃- dans lequel R₃ est un atome d'hydrogène, un radical alkyle en C₁-C₆ ou un radical acyle en C₁-C₂₀ ;
ou un de ses sels pharmaceutiquement acceptables.

5. Composé selon la revendication 1, de formule (Ib) 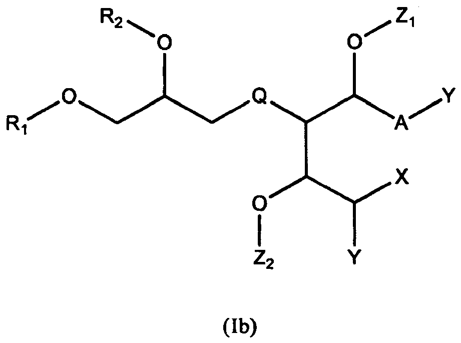 dans laquelle
■ R₁ et R₂, Z₁ et Z₂, Q et Y sont tels que définis à la revendication 1 ;
■ A représente -CH₂- ;
■ X représente un atome d'hydrogène ;
ou un de ses sels pharmaceutiquement acceptables.

6. Composé selon l'une des revendications 1 ou 2, dans lequel A et X forment ensemble une liaison pour conduire à un cycle à 6 chaînons dans lequel
■ R₁ et R₂ représentent, indépendemment l'un de l'autre, un radical acyle en C₁-C₂₀ ;
■ Z₁ représente le mannose ;
■ Z₂ représente un atome d'hydrogène ;
■ Q représente -OP(O)₂O- ;
■ A représente un -CH- ;
■ X représente un -CH(OH)-
■ Y représente un radical hydroxyl ;
■ le cycle à 6 chaînons est de configuration myo-inositol avec Z₁ en position 1 ;
ou un de ses sels pharmaceutiquement acceptables

7. Composé selon l'une des revendications 1 à 6 dans lequel les sels pharmaceutiquement acceptables sont des sels de sels de sodium, potassium, calcium, ammonium, magnésium, les sels hydrochlorique, hydrobromique, nitrique, carbonique, monohydrogencarbonique, phosphorique, monohydrogenphosphorique, dihydrogenphosphorique, sulfurique, monohydrogensulfurique, hydriodique, les sels acétique, propionique, isobutyrique, maléique, malonique, benzoïque, succinique, subérique, fumarique, lactique, mandélique, phthalique, benzènesulfonique, p-tolylsulfonique, citrique, tartarique, méthanesulfonique.

8. Procédé de préparation de composés tels que définis à l'une quelconque des revendications 1 à 7, dans lequel :
a) on condense un dérivé di-O-acylé ou di-O-alkylé du glycérol de formule (III) 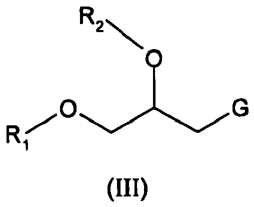 dans laquelle
• G = OH ou NH₂
• R₁ et R₂ ont les mêmes définitions que dans la revendication 1 avec un agent de phosphitylation, de phosphorylation ou de carbonylation pour donner un intermediaire de formule (IV) 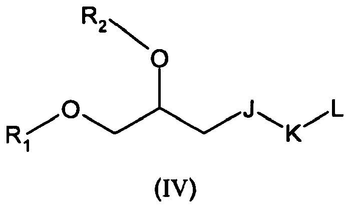 dans laquelle
• J = O ou NH,
• K = P-OBn, P(O)-OBn ou C=O
• L = groupe sortant
• R₁ et R₂ ont les mêmes définitions que dans la revendication 1 ;
b) on condense l'intermédiaire (IV) avec un dérivé d'un polyol ou aminopolyol de formule générale (V) 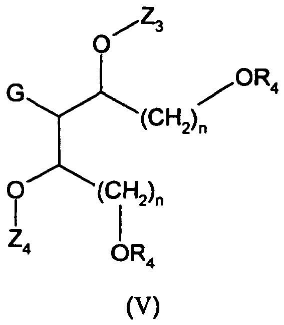 dans laquelle
• G = OH ou NH₂
• Z₃ et Z₄ représentent indépendamment l'un de l'autre un hexopyranose de configuration *manno, gluco* ou *galacto* et portant des groupes protecteurs acétyle ou methoxyacétyle, ou un groupement benzyle, étant entendu que l'un au moins des groupes Z₃ et Z₄ représente un sucre protégé
• R₄ représente un groupe protecteur choisi dans le groupe comprenant un groupe benzyle, ou un radical alkoxyacétyle en C₁-C₆,
ou on condense l'intermédiaire (IV) avec un composé cyclique de formule générale (VI) 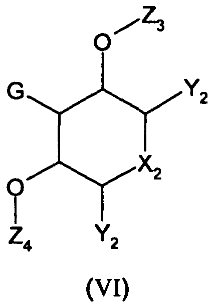 dans laquelle
• G = OH, NH₂
• Z₃ et Z₄ ont les mêmes définitions que précédemment
• Y₂ = H ou un groupe -OR₄ ou un groupe -(CH₂)ₙOR₄ dans lequel R₄ a la même définition que précédemment
• X₂ = -CH₂, -CHOR₄, -NR₃ ou O dans lequel R₃ est un atome d'hydrogène, un radical alkyle en C₁-C₆ ou un radical acyle en C₁-C₂₀ et R₄ représente un groupe protecteur choisi dans le groupe comprenant un groupe benzyle, ou un radical alkoxyacétyle en C₁-C₆
éventuellement en présence d'un agent de couplage ;
c) on soumet, éventuellement, le composé obtenu à l'étape b) à une réaction d'oxydation, pour donner un composé de formule générale (VII) 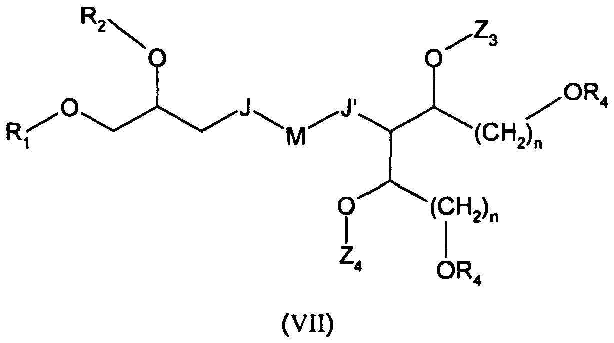 dans laquelle
• J et J' = O ou NH
• M = P(O)OBn ou C=O
• Z₃, Z₄, R₁, R₂, R₄ ont les mêmes définitions que précédemment
ou un composé de formule générale (VIII) 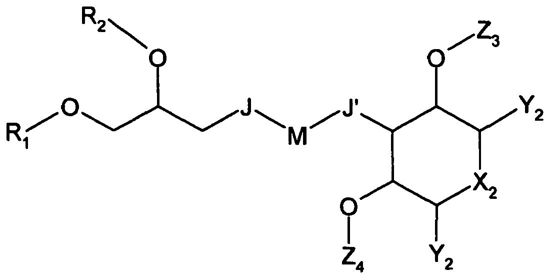 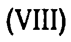 dans laquelle
• J et J' = O ou NH
• M = P(O)OBn ou C=O
• X₂, Y₂, Z₃, Z₄, R₁, R₂, ont les mêmes définitions que précédemment ;
d) on soumet le produit de formule générale (VII) ou de formule générale (VIII) à une déprotection en deux étapes qui consiste en ce que l'on les traite d'abord avec une alkylamine choisi dans le groupe comprenant la t-butylamine puis soumet les produits désacylés à une hydrogénation catalytique.

9. Composé tel que défini à l'une quelconque des revendications 1 à 7 comme médicament.

10. Composition pharmaceutique comprenant au moins un composé de tel que défini à l'une quelconque des revendications 1 à 7 et tout excipient pharmaceutiquement acceptable.

11. Utilisation d'un composé tel que défini à l'une quelconque des revendications 1 à 7, pour la fabrication d'un médicament destiné à la prévention ou au traitement d'une maladie associée à la surexpression de cytokines ou de chimiokines, notamment du TNF et/ou de l'IL-12, ladite maladie comprenant :
A) les maladies immunes ou auto-immunes choisies dans le groupe comprenant la polyarthrite rhumatoïde, le diabète sucré, le lupus érythémateux disséminé ou la maladie de Basedow ;
B) le rejet de greffe,
C) les infections virales et/ou parasitaires ;
D) les chocs résultant d'une infection chronique ou aigue d'origine bactérienne virale et/ou parasitaire ;
E) les maladies inflammatoires choisies dans le groupe comprenant les maladies inflammatoires chroniques et les maladies inflammatoires vasculaires ;
F) les maladies neurodégénératives choisies dans le groupe comprenant les maladies démyélinisantes, les maladies extrapyramidales et cérébelleuses ;
G) les pathologies malignes impliquant des tumeurs sécrétant du TNF ou impliquant le TNF choisies dans le groupe comprenant la leucémie, le lymphome ; et
H) l'hépatite induite par l'alcool.

12. Utilisation selon la revendication 11, selon laquelle le médicament est destiné à la prévention ou au traitement d'une maladie inflammatoire.

## Patentansprüche

1. Verbindung der allgemeinen Formel (I): 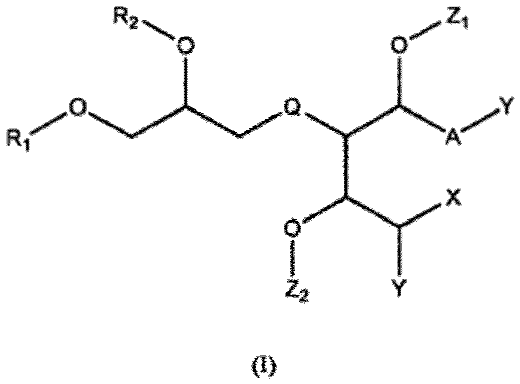 in der:
■ R₁ und R₂ unabhängig voneinander ein Wasserstoffatom, einen C₁-C₂₀-Alkylrest, einen C₁-C₂-Acylrest bedeuten, wobei gilt, dass, wenn einer der Substituenten R₁ und R₂ ein Wasserstoffatom ist, sich der andere Substituent von Wasserstoff unterscheidet;
■ Z₁ und Z₂ unabhängig voneinander ein Wasserstoffatom, mindestens einen Zucker, ausgewählt aus der Gruppe Mannose, Glukose, Galaktose bedeuten, wobei gilt, dass, wenn einer der Substituenten Z₁ oder Z₂ ein Wasserstoffatom ist, sich der andere Substituent von Wasserstoff unterscheidet;
■ Q -OP(O)₂O-, -OCO₂-, -NHCO₂-, -NHCONH- bedeutet;
■ Y ein Wasserstoffatom, einen Hydroxylrest, einen C₁-C₆-Alkoxyrest, -(CH₂)ₙ-OH, wobei n eine ganze Zahl gleich 1, 2 oder 3 ist, bedeutet, wobei gilt, dass, wenn Y ein Hydroxylrest ist, Z₁ und Z₂ nicht beide ein Wasserstoffatom bedeuten;
■ A -CH₂- bedeutet;
■ X ein Wasserstoffatom bedeutet;
■ oder A und X gemeinsam eine Bindung bilden, wodurch ein Zyklus mit 6 Ringgliedern gebildet wird, in dem
• A -CH- bedeutet,
• X -CH₂-, -CH(OH)-, ein Sauerstoffatom, -NR₃-, worin R₃ ein Wasserstoffatom, ein C₁-C₆-Alkylrest oder ein C₁-C₂₀-Acylrest ist, bedeutet,
wobei gilt, dass, wenn
• A und X eine Bindung bilden, wodurch ein Zyklus mit 6 Ringgliedern gebildet wird,
• X = -CH(OH)-,
• Y = -OH und
• Z₁ und Z₂ unabhängig voneinander mindestens einen Zucker, ausgewählt aus der Gruppe Mannose, Glukose, Galaktose, bedeuten,
der 6-gliedrige Zyklus die myo-Inositolkonfiguration aufweist, wobei sich Z₁ oder Z₂ in Position 1 befinden und mindestens einen Zucker bedeuten;
wobei "Alkyl" ein geradkettiges, verzweigtes oder cyclisches, gesättigtes oder ungesättigtes, gegebenenfalls substituiertes Kohlenstoffradikal mit 1 bis 20 Kohlenstoffen bedeutet,
wobei "Acyl" ein -COR'-Radikal, in dem R' ein Aklylradikal wie oben definiert ist, bedeutet, wobei "Alkoxy" ein -OR'-Radikal, in dem R' ein Alkylradikal wie oben definiert ist, bedeutet; oder eines ihrer pharmazeutisch unbedenkliches Salze;
wobei "pharmazeutisch unbedenkliche Salze" Salze, die mit nichttoxischen Säuren oder Basen hergestellt sind, bedeutet.

2. Verbindung nach Anspruch 1, in der A und X gemeinsam eine Bindung bilden, wodurch ein Cyclus mit 6 Ringgliedern der Formel (Ia) 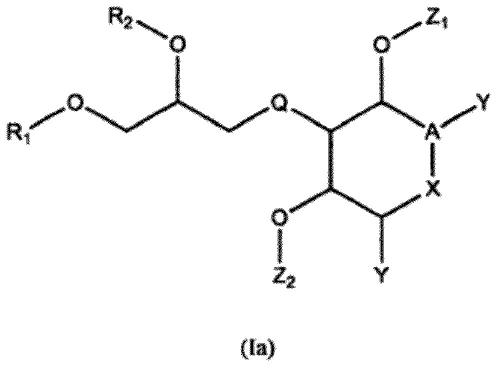 gebildet wird, worin
■ R₁ und R₂, Z₁ und Z₂, Q und Y wie in Anspruch 1 definiert sind;
■ A -CH- bedeutet;
■ X -CH₂-, -CH(OH)-, ein Sauerstoffatom, -NR₃-, worin R₃ ein Wasserstoffatom, ein C₁-C₆-Alkylrest oder ein C₁-C₂₀-Acylrest ist, bedeutet,
wobei gilt, dass, wenn
• X = -CH(OH)-,
• Y = -OH und
• Z₁ und Z₂ unabhängig voneinander mindestens einen Zucker, ausgewählt aus der Gruppe Mannose, Glukose, Galaktose, bedeuten,
der 6-gliedrige Zyklus die myo-Inositolkonfiguration aufweist, wobei sich Z₁ oder Z₂ in Position 1 befinden und mindestens einen Zucker bedeuten;
oder eines ihrer pharmazeutisch unbedenklichen Salze.

3. Verbindung nach einem der Ansprüche 1 oder 2, in der A und X gemeinsam eine Bindung bilden, wodurch ein Cyclus mit 6 Ringgliedern gebildet wird, in dem
■ R₁ und R₂ wie in Anspruch 1 definiert sind;
■ Q -OP(O)₂O- bedeutet;
■ A -CH- bedeutet;
■ X -CH(OH)- bedeutet;
■ Y einen Hydroxylrest bedeutet:
■ Z₁ und Z₂ unabhängig voneinander mindestens einen Zucker, ausgewählt aus der Gruppe Mannose, Glukose, Galaktose, bedeuten,
■ der 6-gliedrige Zyklus die myo-Inositolkonfiguration aufweist, wobei sich Z₁ oder Z₂ in Position 1 befinden und mindestens einen Zucker bedeuten;
oder eines ihrer pharmazeutisch unbedenklichen Salze.

4. Verbindung nach einem der Ansprüche 1 oder 2, in der A und X gemeinsam eine Bindung bilden, wodurch ein Cyclus mit 6 Ringgliedern gebildet wird, in dem
■ R₁ und R₂, Z₁ und Z₂ sowie Y wie in Anspruch 1 definiert sind;
■ Q -OP(O)₂O- bedeutet;
■ A -CH- bedeutet;
■ X -NR₃-, worin R₃ ein Wasserstoffatom, ein C₁-C₆-Alkylrest oder ein C₁-C₂₀-Alkylrest ist, bedeutet;
oder eines ihrer pharmazeutisch unbedenklichen Salze.

5. Verbindung nach Anspruch 1 der Formel (Ib) 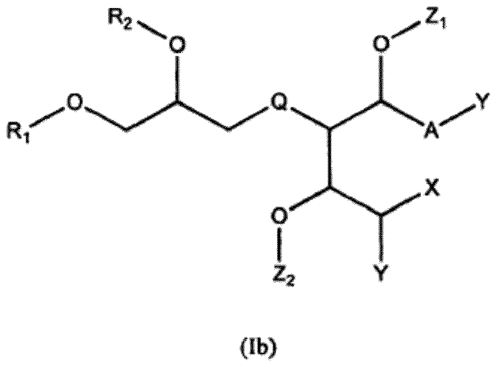 worin
■ R₁ und R₂, Z₁ und Z₂, Q und Y wie in Anspruch 1 definiert sind;
■ A -CH₂- bedeutet;
■ X ein Wasserstoffatom bedeutet;
oder eines ihrer pharmazeutisch unbedenklichen Salze.

6. Verbindung nach einem der Ansprüche 1 oder 2, in der A und X gemeinsam eine Bindung bilden, wodurch ein Cyclus mit 6 Ringgliedern gebildet wird, in dem
■ R₁ und R₂ unabhängig voneinander einen C₁-C₂₀-Acylrest bedeuten,
■ Z₁ Mannose bedeutet;
■ Z₂ ein Wasserstoffatom bedeutet;
■ Q -OP(O)₂O- bedeutet;
■ A -CH- bedeutet;
■ X -CH(OH)- bedeutet;
■ Y einen Hydroxylrest bedeutet;
■ der Cyclus mit 6 Ringgliedern die myo-Inositolkonfiguration aufweist, wobei Z₁ in Position 1 steht; oder eines ihrer pharmazeutisch unbedenklichen Salze.

7. Verbindung nach einem der Ansprüche 1 bis 6, wobei es sich bei den pharmazeutisch unbedenklichen Salzen um Natriumsalze, Kaliumsalze, Kalziumsalze, Ammoniumsalze, Magnesiumsalze, die Salze der Chlorwasserstoffsäue, Bromwasserstoffsäure, Salpetersäure, Kohlensäure, Monohydrogenkohlensäure, Phosphorsäure, Monohydrogenphosphorsäure, Dihydrogenphosphorsäure, Schwefelsäure, Monohydrogenschwefelsäure, Jodwasserstoffsäure, die Salze der Essigsäure, Propionsäure, Isobuttersäure, Maleinsäure, Malonsäure, Benzoesäure, Bernsteinsäure, Korksäure, Fumarsäure, Milchsäure, Mandelsäure, Phthalsäure, Benzolsulfonsäure, p-Tolylsulfonsäure, Zitronensäure, Weinsäure, Methansulfonsäure handelt.

8. Verfahren zur Herstellung von Verbindungen wie in einem der Ansprüche 1 bis 7 definiert, in dem:
a) man ein di-O-acyliertes oder di-O-alkyliertes Derivat des Glycerins der Formel (III) 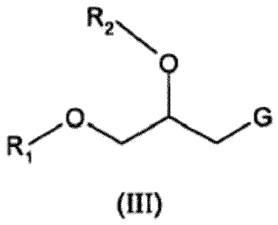 worin
• G = OH oder NH₂
• R₁ und R₂ wie in Anspruch 1 definiert sind mit einem Phosphitylierungsmittel, Phosphorylierungsmittel oder Carbonylierungsmittel kondensiert, wodurch man zu einem Zwischenprodukt der Formel (IV) 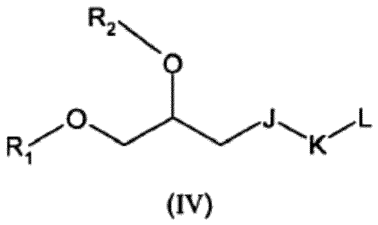 worin
• J = 0 oder NH,
• K = P-OBn, P(O)-OBn oder C=O
• L = Abgangsgruppe
• R₁ und R₂ wie in Anspruch 1 definiert sind, gelangt;
b) man das Zwischenprodukt (IV) mit einem Polyol- oder Aminopolyolderivat der allgemeinen Formel (V) 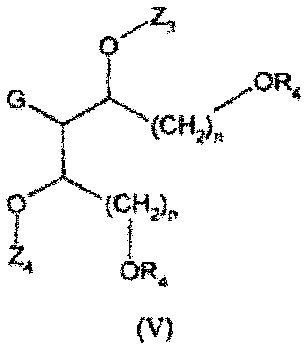 worin
• G = OH oder NH₂
• Z₃ und Z₄ unabhängig voneinander eine Hexopyranose mit *manno-, gluco-* oder galacto-Konfiguration, die Acetyl- oder Methoxyacetylschutzgruppen trägt, oder eine Benzylgruppe bedeuten, wobei gilt, dass mindestens eine der Gruppen Z₃ und Z₄ einen geschützten Zucker bedeutet,
• R₄ eine Schutzgruppe ausgewählt aus der Gruppe umfassend eine Benzylgruppe oder einen C₁-C₆-Alkoxyacetylrest bedeutet, kondensiert
oder man das Zwischenprodukt (IV) mit einer cyclischen Verbindung der allgemeinen Formel (VI) 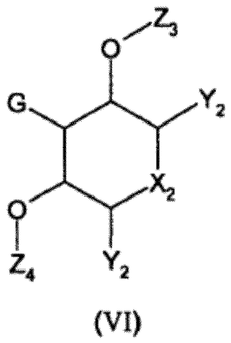 worin
• G = OH oder NH₂
• Z₃ und Z₄ wie zuvor definiert sind,
• Y₂ = H oder eine -OR₄-Gruppe oder eine -(CH₂)ₙO₄-Gruppe, worin R₄ wie zuvor definiert ist,
• X₂ = -CH₂, -CHOR₄, -NR₃ oder 0 bedeutet, worin R₃ ein Wasserstoffatom, ein C₁-C₆-Alkylrest oder ein C₁-C₂₀-Acylrest ist und R₄ eine Schutzgruppe ausgewählt aus der Gruppe umfassend eine Benzylgruppe oder einen C₁-C₆-Alkoxyacetylrest bedeutet,
kondensiert,
gegebenenfalls in Gegenwart eines Kupplungsmittels;
c) oder man unterwirft gegebenenfalls die in Schritt b) erhaltene Verbindung einer Oxidationsreaktion , wodurch man zu einer Verbindung der allgemeinen Formel (VII) 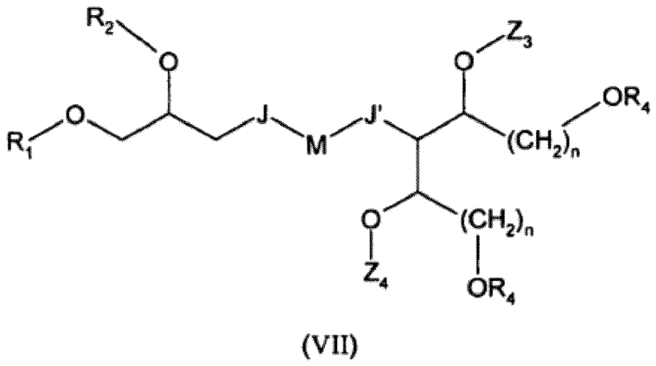 worin
• J und J' = 0 oder NH
• M = P(O)OBn oder C=O
• Z₃, Z₄, R₁, R₂, R₄ wie zuvor definiert sind, oder einer Verbindung der allgemeinen Formel (VIII) 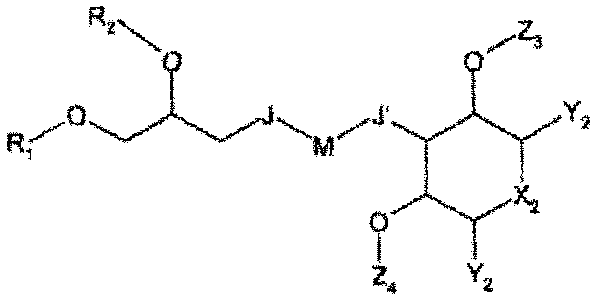 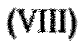 worin
• J und J' = 0 oder NH
• M = P(O)OBn oder C=O
• X₂, Y₂, Z₃, Z₄, R₁, R₂ wie zuvor definiert sind, gelangt;
d) das Produkt der allgemeinen Formel (VII) oder der allgemeinen Formel (VIII) in zwei Stufen entschützt wird, was darin besteht, dass man sie zuerst mit einem Alkylamin ausgewählt aus der Gruppe umfassend t-Butylamin behandelt und die desacylierten Produkte dann katalytisch hydriert werden.

9. Zusammensetzung wie in einem der Ansprüche 1 bis 7 definiert als Arzneimittel.

10. Pharmazeutische Zusammensetzung umfassend mindestens eine Verbindung wie in einem der Ansprüche 1 bis 7 definiert sowie jeglichen pharmazeutisch unbedenklichen Grundstoff.

11. Verwendung einer Verbindung wie in einem der Ansprüche 1 bis 7 definiert zur Herstellung eines Arzneimittels zur Vorbeugung oder Behandlung einer mit der Überexpression von Zytokinen oder Chimiokinen, inbesondere TNF und/oder IL-12, assoziierten Krankheit, wobei die Krankheit Folgendes umfasst:
A) Immun- oder Autoimmunkrankheiten, ausgewählt aus der Gruppe rheumatoide Polyarthritis, Zuckerkrankheit, disseminierter Lupus erythematodes oder Basedow-Krankheit;
B) Transplantatabstoßung,
C) Virusinfektionen und/oder parasitäre Infektionen;
D) Schock aufgrund von chronischer oder akuter Infektion bakteriellen, viralen und/oder parasitären Ursprungs;
E) Entzündungserkrankungen ausgewählt aus der Gruppe chronische Entzündungserkrankungen und Gefäßentzündungserkrankungen;
F) neurodegenerative Erkrankungen ausgewählt aus der Gruppe entmyelinisierende Erkrankungen, extrapyramidale Erkrankungen und Cerebellum-Erkrankungen;
G) maligne Pathologien, an denen TNF-sezernierende Tumore beteiligt sind oder an denen TNF beteiligt ist, ausgewählt aus der Gruppe Leukämie und Lymphom; sowie
H) alkoholinduzierte Hepatitis.

12. Verwendung nach Anspruch 11, wobei das Arzneimittel zur Vorbeugung oder Behandlung einer Entzündungserkrankung bestimmt ist.

## Claims

1. A compound of general formula (I): 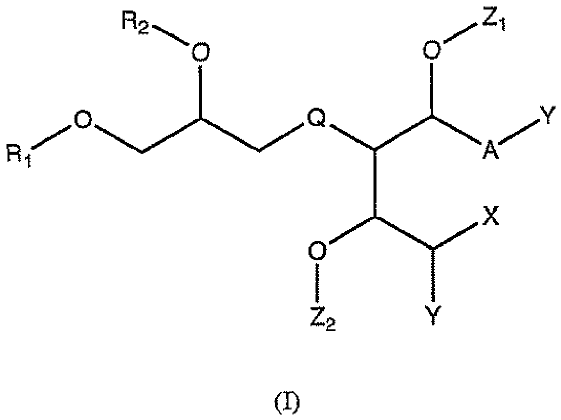 in which:
■ R₁ and R₂ represent, independently of one another, a hydrogen atom, a C₁-C₂₀ alkyl radical, a C₁-C₂₀ acyl radical, it being understood that, when one of the substituents R₁ or R₂ is a hydrogen atom, the other substituent is other than hydrogen;
■ Z₁ and Z₂ represent, independently of one another, a hydrogen atom, at least one sugar chosen from the group comprising mannose, glucose, galactose, it being understood that, when one of the substituents Z₁ or Z₂ is a hydrogen atom, the other substituent is other than hydrogen;
■ Q represents -OP(O)₂O-, -OCO₂-, -NHCO₂-, -NHCONH-;
■ Y represents a hydrogen atom, a hydroxyl radical, a C₁-C₆ alkoxy radical, -(CH₂)ₙ-OH with n being an integer equal to 1, 2 or 3, it being understood that when Y is a hydroxyl radical, Z₁ and Z₂ do not both represent a hydrogen atom;
■ A represents -CH₂-;
■ X represents a hydrogen atom;
■ or A and X together form a bond so as to result in a 6-membered ring in which:
• A represents a -CH-,
• X represents a -CH₂-, -CH(OH)-, an oxygen atom, an -NR₃- in which R₃ is a hydrogen atom, a C₁-C₆ alkyl radical or a C₁-C₂₀ acyl radical,
it being understood that, when
• A and X form a bond so as to result in a 6-membered ring,
• X = -CH(OH)-,
• Y = -OH, and
• Z₁ and Z₂ represent, independently of one another, at least one sugar chosen from the group comprising mannose, glucose, galactose,
the 6-membered ring is in the myo-inositol configuration with Z₁ or Z₂ in position 1 and representing at least one sugar;
knowing that "alkyl" means a linear, branched or cyclic, saturated or unsaturated, optionally substituted, carbon radical containing 1 to 20 carbon atoms
knowing that "acyl" means a -COR' radical in which R' is an alkyl radical as defined above knowing that "alkoxy" means an -OR' radical in which R' is an alkyl radical as defined above;
or a pharmaceutically acceptable salt thereof;
knowing that "pharmaceutically acceptable salt" means salts prepared with nontoxic acids or bases.

2. The compound as claimed in claim 1, in which A and X together form a bond so as to result in a 6-membered ring, of formula (Ia) 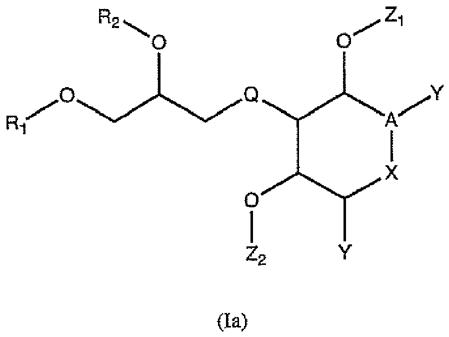 in which:
■ R₁ and R₂, Z₁ and Z₂, Q and Y are as defined in claim 1;
■ A represents a -CH-;
■ X represents a -CH₂-, -CH(OH)-, an oxygen atom, an -NR₃- in which R₃ is a hydrogen atom, a C₁-C₆ alkyl radical or a C₁-C₂₀ acyl radical,
it being understood that, when
• X = -CH(OH)-,
• Y = -OH, and
• Z₁ and Z₂ represent, independently of one another, at least one sugar chosen from the group comprising mannose, glucose, galactose, the 6-membered ring is in the *myo*-inositol configuration with Z₁ or Z₂ in position 1 and representing at least one sugar;
or a pharmaceutically acceptable salt thereof.

3. The compound as claimed in either of claims 1 and 2, in which A and X together form a bond so as to result in a 6-membered ring, in which:
■ R₁ and R₂ are as defined in claim 1;
■ Q represents -OP(O)₂O-;
■ A represents a -CH-;
■ X represents -CH(OH)-
■ Y represents a hydroxyl radical;
■ Z₁ and Z₂ represent, independently of one another, at least one sugar chosen from the group comprising mannose, glucose, galactose,
the 6-membered ring is in the *myo*-inositol configuration with Z₁ or Z₂ in position 1 and representing at least one sugar;
or a pharmaceutically acceptable salt thereof.

4. The compound as claimed in either of claims 1 and 2, in which A and X together form a bond so as to result in a 6-membered ring, in which:
■ R₁ and R₂, Z₁ and Z₂ and Y are as defined in claim 1;
■ Q represents -OP(O)₂O-;
■ A represents a -CH-;
■ X represents an -NR₃- in which R₃ is a hydrogen atom, a C₁-C₆ alkyl radical or a C₁-C₂₀ acyl radical;
or a pharmaceutically acceptable salt thereof.

5. The compound as claimed in claim 1, of formula (Ib) 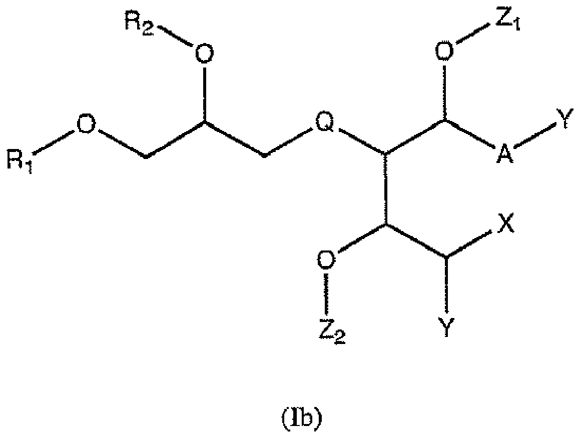 in which:
■ R₁ and R₂, Z₁ and Z₂, Q and Y are as defined in claim 1;
■ A represents -CH₂-;
■ X represents a hydrogen atom;
or a pharmaceutically acceptable salt thereof.

6. The compound as claimed in either of claims 1 and 2, in which A and X together form a bond so as to result in a 6-membered ring, in which:
■ R₁ and R₂ represent, independently of one another, a C₁-C₂₀ acyl radical;
■ Z₁ represents mannose;
■ Z₂ represents a hydrogen atom;
■ Q represents -OP(O)₂O-;
■ A represents a -CH-;
■ X represents a -CH(OH)-;
■ Y represents a hydroxyl radical;
a the 6-membered ring is in the *myo*-inositol configuration with Z₁ in position 1;
or a pharmaceutically acceptable salt thereof.

7. The compound as claimed in any one of claims 1 to 6, in which pharmaceutically acceptable salts are salts of sodium, potassium, calcium, ammonium, magnesium salts, salts of hydrochloric acid, hydrobromic acid, nitric acid, carbonic acid, monohydrogencarbonic acid, phosporic acid, monohydrogenphosphoric acid, dihydrogenphosphoric acid, sulfuric acid, monohydrogensulfuric acid or hydriodic acid, salts of acetic acid, propionic acid, isobutyric acid, maleic acid, malonic acid, benzoic acid, succinic acid, suberic acid, fumaric acid, lactic acid, mandelic acid, phthalic acid, benzenesulfonic acid, p-tolylsulfonic acid, citric acid, tartaric acid or methanesulfonic acid.

8. A method for preparing compounds as defined in any one of claims 1 to 7, in which:
a) a di-O-acylated or di-O-alkylated derivative of glycerol of formula (III) 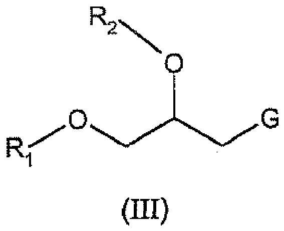 in which:
• G = OH or NH₂,
• R₁ and R₂ have the same definitions as in claim 1,
is condensed with a phosphitylating, phosphorylating or carbonylating agent so as to give an intermediate of formula (IV) 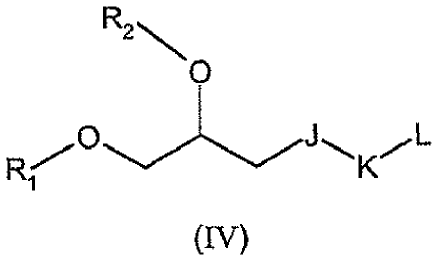 in which:
• J = 0 or NH,
• K = P-OBn, P(O)-OBn or C=O,
• L = leaving group,
• R₁ and R₂ have the same definitions as in claim 1;
b) the intermediate (IV) is condensed with a derivative of a polyol or aminopolyol of general formula (V) 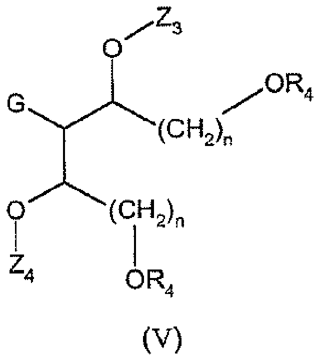 in which:
• G = OH or NH₂,
• Z₃ and Z₄ represent, independently of one another, a hexopyranose of *manno*, *gluco* or *galacto* configuration and bearing acetyl or methoxyacetyl protective groups, or a benzyl group, it being understood that at least one of the Z₃ and Z₄ groups represents a protected sugar,
• R₄ represents a protective group chosen from the group comprising a benzyl group or a C₁-C₆ alkoxyacetyl radical,
or the intermediate (IV) is condensed with a cyclic compound of general formula (VI) 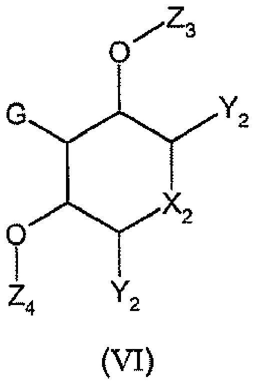 in which:
• G = OH or NH₂,
• Z₃ and Z₄ have the same definitions as above,
• Y₂ = H or an -OR₄ group or a -(CH₂)ₙOR₄ group in which R₄ has the same meaning as above,
• X₂ = -CH₂, -CHOR₄, -NR₃ or 0 in which R₃ is a hydrogen atom, a C₁-C₆ alkyl radical or a C₁-C₂₀ acyl radical and R₄ represents a protective group chosen from the group comprising a benzyl group or a C₁-C₆ alkoxyacetyl radical,
optionally in the presence of a coupling agent;
c) the compound obtained in step b) is optionally subjected to an oxidation reaction, so as to give a compound of general formula (VII) 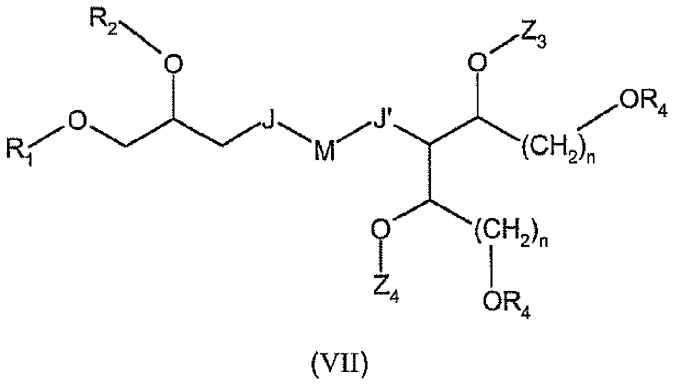 in which:
• J and J' = 0 or NH,
• M = P(O)OBn or C=O,
• Z₃, Z₄, R₁, R₂ and R₄ have the same definitions as above,
or a compound of general formula (VIII) 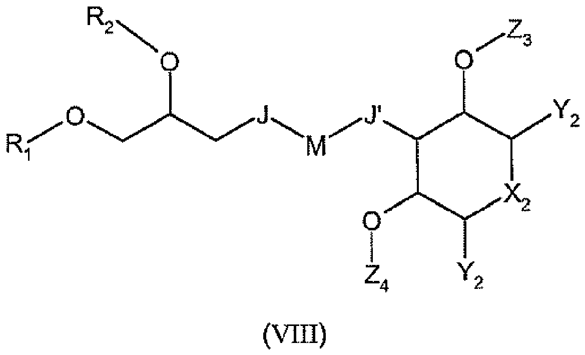 in which:
• J and J' = 0 or NH,
• M = P(O)OBn or C=O,
• X₂, Y₂, Z₃, Z₄, R₁ and R₂ have the same definitions as above;
d) the product of general formula (VII) or of general formula (VIII) is subjected to a two-stage deprotection which consists in first treating them with an alkylamine chosen from the group comprising t-butylamine, and then subjecting the deacylated products to catalytic hydrogenation.

9. The compound as defined in any one of claims 1 to 7, as a medicament.

10. A pharmaceutical composition comprising at least one compound as defined in any one of claims 1 to 7 and any pharmaceutically acceptable excipient.

11. The use of a compound as defined in any one of claims 1 to 7, for the production of a medicament intended for the prevention or treatment of a disease associated with the overexpression of cytokines or of chemokines, in particular of TNF and/or of IL-12, said disease comprising:
A) immune or autoimmune diseases chosen from the group comprising rheumatoid arthritis, diabetes mellitus, systemic lupus erythematosus or Basedow's disease;
B) transplant rejection;
C) viral and/or parasitic infections;
D) shocks resulting from a chronic or acute infection of bacterial, viral and/or parasitic origin;
E) inflammatory diseases chosen from the group comprising chronic inflammatory diseases and vascular inflammatory diseases;
F) neurodegenerative diseases chosen from the group comprising demyelinating diseases, and extrapyramidal and cerebellar diseases;
G) malignant pathological conditions involving TNF-secreting tumors or involving TNF, chosen from the group comprising leukemia and lymphoma; and
H) alcohol-induced hepatitis.

12. The use as claimed in claim 11, according to which the medicament is intended for the prevention or treatment of an inflammatory disease.
